# EUROPÄISCHE PATENTSCHRIFT

(11) **EP 3 006 917 B1**
(45) Veröffentlichungstag und Bekanntmachung des Hinweises auf die Patenterteilung: **02.03.2022**
(21) Anmeldenummer: 15187203.3
(22) Anmeldetag: 28.09.2015
(51) Int. Cl.: G01N 11/04, G01N 3/10, G01N 33/44

(54) **FLIESSPRÜFMASCHINE UND EIN DAZU GEHÖRENDES MESSVERFAHREN SOWIE EIN DAZUGEHÖRIGES REINIGUNGSVERFAHREN**
FLOW TEST MACHINE AND AN ASSOCIATED MEASUREMENT METHOD, AS WELL AS AN ASSOCIATED CLEANING PROCESS
MACHINE DE VERIFICATION D'ECOULEMENT ET PROCEDE DE MESURE CORRESPONDANT ET PROCEDE DE NETTOYAGE CORRESPONDANT

(30) Priorität: 29.09.2014 DE 102014114117
(43) Veröffentlichungstag der Anmeldung: 13.04.2016
(73) Patentinhaber: ZwickRoell GmbH & Co. KG, 89079 Ulm (DE)
(72) Erfinder: Ganser, Franz, 88471 Laupheim (DE); Völzke, David, 70736 Fellbach (DE); Flock, Marcus, 88480 Achstetten (DE); Kindermann, Ulrich, 89155 Erbach (DE)
(74) Vertreter: Cremer, Christian

(56) Entgegenhaltungen:
- WO-A1-2008/049051
- JP-A- S58 113 734
- US-A- 5 008 081
- US-A- 5 347 851

## Beschreibung

In der Werkstoffprüfung von Thermoplasten werden üblicherweise Fließprüfmaschinen, die von einigen Herstellern auch als Kapillar-Viskosimeter bezeichnet werden, verwendet, durch die insbesondere aufschmelzbare oder verschiebliche Kunststoffe auf ihr Fließverhalten beim Verpressen getestet werden. Einige der Prüfmaschinen arbeiten mit der Erfassung von Schmelze-Massefließraten, andere arbeiten mit Schmelze-Volumenfließraten. Schmelze-Massefließraten und Schmelze-Volumenfließraten können bei bekannter Dichte der Schmelze unter Berücksichtigung der unterschiedlichen Prüfbedingungen ineinander umgerechnet werden. Bei beiden Fließratenmessungen ist eine Messgröße die Zeit. Die vorliegende Erfindung betrifft also eine Fließprüfmaschine, insbesondere für Viskositätsprüfungen, z. B. von Kunststoffen, nach dem Oberbegriff des Anspruchs 1, ein Verfahren zur Prüfung von Viskositäten nach dem Oberbegriff des Anspruchs 14 sowie ein Verfahren zur Reinigung einer Fließprüfmaschine nach dem Oberbegriff des Anspruchs 19.

### Stand der Technik

Fließprüfmaschinen werden z. B. für Vergleichsuntersuchungen an Kunststoffen eingesetzt, weshalb sich verschiedene Hersteller schon seit längerem bemühen, Fließprüfmaschinen bereitzustellen, mit denen Erkenntnisse über das Fließverhalten eines Kunststoffs oder einer Kunststoffmischung, insbesondere unter ausgewählten Versuchsparametern, gewonnen werden können.

So wird ein Schmelzviskositätsprüfgerät u. a. in der DE 2 140 619 A (Anmelderin: Badische Anilin- & Soda-Fabrik AG; Offenlegungstag: 22.02.1973) vorgestellt. Ein Schmelzviskositätsprüfgerät gemäß DE 2 140 619 A soll ein teilbares Gewicht, ein "Stopfgewicht" und ein "Messgewicht" umfassen, die in entsprechenden Phasen einer Prüfung verwendet werden. Beide Gewichte zusammengesetzt sollen zum Stopfen der Messprobe im Prüfkanal und zum Reinigen des Prüfkanals nach erfolgtem Messzyklus dienen, wobei aber auch nur das Messgewicht als Belastungsgewicht gewählt werden könne. Eine Aufzugsvorrichtung ist vorgesehen, die allerdings mit Ketten arbeiten soll und deshalb vermutlich nicht zur Beaufschlagung einer Gewichtskraft mit einer Zusatzkraft einsetzbar ist. Beim Stopfen soll ein Druckstempel mit Hilfe eines Getriebemotors der Aufzugsvorrichtung und mit Hilfe der Gewichte mehrmals in den Prüfkanal eingefahren werden. Nach Beendigung einer Aufheizzeit soll eine Entkopplung von Stopfgewicht und Messgewicht erfolgen, wobei eine motorisch betriebene Sperrklinke das Stopfgewicht in einer Lage festhält. Das Messgewicht und der Druckstempel können zusammen auf einem Querbalken abwärtsbewegt werden. An einer Schaltposition kann der Querbalken durch den Getriebemotor vom Messgewicht wegbewegt werden, womit der Messgang freigegeben wird. An einem sogenannten "unteren Totpunkt" ist die Messung zu beenden. Mit dem Getriebemotor soll der Druckstempel wieder nach oben bewegt werden. Die Gewichte sollen, wie bereits gesagt, wieder zusammengeschlossen werden, und zwar an einem "oberen Totpunkt" des Druckstempels. Beide Gewichte sind dann gemeinsam für einen Reinigungsgang verwendbar, um den Prüfkanal und den Schaft des Druckstempels von Probenrückständen zu säubern. Ähnliche Konstruktionen werden in der DE 1 773 874 A (Anmelderin: Badische Anilin- & Soda-Fabrik AG; Offenlegungstag: 13.01.1972) und der US 3 625 050 (Inhaberin: Badische Anilin- & Soda-Fabrik AG; Patentierungstag: 07.12.1971) dargelegt.

Das deutsche Gebrauchsmuster DE 76 40 849 U (Anmelderin: Göttfert-Feinwerk-Technik GmbH; Offenlegungstag: 11.08.1977) betrifft ein Kapillar-Rheometer zur Messung der Viskosität von Kautschuk und thermoplastischen Kunststoffen. Die Prüfung soll mit einem drehzahlgeregelten Antriebsmotor zum Verfahren des Prüfstempels durchgeführt werden, wobei eine Regelung ermöglicht, in Verbindung mit einem Kraftaufnehmer und einem Massedruckaufnehmer, der vor der Düse angeordnet ist, Messungen in drei verschiedenen Betriebsarten vornehmen zu können. Die Betriebsarten sind eine konstante Prüfkraft am Prüfstempel, ein konstanter Prüfmassedruck vor der Düse oder eine konstante Geschwindigkeit des aus der Düse austretenden Extrudats. Zusätzlich ist eine Geschwindigkeitsregelung vorgesehen, um einen Geschwindigkeits-Sollwert einzustellen.

In der US 5 347 851 A (Inhaberin: Dynisco, Inc.; Patentierungstag: 20.09.1994) wird eine als Kapillar-Rheometer bezeichnete Prüfmaschine beschrieben, bei der sich eine Lastschraube an einer als Schraubengegenlager genutzten Traverse abdrückt. Weil Reibungskräfte, u. a. zwischen einem Kolben und einer Reservoirwand, zu einem erheblichen Messfehler führen können, wird in dem US-Dokument eine Weiterentwicklung ihrer eingangs vorgestellten Maschine für die Prüfungen mit einer eingesetzten Lastschraube vorgeschlagen, mittels der ein gewisser Druck auf den Kolben ausgeübt werden kann. Der eigentliche Prüfdruck wird dann über eine sog. Übertragungsanordnung (englisch: "plunger pressure transducer assembly") erzeugt. Die in der US 5 347 851 A beschriebene Anordnung weist für die Druckübertragung eine Kapillare auf, die mit einem fluiden Metall gefüllt sein soll. Folglich setzt sich eine auf ein "melted polymer" aufgebrachte Kraft aus einer durch eine Schraubenvorspannung erzeugten Grundkraft und einer hydraulisch über flüssiges Metall übertragenen Auspresskraft zusammen.

Die internationale Anmeldung WO 2008/ 049 051 A1 (Anmelderin: Ticona LLC; Veröffentlichungstag: 24. April 2008) beschäftigt sich mit einem Kapillar-Rheometer, der einen Antrieb für eine Linear-Bewegung eines Linear-Bewegungsstabs aufweist, wobei der Linearbewegungsstab mit einem Reinigungs- und Verdichtungsstab koaxial verbindbar sein soll. Es ist vorgesehen, nach einem Test zur Bestimmung der thermischen Viskosität eines Polymers das Gehäuse des Kapillar-Rheometers in eine Reinigungsposition zu schwenken, um dort die Vorbereitungsschritte "Reinigung" und "Verdichtung" auszuführen. In dem Gehäuse befinden sich ein Behältnis mit einer Austrittsdüse für das zu testende Polymer und eine Heizung. Nach dem Vorbereitungsschritt wird das Gehäuse in die Testposition zurückgeschwenkt, an der sich ein Druckstempel befindet.

Das Dokument DE 27 35 224 A1 (Anmelderin: Instruments S. A.; Offenlegungstag: 15.02.1979) bezieht sich auf ein Kapillar-Viskosimeter zur Untersuchung der Eignung von Polymeren für den Spritzguss. Einer Kapillare vorgelagert soll eine Scherkammer angeordnet werden, vor der wiederum eine Druckkammer für Rohgut liegen soll. Zur Abscherung des Guts und zu seiner Aufheizung ist der Kolben in Drehung zu versetzen. Weichgemachtes Gut wird durch den Kolben mittels Druckmittelzylinder aus der Vorratskammer in die Kapillare verdrängt.

In der Anmeldeschrift EP 0 276 683 A2 (Anmelderin: Sumitomo Chemical Company, Limited; Offenlegungstag: 17.08.1988) ist eine automatische Reinigungsvorrichtung für ein Extrusions-Plastometer für thermoplastische Materialien beschrieben. Ein zu reinigender Zylinder wird mittels einer Beförderungseinrichtung einer Reinigungseinrichtung zugeführt.

Ein Prozess und eine Anlage zur Messung von Fließeigenschaften eines in einer Flüssigkeit befindlichen Pigments werden in der Patentschrift US 4 574 623 A (Inhaberin: Sun Chemical Corporation; Patentierungstag: 11.03.1986) beschrieben. Die Anlage umfasst einen wasserummantelten Zylinder mit einem Abschneider am Ende und einen zum Zylinder passenden Kolben. Der Kolben hat ein festgelegtes Gewicht, wodurch der Ausfluss des Inhalts aus dem Zylinder durch eine Öffnung hindurch in einer zu messenden Zeit erfolgt.

Die Patentschrift US 4 882 930 A (Inhaberin: Automatik Machinery Corporation; Patentierungstag: 28.11.1989) betrifft ein System zur Bestimmung eines Schmelzindex von Plastik mit einer automatischen Probenzuführung. Es werden Prüfgeräte, die für manuellen Betrieb vorgesehen sind, verwendet. Von einem Teststößel wird eine aufgeschmolzene Probe zur Bestimmung der Viskosität bei einer eingestellten Temperatur mittels aufgelegter Gewichte durch eine kalibrierte Öffnung gedrückt. Die automatisierte Anlage umfasst einen Roboterarm, der auch die Reinigungsmittel handhabt. Rückstände in der Schmelzkammer werden nach den Tests zur Schmelzindex-Bestimmung durch Hochdruckluft ausgeblasen. Es kann auch die Verwendung eines Reinigungstuchs vorgesehen sein. Der Teststößel wird durch Bürsten automatisch gereinigt.

Eine Anlage für automatische Fließratenmessungen von thermoplastischen Polymeren wird in der Patentschrift US 5 016 467 A (Inhaberin Amoco Corporation; Patentierungstag: 21.05.1991) beschrieben. An einen Heizblock, der die Teströhre umfasst, werden für die einzelnen Prozessschritte jeweils gesonderte Halter horizontal herangefahren. Nach dem Einsenken des Prüfstößels an einem Haltemechanismus in die Teströhre wird der Haltemechanismus abgekoppelt, weggefahren und die Heizung wird gestartet. Anschließend wird mit einem Transporter das Prüfgewicht herangefahren und auf einen Kragen des Prüfstößels aufgesetzt. Für die Messung wird das Prüfgewicht freigegeben und nach einem ersten Fallweg wird die eigentliche Messung über einen Schalter gestartet. Die Betätigung eines darunterliegenden Schalters beendet die Messung und der Transporter nimmt das Gewicht wieder auf. Der Halter des Prüfstößels wird danach herangefahren, um den Prüfstößel zu greifen und herauszuziehen. Der Prüfstößel kann danach in eine Reinigungsröhre eingefahren werden. Für die Reinigung der Teströhre ist ebenfalls ein selbständiger Mechanismus vorgesehen. Somit umfasst die Anlage zahlreiche verfahrbare Komponenten.

In der europäischen Patentschrift EP 2 480 863 B1 (Inhaberin: Zwick GmbH & Co. KG; Prioritätstag: 24.09.2009) sowie der deutschen Patentanmeldung DE 10 2009 042 478 A1 Patentanmelderin: Zwick GmbH & Co. KG; Prioritätstag: 24.09.2009) ist ein Kraftmesskopf für Prüfgeräte zur Durchführung von Werkstoffprüfungen mit einem Kraftaufnehmer beschrieben, wobei u. a. der Kraftaufnehmer von einem wärmeisolierenden Abschirmkörper ummantelt ist. Die Figuren zeigen Einzelheiten, die in Fließprüfmaschinen verbaut sein können.

Die US-Patentschrift US 5 008 081 A (Inhaberin: KD Group; Patentierungstag: 16.04.1991) beschreibt ein Gerät und ein dazugehöriges Verfahren zur Bestimmung von Schmelzindexwerten für Substanzen wie Polymere. Mithilfe einer Aktuatorik sollen aus einem Karussell verschiedene Kartuschen mit Substanzen für die Prüfung in ein Probengehäuse eingesetzt werden. Das Probengehäuse ist mit einer Heizung ausgestattet. Auf einer Gewichtsträgerplatte einer Stampfstange befindet sich ein Gewicht, auf das mithilfe einer oberen Plattform eines Hebemechanismus (engl.: "lift mechanism") ein Druck ausgeübt werden kann. Der Hebemechanismus arbeitet mit einer Gewindestange. An der Gewichtsträgerplatte soll eine Markierung mit Fenstern montiert sein. Während der eigentlichen Messphase soll sich, wie in Figur 12 B und in Figur 12 C gezeigt ist, die Stampfstange unter Einfluss des Gewichts über eine vorgegebene Strecke durch die Probenkartusche bewegen. Hierbei erfolgt eine Zeitmessung, während ein als "Extrudat" bezeichneter Anteil der Probe durch eine Zentralöffnung der Probenkartusche gedrückt wird.

Prüfbedingungen für die Prüfung von Thermoplasten, insbesondere zur Ermittlung einer Schmelze-Massefließrate und/oder einer Schmelze-Volumenfließrate sind in den Normen ISO 1133-1: 2011 und ISO 1133-2: 2011 festgelegt. In den Vereinigten Staaten von Amerika findet die Norm ASTM D 1238 Anwendung. Die in den genannten Normen festgelegten Prüfvorschriften, insbesondere in Bezug auf Geometrien, Maße und Prüfbedingungen, gelten als vollumfänglich in den Offenbarungsumfang dieser Anmeldung inkludiert. Durch Einhaltung der Normen können z. B. im technischen Gebiet der Qualitätssicherung aufgrund der gegebenen Standardisierung vergleichbare Ergebnisse gewonnen werden.

Es sei außerdem noch auf zwei Prospekte der Tinius Olsen, Inc. hingewiesen, nämlich das "Bulletin 131-A" (datiert auf 06/2003) und das "Bulletin 131-D" (datiert auf 08/2012), in denen Geräte zur Durchführung von Materialprüfungen nach nordamerikanischen Normen vorgestellt werden.

### Aufgabenstellung

Besonders erstrebenswert wäre es, ein Konzept für eine Fließprüfmaschine zu kennen, das unterschiedlichsten normungsgemäßen Anforderungen genügt. Eine solche Fließprüfmaschine könnte dem Grunde nach in verschiedenen Ländern, in denen diese unterschiedlichen Normen gelten, eingesetzt werden. Die Fließprüfmaschine soll nach einem anderen Aspekt eine besonders effiziente Durchführung von Fließprüfungen ermöglichen. Der vorliegenden Erfindung liegt somit die Überlegung zugrunde, eine Prüfmaschine zu schaffen, mit der Materialprüfungen oder Serien von Materialprüfungen, insbesondere an fließfähigen Thermoplasten, reproduzierbar, insbesondere normungsgemäß, durchführbar sind. Eine besonders vorteilhafte Fließprüfmaschine wäre also eine, die sowohl Prüfungen nach verschiedenen normungsgemäßen Vorgaben als auch mit besonders kurzen Taktungen, insbesondere bei einer Folge von Materialprüfungen, durchführen kann, wobei natürlich einem Fachmann klar ist, dass so manche Vorgabe aus einer Norm wiederum einer kurzen Prüfzeit entgegensteht.

### Erfindungsbeschreibung

Die erfindungsgemäße Aufgabe wird durch eine Fließprüfmaschine nach Anspruch 1 gelöst. Ein geeignetes Verfahren zur Durchführung einer Schmelzviskositätsprüfung lässt sich Anspruch 14 entnehmen. Um mit einer Fließprüfmaschine möglichst gut vergleichbare Messergebnisse erzielen zu können, bietet Anspruch 19 ein erfindungsgemäßes Verfahren zur Reinigung einer Fließprüfmaschine. Vorteilhafte Weiterbildungen lassen sich den abhängigen Ansprüchen entnehmen.

Die Schmelze-Massefließrate und die Schmelze-Volumenfließrate sind Materialeigenschaften, die unter anderem von der chemischen Zusammensetzung des untersuchten Kunststoffs oder der Qualität des verwendeten Kunststoffs abhängen. Kunststoffe liegen oft als Polymere vor, deren Fließfähigkeit durch Kettenlängen oder auch eine Anzahl von Brückenbindungen zwischen Polymerketten verlangsamt sein kann. Für viele Kunststoffsorten sind Werte von Fließraten, die auf eine annehmbare Qualität des Kunststoffs schließen lassen, tabellarisch z. B. in einschlägigen Handbüchern oder in Herstellerdatenbanken hinterlegt. Abweichungen von tabellarisch hinterlegten Werten können auch auf Schädigungen und/oder Fremdstoffe in einem zu prüfenden Thermoplast hinweisen. Änderungen in einer Struktur von Kunststoffen, z. B. ein Bruch in Polymerketten von Kunststoffmolekülen, können beispielsweise durch Alterung, durch energiereiche, insbesondere ultraviolette Strahlung, durch mechanische Belastungen oder durch kurzfristige Überhitzung bewirkt worden sein. Bei bekannten Kunststoffen finden die ermittelten Schmelze-Massefließraten und Schmelze-Volumenfließraten, insbesondere ohne Umrechnung, als ein Parameter der Qualitätssicherung Anwendung. Gemessene Fließzeiten sind ein Maß für die Viskosität der untersuchten Kunststoffe. Die Viskosität ist mittels ausgewerteter Messdaten einer Fließprüfmaschine berechenbar. Anders gesagt, ist der Kehrwert der Viskosität, die Fluidität, ein Maß für die Fließfähigkeit eines Fluids. In Bezug auf viele zu untersuchende Kunststoffe gilt die Regel, dass die Viskosität eines Fluids bei einer Temperaturerhöhung des Fluids abnimmt.

Eine Fließprüfmaschine, oft auch Kapillarrheometer genannt, ist ein Messgerät, mit dem Fließraten von insbesondere flüssigen Materialien, wie z. B. aufgeschmolzenen Kunststoffen, gemessen werden können. Die Fließprüfmaschine dient zur Messung eines Verformungs- und/oder eines Fließverhaltens von Materialien. Es können insbesondere Materialeigenschaften, wie die Viskosität oder von der Viskosität abgeleitete Parameter, von Werkstoffen in einem flüssigen Aggregatzustand ermittelt werden. Die Fließprüfmaschine ist zur Ermittlung von Fließeigenschaften von thermoplastischen Kunststoffen ausgestaltet. Thermoplastische Kunststoffe sind Materialien, die in einem ersten Temperaturbereich, der insbesondere durch die thermische Zersetzungstemperatur des Kunststoffs zu höheren Temperaturen hin begrenzt ist, in einem fließfähigen Aggregatzustand vorliegen. Grundsätzlich können manche thermisch stabilen Kunststoffe auch als Dampf vorliegen, dessen Viskositätseigenschaften in einer Fließprüfmaschine untersucht werden können. Fließprüfmaschinen eignen sich besonders zur Untersuchung kondensierter Materie. An den ersten Temperaturbereich schließt sich zu niedrigeren Temperaturen ein zweiter Temperaturbereich an, in dem thermoplastische Kunststoffe plastisch verformbar sind. In einem dritten Temperaturbereich, der unterhalb des zweiten Temperaturbereichs liegt, sind thermoplastische Kunststoffe elastisch. In einem vierten, noch niedrigeren Temperaturbereich, beispielsweise unterhalb von 20 °C sind viele Kunststoffe strukturfest und können bei Untersuchungen zu tieferen Temperaturen hin eine Zunahme von Sprödigkeit zeigen. Aufgrund der besonders hohen Kräfte, die im Allgemeinen zum Verpressen elastischer oder fester Kunststoffe erforderlich sind, werden Fließprüfmaschinen für derart vorliegende Kunststoffe eher selten eingesetzt. Nach einer plastischen Verformung bleibt die eingenommene Form des Kunststoffs erhalten. Eine elastische Verformung relaxiert, wenn die formgebende Kraft abgestellt wird. Zahlreiche polymerische Kunststoffe, z. B. Polymere basierend auf Einheiten von Olefinen, Styrolen, Amiden, Lactaten, Methacrylaten, Carbonaten, Ethylenterephthalaten oder Vinylchloriden, besitzen thermoplastische Eigenschaften.

Die Fließprüfmaschine weist einen Prüfkanal auf. Der Prüfkanal ist eine, insbesondere zylinderförmige, Ausnehmung, wie eine Bohrung, in einem festen, druckbeständigen und vorzugsweise chemisch inerten Material, wie z. B. einem Stahl. Der Prüfkanal weist einen Kanaldurchmesser und eine Kanallänge auf, wobei vorzugsweise die Kanallänge größer als der Kanaldurchmesser ist. Der Prüfkanal ist vorteilhafterweise zylinderförmig. Der Prüfkanal ist mit einer Materialprobe des zu prüfenden Materials befüllbar. Die Materialprobe kann z. B. als Stab, als Granulat oder als Pulver mit einem Durchmesser kleiner als der Kanaldurchmesser oder auch als eine Flüssigkeit vorliegen. Die Größe des Durchmessers des Prüfkanals begünstigt das Auffüllen des Prüfkanals, z. B. mit einer festen, granulatartigen Materialprobe. Die Kanallänge des Prüfkanals ist vorzugsweise entlang einer Senkrechten ausrichtbar.

Dem Prüfkanal ist mindestens ein Prüfkolben zugeordnet. Der Prüfkolben weist einen Prüfkolbendurchmesser auf, der kleiner als der Prüfkanaldurchmesser ist, sodass der Prüfkolben in den Prüfkanal einführbar ist. Eine Länge des Prüfkolbens kann größer oder gleich einer Länge des Prüfkanals sein. Der Prüfkolben ist vorzugsweise in einer Nutzstellung entlang des Prüfkanals, insbesondere entlang einer Zentralachse des Prüfkanals, angeordnet. Zumindest ein Achsenabschnitt der Zentralachse kann auch als Prüfachse bezeichnet werden. Eine Prüfkolbenachse liegt zumindest in einem Messtakt auf der Zentralachse des Prüfkanals. Der Prüfkolben kann in Bezug auf den Prüfkanal ausrichtbar sein. In einem Bereich des Prüfkolbens, vorzugsweise einem, insbesondere zylindrischen, Endbereich, an dem sich eine Pressfläche befindet, kann der Prüfkolben umfänglich fluiddicht an einer Innenwand des Prüfkanals anliegen. Nach einem Aspekt ist der Prüfkolben als ein fester Stab ausgebildet. Es ist möglich, dass mindestens ein Hohlraum in dem Prüfkolben vorgesehen ist, z. B. um ein Gewicht des Prüfkolbens zu verringern. Der Prüfkolben sollte steif gegen Biegungen gestaltet sein. Eine Prüfkraft ist von einem ersten Endbereich des Prüfkolbens zu einem zweiten Endbereich des Prüfkolbens, vorzugsweise verlustfrei, übertragbar.

Es ist mindestens ein Prüfgewicht vorgesehen. Das Prüfgewicht dient dazu, eine definierte Gewichtskraft bereitzustellen. Das Prüfgewicht hat eine vorgegebene Masse. Das Prüfgewicht kann aus stapelbaren Prüfgewichtskörpern zusammengesetzt sein. Günstig für eine zentrierte Bereitstellung einer summarischen Gewichtskraft sind Zylinderscheiben als Prüfgewichtskörper. An den gegenüberliegenden Flächen der Zylinderscheiben können ein Auflagebereich und ein Zentrierbereich vorgesehen sein, wobei ein erster Zentrierbereich eines ersten Prüfgewichtskörpers in einen zweiten Zentrierbereich eines zweiten Prüfgewichtskörpers passt.

Eine Masse des Prüfgewichts wird normungsgemäß in der Regel abhängig von dem zu testenden Material festgelegt. Die Masse eines Prüfgewichts kann beispielsweise 20 kg (Kilogramm) betragen. Prüfgewichte können aber auch weniger als 1 kg schwer sein. Weil die Erdbeschleunigung vom Ort der Messung abhängig ist, kann die Gewichtskraft am jeweiligen Ort der Messung zur weiteren Erhöhung der Messgenauigkeit berücksichtigt werden, z. B. durch Korrekturgewichte im Promillebereich der jeweiligen Masse. Ein erster Körper, der ein Gewicht hat und daher als Prüfgewicht bezeichnet werden kann, ist der Prüfkolben. Ein, insbesondere erster, Prüfgewichtskörper ist mit dem Prüfkolben, insbesondere mit einem ersten Endbereich des Prüfkolbens, in eine formschlüssige und kraftschlüssige Verbindung verbringbar. Ein weiterer Prüfgewichtskörper, wie ein zweiter Prüfgewichtskörper, kann auf den Prüfgewichtskörper, insbesondere den ersten Prüfgewichtskörper, aufsetzbar sein. Die Gewichtskraft des Prüfgewichtskörpers wirkt auf den Prüfkolben, insbesondere entlang einer Zentralachse des Prüfkolbens. Das Prüfgewicht kann den Prüfkolben in den Prüfkanal hineindrücken. Die Gewichtskraft eines aufgesetzten Prüfgewichtskörpers trägt vektoriell zu der Kraft bei, die den Prüfkolben bewegt. Der Prüfkolben ist von einer Gewichtskraft, die insbesondere zumindest eine Reibungskraft zwischen Prüfkolben und Prüfkanal übersteigt, durch den Prüfkanal bewegbar. Ein erster Betriebszustand der Fließprüfmaschine arbeitet vorzugsweise mit dem Prüfgewicht, wobei der erste Betriebszustand eingenommen wird, bevor ein Messbetriebszustand vorliegt.

Die Fließprüfmaschine ist mit einer Antriebseinheit ausgestattet. Die Antriebseinheit kann beispielsweise einen hydraulischen oder einen pneumatischen Aktuator umfassen. Es ist auch möglich, elektromotorische Antriebe zu verwenden, um Kräfte in der Prüfmaschine zu generieren. Der Aktuator kann mit einem, insbesondere zweiseitig ansteuerbaren, Hydraulik- oder Pneumatikzylinder ausgebildet sein. Durch die Antriebseinheit, wie den hydraulischen oder pneumatischen Aktuator, ist eine innerhalb von Toleranzgrenzen vorgebbare Kraft, z. B. über eine Kraftregelungseinheit, ausgehend von einem Quelldruck eines Hydraulikmittels oder einer Druckluftquelle, aufbringbar. Nach einem weiteren Aspekt stellt die Antriebseinheit mindestens einen Fahrweg bereit, auf dem die Kraft der Antriebseinheit verfügbar ist. Entlang des Fahrwegs kann eine Regelung die von der Antriebseinheit bereitgestellte Kraft auf einen konstanten Kraftbetrag stabilisieren. Ein Fahrweg der Antriebseinheit erstreckt sich vorzugsweise entlang der Zentralachse des Prüfkanals.

Nach einem Aspekt kann die Antriebseinheit den Prüfkolben, insbesondere in dem Prüfkanal, entgegen einer Schwerkraft, insbesondere des Prüfgewichts, bewegen. Anders gesagt, ist das Prüfgewicht durch die Antriebseinheit hebbar, wobei eine Bewegungsrichtung entgegen der Schwerkraft möglich ist. Es ist allerdings auch möglich, das Prüfgewicht durch die Antriebseinheit abzusenken. In einer weiteren Konfiguration ist es möglich, insbesondere zusätzlich möglich, eine Gewichtskraft des Prüfgewichts durch die Antriebseinheit zu verringern. Wenn auf den Prüfkolben eine von der Antriebseinheit herrührende Kraft ausgeübt wird, stehen der Prüfkolben und die Antriebseinheit in einer vorzugsweise mechanischen Verbindung. Alternativ ist es auch möglich, eine Magnetkraftkopplung zur Kraftübertragung vorzusehen, z. B. um eine mögliche Übertragung von Schwingungen auf den Prüfkolben zu unterbinden. Es kann auch gesagt werden, dass das Prüfgewicht und die Antriebseinheit in einer Kraftkopplungsverbindung stehen. Das Bestehen einer Kraftkopplungsverbindung kann als ein Antriebszustand bezeichnet werden. In dem Antriebszustand liegt an dem Prüfkolben eine von der Antriebseinheit erzeugte, aktuatorische Zusatzkraft an. Der erste Betriebszustand kann ein Antriebszustand sein. In einem weiteren Betriebszustand bzw. zur Einnahme dieses Betriebszustands ist die Kraftkopplungsverbindung lösbar, sodass zwischen Antriebseinheit und Prüfkolben kein Anschluss mehr besteht, also keine aktuatorische Zusatzkraft vorliegt.

Obwohl vorrangig die Bewegungen des Prüfkolbens in Bezug zu dem, vorzugsweise statisch angeordneten, Prüfkanal betrachtet werden, sind auch Ausführungen möglich, in denen die Antriebseinheit eine Relativbewegung zwischen Prüfkolben und Prüfkanal, z. B. durch einen Anschluss an den Prüfkanal oder auch nach Art einer Zangenbewegung, bewirkt oder unterstützt.

In Bezug auf den Prüfkanal können dem Prüfkolben mehrere Stellungen zugeordnet werden, die im Folgenden näher beschrieben werden. Eine erste Stellung kann als Anfahrstellung bezeichnet werden. Eine Anfahrstellung kann z. B. von dem Prüfkolben eingenommen sein, wenn ein Endbereich des Prüfkolbens, insbesondere die Pressfläche des Prüfkolbens, einem Endbereich des Prüfkanals, insbesondere einer Einfüllöffnung des Prüfkanals, zugeordnet ist. In der Anfahrstellung setzt die aktuatorische Zusatzkraft ein. Aus der Anfahrstellung heraus ist der Prüfkolben durch die aktuatorische Zusatzkraft beschleunigbar. Der Prüfkolben nimmt ab der Anfahrstellung an Fahrt auf. Eine Anfahrstellung kann auch über ein festgelegtes Zeitintervall hinweg eine Ruhestellung des Prüfkolbens sein. In der Ruhestellung wird der Prüfkolben z. B. durch das Volumen, das eine Kunststofffüllung in dem Prüfkanal einnimmt, in Stellung gehalten. Eine zweite Stellung ist eine Endfahrstellung. Eine Endfahrstellung kann eine Beabstandung der Pressfläche zu der Anfahrstellung längs des Prüfkanals in Richtung der Gewichtskraft des Prüfgewichts haben. Zwischen einer Anfahrstellung und einer Endfahrstellung liegt eine Teilstrecke, wie eine erste Teilstrecke, entlang des Prüfkanals. In der Endfahrstellung endet die Zufuhr der aktuatorischen Zusatzkraft. Die Anfahrstellung und die Endfahrstellung sind entlang der Prüfachse angeordnet. Es können auch mehrere Anfahr- und Endfahrstellungen des Prüfkolbens vorgesehen sein. Zwischen der Anfahrstellung und der Endfahrstellung ist der Prüfkolben sowohl durch die Gewichtskraft als auch durch eine aktuatorische Zusatzkraft beaufschlagt. Es kann auch gesagt werden, dass ein Antriebszustand vorliegt. Der Prüfkolben ist aktuatorisch angetrieben. Die akuatorische Zusatzkraft wird von der Antriebseinheit aufgebracht. Am Prüfkolben greifen in dieser Phase also zumindest die Gewichtskraft und die vektoriell gleichgerichtete, aktuatorische Zusatzkraft an. Wird die Bewegung des Prüfkolbens unter gleichzeitigen Wirkung der Gewichtskraft und der aktuatorischen Zusatzkraft mit einer Bewegung des Prüfkolbens, der nur unter der Wirkung der Gewichtskraft steht, verglichen, führt der Prüfkolben mit der aktuatorischen Zusatzkraft, insbesondere in einem Betriebszustand, eine schnellere Bewegung aus. Der Prüfkolben legt durch die zugeführte aktuatorische Zusatzkraft eine Wegstrecke innerhalb des Prüfkanals zwischen der ersten Stellung und der zweiten Stellung in einer kürzeren Zeit zurück. Wenn sich in dem Prüfkanal ein fließfähiger Kunststoff befindet, so ist durch die aktuatorische Zusatzkraft eine in einer Zeiteinheit, wie z. B. 1 Sekunde, verschiebbare oder auspressbare Kunststoffmasse, anders gesagt, eine verdrängbare Kunststoffmasse, größer als die unter Wirkung der Gewichtskraft des Prüfgewichts verdrängbare Kunststoffmasse. Die auspressbare Kunststoffmasse tritt unter der Wirkung, es kann auch gesagt werden unter dem Druck, des Prüfkolbens aus dem Prüfkanal, insbesondere aus einem der Einfüllöffnung des Prüfkanals gegenüberliegenden Ende des Prüfkanals, aus. Die Messung der Fließzeit in der Fließprüfung erfolgt entlang eines vorbestimmten Streckenbereichs des Prüfkanals, der auch als Versuchsstrecke, Prüfstrecke oder Messstrecke bezeichnet werden kann. Die Anfahrstellung und die Endfahrstellung liegen außerhalb oder am Rand der Versuchsstrecke. Durch die schnellere Bewegung mittels aktuatorischer Zusatzkraft kann zumindest die Versuchsstrecke durch den Prüfkolben schneller erreicht werden.

Die Taktzeiten bei Fließprüfungen können so verkürzt werden. Mit derart ausgebildeten Fließprüfmaschinen können mehr Prüfzyklen in einer Zeiteinheit durchgeführt werden. Die Zeiteffizienz bei der Durchführung von Fließprüfungen wird erhöht.

Während des Messvorgangs legt der Kolben eine Messtrecke zurück. Gemäß einer bevorzugten Ausführungsform kann im Anschluss an die Messstrecke, also räumlich von der ersten Teilstrecke getrennt, der Prüfkolben eine weitere Anfahrstellung aufweisen, ab der die akuatorische Zusatzkraft wieder auf den Prüfkolben aufbringbar ist. Mindestens eine zweite Teilstrecke kann sich an die zweite Anfahrstellung anschließen. Auf der zweiten Teilstrecke wirkt die zusätzliche aktuatorische Kraft auf den Prüfkolben. In diesem Fall führt der Prüfkolben insbesondere eine schnellere Bewegung entlang des Prüfkanals aus. Die zweite Teilstrecke reicht bis zu einer zweiten Endfahrstellung. In allen Teilstrecken, in denen der Prüfkolben die Bewegung unter Wirkung der aktuatorischen Zusatzkraft ausführt, ist Prozesszeit einsparbar, was zu kurzen Zeitschritten führt. Es ist möglich, auf einer zweiten Teilstrecke eine größere aktuatorische Zusatzkraft anzuwenden als auf einer ersten Teilstrecke und damit eine weitere Verkürzung der Taktung zu erzielen.

Eine Schmelzviskositätsprüfung zur Bestimmung von Materialeigenschaften, die Auskunft über oder von dem Fließverhalten geben, z. B. von thermoplastischen Kunststoffen, kann in einer insbesondere zuvor beschriebenen Fließprüfmaschine ausgeführt werden. Das zu prüfende Material in einer vorgegebenen Materialmenge wird in einem ersten Schritt erwärmt. Mittels einer Wärmequelle wird das Prüfmaterial insbesondere auf eine Temperatur verbracht, die in einem ersten oder in einem zweiten Temperaturbereich liegt. Der Heizschritt wandelt das Material in einen verpressbaren Zustand, falls eine Ausgangskonsistenz des Prüfmaterials nicht bereits eine ausreichende Fließfähigkeit aufweist. Die Erwärmung kann insbesondere in einem gleichmäßig beheizten Prüfkanal erfolgen. Während des Heizschritts können z. B. Lufteinschlüsse aus dem Material entweichen. Kavitäten in dem Material, die den Messvorgang beeinträchtigen können, werden zumindest vermindert. Insbesondere bei der Fließprüftemperatur liegt das Prüfmaterial durch Wärmeeinwirkung in einem Fließzustand vor. Ein Fließzustand ist ein Zustand, in dem ein Material zum Fließen gebracht werden kann, indem z. B. eine das Fließen behindernde Sperre oder Verschlussplatte beseitigt wird.

Die Fließprüftemperatur kann oberhalb des Schmelzpunkts des zu prüfenden Materials liegen. Die Zersetzungstemperatur des Prüfmaterials sollte während der Durchführung des Prüfverfahrens möglichst unerreicht bleiben. Es haben sich für viele Kunststoffe z. B. Temperaturen zwischen 180 °C und 300 °C als geeigne t erwiesen. Für Polyethylene liegt eine typische Fließprüftemperatur bei 190 °C. Geeignete Fließprüftemperaturen zu einzelnen Kunststoffen lassen sich aus Daten von Kunststoffherstellern ableiten.

Im Anschluss an den Heizschritt kann die Prüfmasse noch für eine bestimmte Zeitspanne, die Anschmelzzeit, auf einer konstanten Temperatur, der Fließprüftemperatur, gehalten werden, um die Wärme gleichmäßig in der Masse zu verteilen. Es kann auch gesagt werden, dass der Heizschritt eine Aufheizzeit und eine Anschmelzzeit umfassen kann. Die Prüfmasse schmilzt und homogenisiert sich oder wird zumindest plastisch so verformbar, dass sie durch den Prüfkanal drückbar ist. Eine Anschmelzzeit der Prüfmasse für viele Kunststoffe kann 6 Minuten bis 7 Minuten dauern. Die Prüfmasse kann auch als Prüfmaterial bezeichnet werden, das insbesondere in einem Prüfraum, vorzugsweise in einem Versuchsbereich, vorliegt.

Nach dem Heizschritt, insbesondere nach einer Anschmelzzeit, wird die Prüfung der Prüfmasse eingeleitet. Diese Phase des Heizschritts kann auch als Messvorbereitungsschritt charakterisiert werden. Der Prüfkolben wird in dem Messvorbereitungsschritt z. B. aus einer ersten Stellung in eine zweite Stellung bewegt. Ein erster Teil der Prüfmasse wird in dem Messvorbereitungsschritt, insbesondere in kurzer Zeit, also schnell, z. B. innerhalb von nur 5 Sekunden, aus dem Prüfkanal gepresst und von der Prüfmasse getrennt. Es erfolgt eine Verschiebung des Prüfmaterials. Die Verschiebung führt die Prüfmasse einer Austrittsöffnung des Prüfkanals zu. Die Austrittsöffnung befindet sich bei einer senkrechten Anordnung des Prüfkanals unter einer Einfüllöffnung des Prüfkanals. Es erfolgt - mit anderen Worten - eine Absenkung von Prüfmasse. In dem Messvorbereitungsschritt ist die aktuatorische Zusatzkraft an den Prüfkolben angekoppelt. Die aktuatorische Zusatzkraft wirkt entlang einer Gewichtskraftrichtung. Die aktuatorischen Zusatzkraft zielt in die gleiche Richtung wie die Gewichtskraftrichtung, womit der Prüfkolben antreibbar ist. Durch Kraftkopplung wird eine Bewegung des Prüfkolbens bewirkt, die insbesondere schneller ist als ohne Kraftkopplung.

Die Messung, insbesondere einer Auspresszeit, findet nach dem Messvorbereitungsschritt mit einer Prüfmasse aus einem nach der Befüllung des Prüfkanals mittleren Streckenbereich des Prüfkanals statt. Während eines Messschritts wird die Prüfmasse von dem Prüfkolben durch den Prüfkanal gedrückt. Der Prüfkolben übt also eine Kraft auf die im Heizschritt erwärmte Masse aus. Die Kraft hat zur Folge, dass die Prüfmasse aus mindestens einer Öffnung des Prüfkanals austritt.

Während des Messschritts wirkt in einem ersten Messbetriebszustand eine vorgegebene Kraft, die nur durch das Gewicht ausgeübt wird, auf den Prüfkolben. Eine Verbindung zwischen dem Prüfkolben und der Antriebseinheit ist unterbrochen, es kann kein Kraftschluss erfolgen. Das Prüfgewicht bietet die einzige antreibende Kraft auf. Der Prüfkolben in dem Prüfkanal kann aufgrund von Gegenkräften, die z. B. durch Reibungs- und/oder Viskositätseffekte der Prüfmasse verursacht werden, zu einem Stillstand kommen, wenn das Prüfgewicht zu gering ausgelegt ist. Der Gewichtskraft des Prüfgewichts wirkt ein Widerstand entgegen. Eine sehr hohe Genauigkeit kann allerdings auch in einem zweiten möglichen Messbetriebszustand erzielt werden, in dem eine aktuatorische Zusatzkraft einen Prüfkolben während des Messschritts, insbesondere zusätzlich zu einer Gewichtskraft, antreibt, wodurch ein effektives Prüfgewicht bereitgestellt ist. In einer Einstellung ist die aktuatorische Zusatzkraft darauf abgestimmt, den Widerstand, der der Gewichtskraft entgegenwirkt, auszugleichen. In einem solchen Fall erzeugt die Gewichtskraft (vollständig) die Vortriebskraft auf den Prüfkolben.

Nach dem Messschritt kann im Anschluss ein Reinigungsschritt erfolgen. Der Reinigungsschritt dient z. B. dazu, Prüfmaterial, das sich nach dem Messschritt noch in dem Prüfkanal befindet, auszustoßen. Prüfmaterialreste werden vor einer nächsten Schmelzviskositätsprüfung aus dem Prüfkanal entfernt, insbesondere bevor sich mögliche Ablagerungen in dem Prüfkanal festsetzen könnten. Die aktuatorische Zusatzkraft wird im Anschluss an den Messschritt an den Prüfkolben angekoppelt. Der Prüfkolben behält die Bewegungsrichtung des Messschritts bei, wobei eine Beschleunigung des Prüfkolbens und dadurch eine schnellere Ausführung des Reinigungsschritts erfolgen. Es liegt also eine Bewegungsrichtungskontinuität vor, bei der keine Umkehr einer Bewegungsrichtung erfolgt. Die aktuatorische Zusatzkraft ist gleichgerichtet zu der Gewichtskraftrichtung. Weiteres Prüfmaterial wird abgesenkt. Der Messvorbereitungsschritt und der Reinigungsschritt können auch einzeln oder gemeinsam mit dem Messschritt in der Bewegungsrichtungskontinuität kombiniert werden. Damit lassen sich Fließprüfungen sehr genau ausführen.

Eine Fließprüfmaschine sollte während des Gebrauchs bzw. nach jedem Versuch gereinigt werden, um brauchbare Messergebnisse erzielen zu können, was mehr Zeit als eine Zeit für die eigentliche Fließprüfung in Anspruch nehmen kann. Ein erfindungsgemäßes Reinigungsverfahren ist besonders schnell ausführbar und kann z. B. vor oder nach Schmelzviskositätsprüfungen ausgeführt werden. Das Reinigungsverfahren arbeitet mit mindestens einem Reinigungskolben. Der Reinigungskolben ist insbesondere an Stelle eines Prüfkolbens in die Fließprüfmaschine, insbesondere in einen Prüfkanal der Fließprüfmaschine, einsetzbar. Die Fließprüfmaschine weist einen Prüfzylinder auf. Der Prüfzylinder liegt z. B. als eine Komponente einer Antriebseinheit der Fließprüfmaschine vor, die hydraulisch oder pneumatisch zur Erzeugung einer mechanischen Bewegung in einem Fließprüfverfahren beaufschlagbar ist. Die Antriebseinheit treibt in einer Schmelzviskositätsprüfung den Prüfkolben an. Der Prüfzylinder ermöglicht einen Bewegungsspielraum, insbesondere eine, vorzugsweise geradlinige, Fahrstrecke, der Antriebseinheit. Eine von der Antriebseinheit bereitgestellte Kraft kann den Prüfkolben in dem Prüfkanal bewegen. Die Antriebseinheit kann den Prüfkolben zumindest durch eine Teilstrecke des Prüfkanals befördern. Der Prüfkanal kann an einem Ende von einer Auspressdüse begrenzt sein. Die Auspressdüse weist mindestens eine Düsenöffnung auf. Die Auspressdüse ist so ausgelegt, dass sie an dem Prüfkanal zumindest einer einwirkenden Kraft, wie der Gewichtskraft, standhält. Vorzugsweise wird eine Bewegung in eine Richtung auf die Auspressdüse hin angetrieben. Der Prüfkolben wird somit auf eine Auspressdüse zu bewegt. Es ist besonders vorteilhaft, wenn in dem Reinigungsverfahren die Antriebseinheit eine aktuatorische Zusatzkraft zur Durchführung der Reinigung aufbringt. Der Reinigungskolben wird von der Antriebseinheit auf einem Weg des Prüfkolbens durch den Prüfkanal bewegt. Durch die Verwendung der Antriebseinheit der Fließprüfmaschine in dem Reinigungsverfahren werden Verunreinigungen, die mögliche Fehlerquellen bei Fließprüfungen verursachen können, effektiver beseitigt, wodurch die Taktung weiter verbessert wird. Insbesondere Serienprüfungen von z. B. verschiedenen Polymeren oder degradierenden Thermoplasten müssen weniger oft wiederholt werden, um statistisch vertrauenswürdige Messergebnisse zu erhalten.

Nachfolgend werden vorteilhafte Ausgestaltungen und Weiterbildungen dargelegt, die für sich gesehen, sowohl einzeln als auch in Kombination, ebenfalls erfinderische Aspekte offenbaren können.

An der Antriebseinheit kann - gemäß einer vorteilhaften Ausführungsform - eine Kupplungseinheit vorgesehen sein. Eine Kupplungseinheit dient dazu, eine kraftschlüssige Verbindung bereitzustellen. Die Kupplungseinheit kann in der Antriebseinheit angeordnet sein. Vorzugsweise ist die Kupplungseinheit eine Einheit zwischen einem Prüfzylinder und einem Prüfkolben. Schaltbare Komponenten, insbesondere anlegbare Komponenten, wie Klauen, der Kupplungseinheit sind der Antriebseinheit zugeordnet. Die Kupplungseinheit ermöglicht es, die aktuatorische Zusatzkraft von dem Prüfkolben zu nehmen. Insbesondere in einer ersten Schaltstellung liegt eine Abtrennung der aktuatorischen Zusatzkraft von dem Prüfkolben vor. Nach einem weiteren Aspekt kann eine Kupplungseinheit dazu dienen, eine bestehende kraftschlüssige Verbindung zu lösen. In einer zweiten Schaltstellung der Antriebseinheit kann eine Übertragung der aktuatorischen Zusatzkraft von der Antriebseinheit ermöglicht sein. Die aktuatorische Zusatzkraft ist in der zweiten Schaltstellung über die Kupplungseinheit hinweg dem Prüfkolben zuführbar. Die Kupplungseinheit ermöglicht eine präzise Ankopplung oder Abkopplung der Antriebseinheit an den Prüfkolben oder an ein Prüfgewicht, insbesondere einen Prüfgewichtskörper. Die Kupplungseinheit arbeitet bzw. funktioniert schlupffrei. Die Kupplungseinheit ist verwendbar zur Übertragung der aktuatorischen Zusatzkraft. Je nach Schaltstellung der Kupplungseinheit, also in Abhängigkeit von der Schaltstellung der Kupplungseinheit, wird eine aktuatorische Zusatzkraft auf den zu betätigenden Kolben weitergeleitet.

Der Prüfkolben kann einen Prüfkolbenkopf aufweisen. Als Prüfkolbenkopf sind jene Teile des Prüfkolbens bezeichnet, die, ggf. mit weiteren, sich an den Prüfkolbenkopf anschließenden Teilen des Prüfkolbens, außerhalb des Prüfkanals verbleiben, wenn der Prüfkolben entlang einer Länge, die der Kanallänge des Prüfkanals entspricht, in diesem Kanal eingefahren ist. Eine besonders gute Ausrichtung des Prüfkolbens ist erzielbar, wenn ein Durchmesser des Prüfkolbenkopfes größer als ein Durchmesser des Prüfkanals ist. Der Prüfkolbenkopf hat einen ersten Ankoppelbereich. Der Ankoppelbereich ist mit der Antriebseinheit verbindbar. Der Prüfkolbenkopf weist einen Trägerbereich für ein Prüfgewicht, insbesondere für einen Prüfgewichtskörper, auf. Der Trägerbereich und der Ankoppelbereich liegen vorteilhafterweise unmittelbar nebeneinander benachbart im Bereich des Prüfkolbenkopfs. Der Trägerbereich liegt im Bereich eines Endes des Prüfkolbens vor, vorzugsweise bildet das Ende des Prüfkolbens, und dient zur Zusammenfügung mit einem Zentrierbereich eines Prüfgewichtskörpers. Der Kolben wie der Prüfkolben wird vorzugsweise über die Einleitung einer Kraft in eine seiner Endbereiche bewegt. Die Bewegung eines Kolbens ist lanzenartig.

Es kann auch mindestens ein Servicekolben als Komponente der Fließprüfmaschine vorgesehen sein. Der Reinigungskolben ist eine Art Servicekolben. Mit dem Reinigungskolben sind z. B. Bürsten- und/oder Lappenteile in dem Prüfkanal entlang der Kanallänge, insbesondere über eine Prüfkanaloberfläche, bewegbar. Als ein weiterer bzw. anderer Servicekolben kann aber auch ein Verdichtungskolben vorhanden sein. Mit dem Verdichtungskolben kann Prüfmaterial, das z. B. als Granulat oder als Pulver in den Prüfkanal eingefüllt wurde, verdichtet werden. In einer Betriebsweise bzw. Nutzungsart der Fließprüfmaschine kann auch der Prüfkolben als Verdichtungskolben verwendet werden. Zumindest ein erster Verdichtungsschritt ist mit einem Prüfkolben ausführbar. Ein Servicekolben kann z. B. mit einem Longitudinalvibrator oder einem Heizelement ausgestattet sein. Ein Servicekolben kann einen Entlüftungskanal für den Prüfkanal aufweisen. Der Servicekolben hat einen Kopfbereich. Der Kopfbereich ist dazu bestimmt, außerhalb des Prüfkanals zu verbleiben. Am Kopfbereich des Servicekolbens befindet sich ein zweiter Ankoppelbereich. Insbesondere mindestens einer der Ankoppelbereiche weist eine Form auf, die mindestens eine Verbindungsfläche bietet. Die Ausgestaltung der Form ermöglicht eine kraftschlüssige Verbindung zwischen der Antriebseinheit und dem Ankoppelbereich des zugeordneten Kolbens (Prüf- oder Servicekolben). Eine kraft- und/oder formschlüssige Verbindung ist durch eine Ausgestaltung eines Bereichs zwischen der Antriebseinheit und einem Kolben, wie dem Prüfkolben oder dem Reinigungskolben, ausbildbar. Die Form des Ankoppelbereichs ist vorzugsweise an eine Form eines Aufnahmebereichs der Antriebseinheit anschließbar. Damit lassen sich Servicekolben schnell durch Prüfkolben ersetzen. Durch die Vereinfachung der Bedienung lässt sich die Taktung zur Durchführung von zwei Versuchen nacheinander steigern.

Mit einer Fließprüfmaschine lassen sich durch einen Anwender auch besonders zügig Fließprüfungen durchführen, wenn die Antriebseinheit eine schaltbare Halterung aufweist. Die Halterung kann aktuatorisch schaltbar sein. Die Halterung dient dazu, den Prüf- oder den Servicekolben aufzunehmen. Die Halterung ist in eine Freigabestellung schaltbar. In der Freigabestellung kann der jeweils in der Halterung vorhandene Prüf- oder Servicekolben entnommen oder abgesetzt werden. Die Halterung ist in eine Schließstellung schaltbar. Die Halterung kann sich z. B. an den Kopf des Prüfkolbens anschließen. In der Schließstellung ist der Kolben, der sich in der Halterung befindet, mit der Antriebseinheit kraftfest, vorzugsweise auch richtungsfest, verbunden. Die Halterung kann eine Spanneinheit umfassen. Besonders vorteilhaft zur Festlegung einer Position insbesondere eines Prüfkolbens ist eine Spanneinheit, die als eine Zentriereinheit bezogen auf eine Prüfkolbenachse ausgelegt ist. Unter einem anderen Blickwinkel lässt sich auch sagen, dass die Spanneinheit auch eine Zentrierung in Bezug auf die Zentralachse des Prüfkanals bieten kann. Die Zentralachse geht z. B. bei einem kreisförmigen Querschnitt des Prüfkanals durch den Mittelpunkt des Querschnittskreises. Mit anderen Worten, dient die Spanneinheit einer reproduzierbaren Ausrichtung des Prüfkolbens. Aufgrund der zentrierenden Spanneinheit ist eine Schließstellung ohne auf den Prüfkolben wirkende Querkräfte oder Kippkräfte einnehmbar. Insbesondere wird die Prüfkolbenachse auf der Zentralachse des Prüfkanals belassen. Die Spanneinheit kann beispielsweise eine Klammer, wie eine schaltbare Dreipunktklammer, oder eine Zange, wie ein dreifingerartiger Greifer, oder ein Radialgreifer sein. Die Spanneinheit kann mindestens ein Formelement, vorzugsweise drei Formelemente, aufweisen, die an den Prüfkolben anlegbar sind. Die Formelemente bieten, vorzugsweise zwei gegenüberliegende, Anlegeflächen, die in einer Längserstreckung einander angenähert sind. Die Anlegeflächen sind, je nach einzuspannendem Kolben, an den ersten Ankoppelbereich sowie an den zweiten Ankoppelbereich anlegbar. Eine Rastierung ist, insbesondere bidirektional, in Prüfkolbenachsrichtung ausbildbar. Die Spanneinheit kann hydraulisch oder pneumatisch betätigbar sein. Als eine weitere bzw. andere Ausführungsform lässt sich diese auch so gestalten, dass eine elektromagnetisch schaltbare Halterung vorhanden ist. Beispielsweise ein Schloss kann in Verbindung mit der Halterung den Verbund von Antriebseinheit und Prüfkolben als zentrierende Spanneinheit sichern. Das Schloss kann z. B. ein elektromagnetisches Schloss, ein pneumatisches Schloss oder ein hydraulisch betätigbares Schloss sein. Die schnelle Einnahme einer Schließstellung oder einer Freigabestellung ermöglicht einen Zugriff auf den Prüf- oder den Servicekolben in weniger als 10 Sekunden.

Besonders vorteilhaft ist es, wenn der Halterung ein Näherungssensor zugeordnet ist. Der Näherungssensor erkennt, ob sich in einer räumlichen Umgebung des Näherungssensors ein Gegenstand befindet, der dem Prüfkolben oder dem Kopf des Prüfkolbens zuordenbar ist. Der Näherungssensor kann eine Abstandsänderungsbestimmung, insbesondere eine Positionsbestimmung, ermöglichen und insbesondere als Zentrierhilfe ausgebildet sein. Der Näherungssensor arbeitet vorzugsweise induktiv, wobei eine Positionsbestimmung im Wirkungsbereich mindestens eines magnetischen Felds der Fließprüfmaschine erfolgt. Der Näherungssensor ermöglicht besonders schnelle und genaue Annäherungen an den Prüfkolben. Es ist auch möglich, Näherungssensoren zu verwenden, die mechanisch, elektromagnetisch, insbesondere optisch, oder thermisch arbeiten. Das Formelement kann z. B. mit einem Näherungssensor ausgestattet sein. Hierdurch kann, sozusagen zügig, die Bewegung des Kolbens kontrolliert durchgeführt werden.

Gemäß einer weiteren vorteilhaften Weiterbildung einer Fließprüfmaschine ist eine Wegmesseinrichtung vorgesehen. Die Kolbenstellungsmesseinrichtung kann eine Wegmesseinrichtung sein. Eine Wegmesseinrichtung erhebt bzw. misst insbesondere eine Differenz in der Stellung eines Prüfkolbens oder eines Servicekolbens in Form eines bzw. messtechnisch als einen betragsmäßigen Abstand. Die Wegmesseinrichtung kann mit einem Prüfzylinder vorteilhaft in einer Regelung zusammenarbeiten. Die Wegmesseinrichtung ermittelt, an welcher Stelle, insbesondere innerhalb des Prüfkanals, sich der Prüfkolben oder der Servicekolben befindet. Es kann auch gesagt werden, dass die Kolbenstellungsmesseinrichtung oder die Wegmesseinrichtung erkennt, wie weit z. B. der Prüfkolben noch in den Prüfkanal einfahrbar ist, um eine vorgegebene Stellung zu erreichen oder zu überschreiten. Ein zurückgelegter Weg des Prüfkolbens oder des Servicekolbens ist mit der Kolbenstellungsmesseinrichtung ermittelbar. Die Wegmesseinrichtung gibt eine dem Prüfkolben zugeordnete Position als digitales Signal an eine Steuereinheit, insbesondere der Fließprüfmaschine, weiter. Die Wegmesseinrichtung stellt, anders gesagt, ein Stellungssignal bereit. Die Steuereinheit verarbeitet das Stellungssignal, um insbesondere ein Halteelement anzusteuern. Für das Halteelement bieten sich, je nach Gestaltung, unterschiedliche Positionen an. Das Halteelement kann sich an dem Prüfkolben oder an dem Servicekolben befinden. Das Halteelement kann sich an dem Prüfgewicht, insbesondere dem Prüfgewichtskörper, und/oder an einer Prüfgewichtsaufnahme befinden. In einer weiteren Ausgestaltung kann sich das Halteelement aber auch an dem Arm, insbesondere an einem Armende, befinden. Ein Halteelement kann nach einem Aspekt eine körperliche Form umfassen, die für den Anschluss, anders gesagt die Ausbildung, einer Halterung vorgesehen ist. In einem Arbeitsbereich der Fließprüfmaschine, der auch als Versuchsbereich bezeichnet werden kann, sind die dem Halteelement zugeordneten Elemente der Fließprüfmaschine durch die Antriebseinheit annäherbar. Das Stellungssignal kann als Interlock für die Schließstellung oder die Freigabestellung dienen, insbesondere in einem Bereich einer Versuchsstrecke, um einen normgemäßen Messbetriebszustand sicherzustellen.

Die Fließprüfmaschine kann eine Zeitkontrolleinheit, die insbesondere eine Stoppuhr umfasst, aufweisen. Die Zeitkontrolleinheit kann als Zeitgeber, z. B. als eine elektronische Zeitkontrolleinheit, wie durch einen Mikrochip der Steuereinheit, oder als Zeitnehmer arbeiten. Die Zeitkontrolleinheit legt Schaltzeiten im Ablauf einer Fließprüfung fest. Schaltzeiten im Ablauf eines einzelnen Prozessschrittes lassen sich bestimmen. Nach einem weiteren Aspekt dient die Zeitkontrolleinheit zur Überwachung einer Taktung. Mit anderen Worten, erkennt die Zeitkontrolleinheit den Zeitpunkt, an dem die Kupplung oder die Halterung zu öffnen ist, und legt den Zeitpunkt fest, an dem die aktuatorische Zusatzkraft auf den Prüfkolben oder auf den Servicekolben durchzuschalten ist. In Zusammenarbeit mit der Wegmesseinrichtung sind von der Zeitkontrolleinheit Fließraten einer Prüfmasse messbar.

Eine andere vorteilhafte Komponente ist eine Heizeinrichtung in der Fließprüfmaschine, wie eine elektrische Heizung, insbesondere eine Widerstandsheizung, z. B. mit einem Heizdraht, oder eine Wirbelstromheizung. Die Heizeinrichtung ermöglicht längs der Prüfkanallänge eine besonders gleichmäßige Erwärmung. Damit ist es möglich, die Fließfähigkeit in einer Prüfmassensäule zu homogenisieren. Eine erste Heizeinrichtung kann um den Prüfkanal angeordnet sein. Eine zweite Heizeinrichtung kann in dem Prüfkolben oder in dem Servicekolben angeordnet sein. Es kann auch eine Düse mit Heizeinrichtung als modular einsetzbares Element vorgesehen sein.

Die Zeitkontrolleinheit und die Heizeinrichtung können vorteilhaft zusammenarbeiten. Die Heizeinrichtung umfasst vorzugsweise eine Temperaturmesseinheit mit einem Temperaturfühler, der Art nach eines digitalen Thermometers. Mit Hilfe der Temperaturmesseinheit ist überprüfbar, ob die Prüfmasse eine vorgebbare Temperatur, wie einen Schmelzpunkt, erreicht hat. Die Zeitkontrolleinheit überwacht eine vorgebbare Anschmelzzeit. Es ist möglich, einen nächsten Prozessschritt erst dann durch die Steuereinheit zuzulassen, wenn ein Freigabesignal der Temperaturmesseinheit und ein Wartezeitablaufsignal der Zeitkontrolleinheit vorliegen. Die Antriebskraft, wie die Gewichtskraft und/oder die aktuatorische Zusatzkraft, wird z. B. über ein Signal an die Kupplungseinheit in Aktion gesetzt. Anders gesagt, idealerweise wird erst dann ein Antriebszustand eingenommen, wenn die Fließprüftemperatur, z. B. für einen thermoplastischen Kunststoff, erreicht ist und die vorgegebene Anschmelzzeit vergangen ist. In dem Antriebszustand sind eine Kraft der Antriebseinheit und die Gewichtskraft parallel gerichtet. Also wird eine Antriebskraft additiv bereitgestellt.

Die Antriebseinheit kann vorteilhafter Weise auch eine Kraft erzeugen, die der Gewichtskraft entgegengerichtet ist, insbesondere wenn die Antriebseinheit in einem zweiten Betriebszustand arbeitet. Ein zweiter Betriebszustand kann z. B. vorliegen, wenn ein Messbetriebszustand beendet ist. Nach einem Aspekt kann ein zweiter Betriebszustand ein Antriebszustand sein. Die Kraft in eine zweite, der Gewichtskraft entgegengesetzte Richtung ist mittels einer Umlenkvorrichtung in der Antriebseinheit erzeugbar. Die Umlenkvorrichtung kann z. B. mindestens ein Schaltventil sein. Beispielsweise ein hydraulisches oder pneumatisches Schaltwechselventil eignet sich als Schaltventil. Die Umlenkvorrichtung kann auch eine Kombination von mehreren hydraulischen und/oder mehreren pneumatischen Schaltwechselventilen umfassen. Das Schaltventil kann als 3/2-Wege-Ventil ausgebildet sein. Ein erster Arbeitsanschluss ist aktiv, wenn die Antriebseinheit zur Gewichtskraft gleichgerichtet arbeitet. Ein zweiter Arbeitsanschluss ist aktiv, wenn die Antriebseinheit entgegen der Gewichtskraft arbeitet. Das Schaltventil kann mit dem Prüfzylinder zusammenarbeiten. Jeweils über einen Arbeitsanschluss, der zwischen Schaltventil und Prüfzylinder liegt, kann ein Druckmittel in einen Arbeitsraum an einem Ende des Prüfzylinders eingeleitet werden. Es ist günstig, wenn die Kraft in der zweiten Richtung an dem Prüfgewicht ansetzt. Die Kraftwirkung kann z. B. auf einen Prüfgewichtskörper aufbringbar sein. Das hat unter anderem den Vorteil, dass das Prüfgewicht durch die Antriebseinheit, insbesondere in sehr kurzer Zeit, wieder in eine Lage über der ersten Stellung verbringbar ist, insbesondere ohne Eingriff eines Benutzers. Steht die Halterung in Freigabestellung, kann der Prüfkolben in der erreichten Stellung verbleiben. In diesem Konfigurationszustand der Fließprüfmaschine, in dem die Halterung in einer Freigabestellung steht, lässt sich die Masse der Prüfgewichte sicher und schnell abnehmen.

Weiter ist es von Vorteil, wenn eine Druckkontrolleinheit vorgesehen ist. Als Druckkontrolleinheit kann beispielsweise ein proportionales Druck-Regelventil fungieren. Ein Versorgungsdruck kann auch digital über eine Regelschleife mittels eines Druckmessgeräts, wie eines digitalen Manometers, abgeregelt werden. Durch die Druckkontrolleinheit ist die aktuatorische Zusatzkraft regelbar. Die aktuatorische Zusatzkraft ist also zumindest proportional zu einer Druckkraft. Es kann auch gesagt werden, die aktuatorische Zusatzkraft folgt dem eingestellten Druck. Die aktuatorische Zusatzkraft kann stufenlos an die Fließfähigkeit der Prüfmasse angepasst werden. Eine Obergrenze der aktuatorischen Zusatzkraft bestimmt sich aus einem Versorgungsdruck für die hydraulischen und/oder pneumatischen Komponenten der Fließprüfmaschine. Vorzugsweise ist die Obergrenze so gewählt, dass der Prüfkolben eine Maximalgeschwindigkeit nicht überschreitet. Die Druckkontrolleinheit kann die Einhaltung einer Druckobergrenze überwachen. Die Druckkontrolleinheit arbeitet vorteilhaft mit einer Steuereinheit, insbesondere mit einer Steuerungselektronik, zusammen. Die Druckkontrolleinheit und die Steuerungselektronik sind über eine elektronische Steuerleitung verbunden. Durch die Druckkontrolle ist eine Regelung der Prüfkolbengeschwindigkeit ausführbar. Eine Druckerhöhung bewirkt - in der Regel - eine Erhöhung der Prüfkolbengeschwindigkeit, insbesondere wenn die Druckerhöhung in dem Prüfmaterial phasenübergangsfrei erfolgt oder auch insbesondere wenn eine Erhöhung der Prüfkolbengeschwindigkeit keine Strömungsturbulenzen erzeugt oder verstärkt (z. B. rein laminare Strömungen). Die Steuereinheit und die Druckkontrolleinheit können eine Regelung für eine Druckkraft, insbesondere auf einen konstanten Betrag, bilden. Die Regelung kann eine konstante Prüfkolbengeschwindigkeit ergeben. Es ist auch möglich, eine konstante Prüfkolbengeschwindigkeit durch Regelung der Druckkraft zu erhalten. Ein maximaler Druck ist vorgebbar, mit dem der Prüfkolben durch die Antriebseinheit beaufschlagbar ist. Eine Druckquelle kann eine hydraulische Druckquelle, eine pneumatische Druckquelle oder eine elektrische oder mechanische Druckquelle, jeweils zur Druckerzeugung, sein, die eine auf den Prüfkolben beaufschlagbare Druckkraft bereitstellt.

Eine der zweiten Stellungen des Prüfkolbens kann als Arbeitsbereichsendstellung, die auch als Versuchsbereichsendstellung bezeichnet werden kann, vorliegen. Die Versuchsbereichsendstellung befindet sich an einer Abströmseite des Prüfkanals. Eine Öffnung an der Abströmseite des Prüfkanals kann als Düse oder Auspressdüse bezeichnet werden. Anders gesagt, eine Begrenzung des Prüfkanals ist vorzugsweise eine Düse. Der Arbeitsbereich des Prüfkolbens endet an der Düse. Der Prüfkolben kann die Versuchsbereichsendstellung nicht überschreiten, weil die Düse, insbesondere ein Düseneinsatz, einen Weg des Prüfkolbens begrenzt. Nach einem Aspekt lässt sich auch sagen, die Düse kann eine Engstelle des Prüfkanals sein, wie z. B. eine Bohrung mit einem kleineren Durchmesser als der Prüfkanal. Die Düse kann auch als Düseneinsatz des Prüfkanals ausgebildet sein, sodass gegebenenfalls eine verstopfte Düse schnell zu ersetzen ist. Ein entnehmbarer, vorzugsweise ausstoßbarer oder zumindest verlagerbarer, modularer Düseneinsatz erleichtert die Durchführung eines Reinigungsverfahrens für den Prüfkanal. Die Düse weist mindestens einen Durchmesser, z. B. einen Eingangsdurchmesser und einen Ausgangsdurchmesser, die insbesondere durch eine Düsenkanallänge zueinander beabstandet sind, auf. In einer vorteilhaften Weiterbildung ist die Öffnung mit einem Verschluss oder mit einer Schneidevorrichtung, die z. B. einen Schneiddraht umfasst, verbunden. In einer Verschlussstellung kann z. B. der Ausgangsdurchmesser überdeckt sein.

Eine der ersten Stellungen, eine Anfahrstellung, kann als Prüfbereichsendstellung vorgesehen sein. Die Prüfbereichsendstellung kann auch als eine Nach-Prüfbereichsstellung bezeichnet werden, weil die Prüfbereichsendstellung insbesondere einen normungsgemäßen Mindestabstand zur Düse markiert. Anders gesagt, kann eine erste Stellung zwischen einem der Düse zugeordneten Ende der Versuchsstrecke und der Düse angeordnet sein. Die Versuchsbereichsendstellung befindet sich außerhalb der Versuchsstrecke. Der Prüfkolben passiert aus der Versuchsstrecke kommend eine Teilstrecke des Prüfkanals, die auch als Nach-Prüfbereich oder als Prüffolgestrecke bezeichnet wird. Die Prüfbereichsendstellung kann als Ende der Versuchsstrecke und als Beginn der zweiten Teilstrecke angesehen werden. Die zweite Teilstrecke bis zur Versuchsbereichsendstellung ist gemäß einer vorteilhaften Ausführungsform kürzer als 2 cm. Die Versuchsbereichsendstellung kann - mit anderen Worten - auch als eine Ruhestellung bezeichnet werden. Nach einer präzisen Fließprüfung im Prüfkanal verbliebenes Prüfmaterial ist effizient ausstoßbar.

Eine der ersten Stellungen, vorzugsweise eine der Einfüllöffnung des Prüfkanals nächstliegende erste Stellung des Prüfkolbens, kann als Versuchsbeginnstellung bezeichnet werden. Der Prüfkolben befindet sich zu Beginn einer Fließprüfung in der Versuchsbeginnstellung, die insbesondere eine Ruhestellung nach einer Verdichtung der Prüfmasse ist. Eine der zweiten Stellungen, vorzugsweise die im Prüfkanal der Düse nächstliegende, insbesondere dezidierte bzw. ausgewiesene, zweite Stellung, kann als Versuchsendstellung bezeichnet werden. Am Ende der Fließprüfung befindet sich der Prüfkolben in der Versuchsendstellung. Wenn nur eine Versuchsstrecke vorgesehen ist, können die Versuchsendstellung und die Prüfbereichsendstellung zusammenliegen.

Zwischen der Versuchsbeginnstellung und der Versuchsendstellung sind gemäß einer vorteilhaften Weiterbildung eine dritte Stellung und eine vierte Stellung vorgesehen. Durch die dritte und die vierte Stellung kann sich der Prüfkolben, ohne an den Stellungen zu stoppen, hindurchbewegen. Es kann auch gesagt werden, dass die dritte und die vierte Stellung transitorische Stellungen oder Durchgangsstellungen darstellen. Die dritte Stellung ist eine Prüfstartstellung. Sobald sich der Prüfkolben in der Prüfstartstellung befindet, beginnt der Messvorgang, insbesondere erfolgt eine erste Zeitnahme. Die vierte Stellung ist eine Prüfendstellung. In der Prüfendstellung ist der Messvorgang beendet, wobei insbesondere eine zweite Zeitnahme erfolgt. Zwischen der Prüfstartstellung und der Prüfendstellung liegt die Versuchsstrecke, eine der Teilstrecken des Prüfkanals. Die aktuatorische Zusatzkraft ist vor dem Erreichen der dritten Stellung von dem Prüfkolben trennbar. Die Stellung des Prüfkolbens für die Trennung ist eine zweite Stellung, eine Endfahrstellung, die von der dritten Stellung beabstandet ist. In transitorischer, kontinuierlicher Bewegung des Prüfkolbens erfolgt eine Relaxation der Prüfmasse bei einer niedrigeren Prüfkolbengeschwindigkeit bis zur dritten Stellung. Die vor dem Erreichen der zweiten Stellung höhere Prüfkolbengeschwindigkeit ermöglicht einen schnelleren Ausstoß eines Vorlaufs von Prüfmaterial. Damit ist eine schnellere Taktung in der Fließprüfung möglich. Mit anderen Worten, die Stellung sind jeweils, insbesondere gedachte, Punkte entlang des Prüfkanals, die eine Bedeutung für die Ausführung des Versuchs haben, wie z. B. ein Zustandswechsel in der Fließprüfmaschine, wie z. B. eine Zeitnahmeposition, wie z. B. eine Position, die mit einer Kraft- oder sonstigen Beaufschlagungsänderung einhergeht.

Die Versuchsstrecke kann je nach zu testendem Material z. B. eine Länge zwischen 5 mm (Millimeter) und 100 mm aufweisen. Vorzugsweise sind Längen zwischen 6 mm und 26 mm vorgesehen. Bei niedrigen zu erwartenden Schmelze-Volumenfließraten, bzw. bei großer Viskosität, sind kürzere Versuchsstrecken vorteilhaft, bei einer hohen zu erwartenden Schmelze-Volumenfließraten sind längere Versuchsstrecken vorteilhaft, um gute Messergebnisse zu erhalten.

Die Richtung der aktuatorischen Zusatzkraft ist, zumindest in einem der Betriebszustände, ausgehend von der ersten Stellung des Prüfkolbens zu der Gewichtskraft gleichgerichtet. Die in einer betragsmäßigen Größe vorgebbare und einstellbare aktuatorische Zusatzkraft ist in der Richtung umkehrbar. Die Umkehrung der Kraft bewirkt eine Abbremsung des Prüfkolbens. Es ist möglich, die aktuatorische Zusatzkraft betragsmäßig zu erhöhen, so dass der Prüfkolben durch Kompensation der Gewichtskraft zur Ruhe kommt. Der Auspressweg ist bei einer Konfiguration die Teilstrecke des Prüfkanals, die der Prüfkolben nach dem Durchgang durch die vierte Stellung, also nach Ende der Zeitnahme bei der Fließprüfung, bis zu der Versuchsendstellung durchfährt. Auf dem Auspressweg wird der Prüfkanal durch einen Vorschub des Prüfkolbens zumindest teilweise geleert. Durch eine Abbremsung vor einem Ende des Auspresswegs findet eine verlangsamte Annäherung an die zweite Stellung statt. Der Prüfkolben kommt z. B. gezielt an dem Ende des Arbeitsbereichs zu einem Stillstand. Druckspitzen, die sich schädlich auf Fließprüfungen oder auf die Fließprüfmaschine auswirken können, werden vermieden. Es muss kein Sicherheitsabstand mit verbleibendem Prüfmaterial auf dem Weg des Prüfkolbens zur Düse eingehalten werden. Es ist auch möglich, mit der aktuatorischen Zusatzkraft bei Fließprüfungen von besonders niederviskosen Fluiden eine Teilkompensation der Gewichtskraft, die insbesondere von einer Masse des Prüfkolbens herrührt, vorzunehmen.

Der Auspressweg ist einem, insbesondere zweiten, Auspressschritt, wie einem Reinigungsschritt, des Messverfahrens für eine Fließprüfung, also einer Schmelzviskositätsprüfung, zugeordnet. Nach einem zweiten Aspekt kann ein Auspressweg auch einem Messvorbereitungsschritt, welcher somit einen ersten Auspressschritt darstellt, zugeordnet sein.

Die aktuatorische Zusatzkraft aus dem Prüfzylinder ist zu der Gewichtskraftrichtung gleichgerichtet, bis der Prüfkolben einen Kraftwendepunkt erreicht. Ab dem Kraftwendepunkt wirkt eine der Gewichtskraft des Prüfgewichts entgegengerichtete Kraft auf den Prüfkolben. Durch eine Kolbenstellungsmesseinrichtung, die auch Kolbenpositionsmesseinrichtung genannt werden kann, ist die Stellung des Prüfkolbens erkennbar, in der der Kraftwendepunkt liegt. Mit anderen Worten, ist in dem Auspressschritt eine Kraftrichtung, insbesondere mittels Ansteuerung des Prüfzylinders, umkehrbar. Ein Kraftwendepunkt befindet sich vorteilhafterweise in einem Bereich des Prüfkanals, in dem die Druckfläche des Prüfkolbens von der Düse zwischen 0,1 mm und 20 mm entfernt angeordnet ist. Durch die Kolbenstellungsmesseinrichtung ist der Weg eines Kolbens erfassbar.

In einer besonders vorteilhaften Ausgestaltung der Fließprüfmaschine ist ein Arm vorgesehen. Der Arm kann zwischen der Kupplung und der Antriebseinheit angeordnet sein. In einer vorteilhaften Ausführungsform ist der Arm zwischen der Halterung und dem Prüfzylinder eingesetzt, womit sich die Halterung in Bezug auf den Prüfzylinder besonders gut positionieren lässt. Der Arm kann sich zumindest bereichsweise in einer Radialrichtung bezogen auf den Prüfzylinder erstrecken. Der Arm ist durch die Antriebseinheit verfahrbar. Der Arm ist so gestaltet, insbesondere dermaßen dimensioniert, dass er das Prüfgewicht, insbesondere ein maximales Prüfgewicht, aufnehmen bzw. tragen kann. Anders gesagt, der Arm ist ausgelegt, um das Prüfgewicht zu tragen. Der Arm hat zwei Armenden. An einem ersten Armende befindet sich eine Durchgangsöffnung. Die Durchgangsöffnung kann eine, insbesondere zylinderförmige, durchgängige Ausnehmung sein. Die Durchgangsöffnung ist um den Prüfkolben, genauer gesagt um den Prüfkolbenkopf, anordnenbar. Nach einem anderen Aspekt ist der Arm längs des Prüfkolbens, insbesondere längs eines außerhalb des Prüfkanals befindlichen Bereichs des Prüfkolbens, auf einer gedachten Ebene positionierbar. Der Prüfkolben ist berührungsfrei in der Durchgangsöffnung des Arms anordnenbar. Es kann auch gesagt werden, dass sich die Durchgangsöffnung von einer Prüfkanalseite des Arms bis zu einer Prüfgewichtseite des Arms erstreckt. Eine Oberseite des ersten Armendes kann einen Auflagebereich und vorzugsweise einen Zentrierbereich für ein Prüfgewicht, insbesondere für einen ersten Prüfgewichtskörper, aufweisen. Mindestens ein Haltebereich für den Prüfkolbenkopf kann an einer Unterseite, also einer dem Prüfkanal zugewandten Seite, des ersten Armendes vorliegen. In dem Haltebereich kann die Halterung angeordnet sein. Eine räumlich besonders günstige Konfiguration liegt vor, wenn der Arm einen Aktuator aufweist, durch den mindestens zwei Stellungen der Halterung schaltbar sind. Der Haltebereich kann - in einer vorteilhaften konstruktiven Gestaltung - als ein Teil der Halterung ausgebildet sein, und insbesondere eine abstützende Lagerung für die Halterung bieten.

Der Arm ist vorzugsweise drehbar gelagert. Zumindest in einer Endstellung, insbesondere einer Endstellung des Prüfzylinders oder des Prüfkolbens, wie die Versuchsbeginnstellung, also die Stellung zum Versuchsstart, und/oder die Versuchsendstellung, ist eine Schwenkbarkeit des Arms vorteilhaft. Während des Versuchs ist der Arm nicht schwenkbar. Ein Drehgelenk des Arms ist vorzugsweise festsetzbar, um eine geradlinige Führung des Arms durch die Antriebseinheit sicherzustellen. Die Schwenkbarkeit liegt insbesondere in einer Ebene vor, die sich vorzugsweise senkrecht bezogen auf die Richtung der Gewichtskraft erstreckt. Der schwenkbare Arm erleichtert die Durchführung von Wartungsarbeiten, wie eines manuellen Zugriffs auf den Prüfkolben für eine Entnahme des Prüfkolbens oder wie während einer Befüllung des Prüfkanals mit Prüfmaterial. Es darf hervorgehoben werden, das Prüfgewicht ist schneller bereitstellbar. Mit Hilfe des Arms kann vorteilhafterweise ein erstes Prüfgewicht durch ein zweites Prüfgewicht ersetzt werden. Prüfgewichtskörper können in einem Prüfgewichtsmagazin vorliegen. Mittels Arm ist ein Prüfgewichtskörper des Prüfgewichts in dem Prüfgewichtsmagazin absetzbar oder ein Prüfgewichtskörper aus dem Prüfgewichtsmagazin aufnehmbar, insbesondere dem schon vorhandenen Prüfgewicht, das auf dem Prüfkolben angeordnet sein kann, hinzufügbar.

Vorzugsweise ist ein zweites Ende des Arms, ein zweites Armende, mit der Antriebseinheit verbunden. Die Verbindung kann über ein Drehgelenk erfolgen. Das zweite Armende kann in einem Winkelbereich um eine Achse drehbar sein. Es ist möglich die Drehbarkeit des Arms durch die Antriebseinheit, z. B. mittels Schrittmotor, vorzusehen.

Gemäß einer weiteren vorteilhaften Ausgestaltung ist an der Antriebseinheit mindestens ein Anschlag vorhanden. Der Anschlag kann als ein Endkontaktschalter ausgeführt sein. Der Anschlag kann beispielsweise dem zweiten Ende des Armes zugeordnet sein. Der Anschlag bietet eine Positionsfestlegung des Armes, insbesondere der Durchgangsöffnung des Armes, in Bezug auf den Prüfkanal. Der Anschlag kann auch eine Positionsüberwachung ermöglichen. Der Anschlag begrenzt nach einem Aspekt eine Drehbarkeit des Armes. Der Anschlag kann nach einem weiteren Aspekt einer zweiten Stellung zugeordnet sein. Beispielsweise kann der Anschlag einer Endstellung, wie der Versuchsbereichsendstellung oder der Versuchsendstellung, anders gesagt, dem Prüfkolben oder auch dem Prüfzylinder, zugeordnet sein. Vorzugsweise arbeitet der Anschlag als ein Schalter der Antriebseinheit, um einen präzisen Versuchsablauf sicherzustellen. Wenn sich der Prüfkolben in der Endstellung befindet, ist, z. B. von der Steuereinheit, die Halterung, insbesondere ein Aktuator der Halterung, in die Freigabestellung schaltbar. In der Anschlagsstellung ist der Prüfkolben von der Antriebseinheit abkoppelbar.

Es ist möglich, als einen ersten Anschlag einen Drehbarkeitsbegrenzungsanschlag vorzusehen. Der Drehbarkeitsbegrenzungsanschlag umfasst vorzugsweise einen Anschlagsschalter, der mit einer Gelenkfeststelleinrichtung verbunden ist. Der erste Anschlag erlaubt das Schalten einer Fahrtrichtung der Antriebseinheit in Richtung der Gewichtskraft. Als ein zweiter Anschlag kann ein Endstellungsanschlag vorgesehen sein. Der zweite Anschlag kann als ein, vorzugsweise zwei, in der Steuereinheit vorgehaltener Kolbenstellungsextremwert der Kolbenstellungsmesseinrichtung oder der Wegmesseinrichtung realisiert sein. Der zweite Anschlag sperrt die Fahrtrichtung der Antriebseinheit, insbesondere in Richtung der Gewichtskraft.

Für die Durchführung von Fließprüfungen zur Schmelzviskositätsprüfung hat es sich als vorteilhaft erwiesen, wenn eine Bewegungsrichtung des Prüfkolbens, insbesondere ausgehend von der ersten Stellung, beibehalten wird. Die Bewegungsrichtung bleibt konstant, bis ein Anschlag erreicht ist. Als Alternative kann die Bewegungsrichtung auch unverändert bleiben, bis sich der Prüfkolben in einer Endstellung befindet. Die Endstellung kann beispielsweise die Versuchsbereichsendstellung oder die Versuchsendstellung sein. Die Bewegungsrichtung wird durch die aktuatorische Zusatzkraft unterstützt. Eine Beschleunigung wirkt auf den Prüfkolben. Die Beschleunigung kann zusätzlich zur Gravitation erfolgen.

Insbesondere im Anschluss an die beschleunigte Bewegung kann der Prüfkolben durch die aktuatorische Zusatzkraft abgebremst werden. Das Abbremsen des Prüfkolbens kann dadurch eingeleitet werden, dass die Antriebseinheit umgeschaltet wird. Mit Hilfe eines Bewegungselements der Antriebseinheit kann ein Umstellen einer Antriebsrichtung vorgenommen werden, insbesondere unter Beibehaltung der Bewegungsrichtung. Mit anderen Worten, auch während des Abbremsens, zumindest während eines Bruchteils einer Abbremszeit, verlässt noch Prüfmaterial den Prüfkanal durch eine Düse.

Die aktuatorische Zusatzkraft weist vorzugsweise in einem Messbetriebszustand einen konstanten Betrag auf. Während eines Auspressschrittes ist es möglich, eine Kraftrichtung der aktuatorischen Zusatzkraft zu ändern. Als geänderte Kraftrichtung ist eine umgekehrte Kraftrichtung vorgesehen. Die Änderung kann durch Betragsänderung der Zusatzkraft stetig erfolgen. Die Zusatzkraft wirkt auf den Kolben, vorteilhafter Weise auf den Prüfkolben, um eine möglichst gleichmäßige Kolbenbewegung zu erhalten. Die Zusatzkraft kann auch z. B. auf den Reinigungskolben angewendet werden, wobei während eines Auspressschrittes eine Umkehrung der Kraftrichtung möglich ist, insbesondere um den Kolben in einem Endstellungsbereich aufzufangen. Die Kraftrichtung der aktuatorischen Zusatzkraft gleicht der Richtung der Gewichtskraft, bis ein Kraftwendepunkt erreicht wird, an dem die aktuatorische Zusatzkraft auf den Kolben eine Änderung erfährt. In einem Bereich um den Kraftwendepunkt wird die aktuatorische Zusatzkraftrichtung der Gewichtskraftrichtung entgegengerichtet. Es resultiert eine Änderung der Bewegung, insbesondere im Sinne einer Bewegungsrichtung. Mit einer Kolbenpositionsmesseinrichtung ist vorteilhaft das Erreichen des Kraftwendepunktes überprüfbar. Am Kraftwendepunkt ist z. B. der Prüfkolben für eine Wende der Richtung der aktuatorischen Zusatzkraft bereit. Damit ist sichergestellt, dass die Kraftrichtung in einem Prüfverfahren oder auch in einem Reinigungsschritt korrekt eingestellt wird.

Wenn der Messvorbereitungsschritt beendet ist, wird der Messschritt eingeleitet. Eine Verbindung zwischen dem Prüfkolben und einer Antriebseinheit, die einer Übertragung der aktuatorischen Zusatzkraft dient, wird geöffnet. Vorzugsweise nach einer Deaktivierung der Zusatzkraft wird der Kraftschluss getrennt, um eine möglichst freie Bewegung zu erhalten. Die Trennung wird vorteilhaft mittels einer Kupplung ausgeführt, um Kraftspitzen zu vermeiden. Nach der Trennung kann noch eine Relaxationszeit vor dem Beginn des Messschritts vorgesehen sein. Die Relaxationszeit ist eine Zeit freier Bewegung des Prüfkolbens unter dem Prüfgewicht. Die Relaxationszeit ist so bemessen, dass eine gleichförmige Prüfkolbenbewegung erlangt wird. Anders gesagt, kann sich die Prüfmasse, belastet durch das Prüfgewicht, entspannen. Die Relaxationszeit kann auch vorteilhaft dazu benutzt werden, einen Wärmefluss in einem Prüfraum abzuwarten. Ein Prüftemperaturprofil des Bereichs des Prüfraums, der dem Prüfkanal zugeordnet ist, insbesondere ein Temperaturprofil in der für die Messung vorgesehenen Prüfmasse, die in dem Messvorbereitungsschritt in eine Nähe der Düse verschoben wurde, kann sich räumlich vergleichmäßigen. Der Aspekt der Vergleichmäßigung besagt, dass eine Temperatur räumlich homogen in der Prüfmasse vorliegt, wobei die Temperatur vorzugsweise gleich einer Düsentemperatur ist. Die Vergleichmäßigung kann unter konstanter Zufuhr von Wärme aus einer Heizeinrichtung erfolgen. Nach Ablauf der Relaxationszeit sind die Prüfbedingungen besonders verlässlich. Eine Zeitnahme stellt den Beginn des Messschritts dar. Während der Messung befördert ein Prüfgewicht die Prüfmasse, z. B. durch die Düse. Eine Gewichtskraft treibt über den Prüfkolben die Prüfmasse an. Der Messschritt wird durch eine zweite Zeitnahme beendet. Es ist möglich, Zwischenzeiten zu erfassen, um den Messschritt auf zeitliche Entwicklungen hin zu analysieren, z. B. um chemische Änderungen der Prüfmasse zu erkennen.

Aus einer Bewegung des Prüfkolbens wird mindestens eine Materialeigenschaft der Prüfmasse bestimmt. Die Auswertung von Daten, die aus einem Messschritt erhalten werden, erfolgt in einer Recheneinheit. Die Recheneinheit kann ein Teil der Fließprüfmaschine sein. Es ist vorteilhaft, wenn die Recheneinheit mit der Steuereinheit der Prüfmaschine zusammenarbeitet, um schnell zu Ergebnissen zu kommen. Der Messschritt kann eine Weg-Zeit-Messung umfassen. Der Weg ist eine Wegstrecke des Prüfkanals, die ein Volumen der Prüfmasse, wie ein Prüfvolumen, enthält. Das Prüfvolumen wird in einem Zeitintervall, z. B. durch die Düse, gepresst. Es kann auch ein Prüfmassenabschnitt gekappt werden, welcher die Prüfmasse des Messschritts umfasst. Durch eine Wägung kann die ausgetretene Prüfmasse des Prüfmaterials erhoben bzw. bestimmt werden, die insbesondere nicht dem Auspressschritt, also auch nicht dem Messvorbereitungsschritt zuzuordnen ist. Eine Wägeeinrichtung kann ein Teil der Fließprüfmaschine sein. Die Wägeeinrichtung arbeitet vorzugsweise mit der Recheneinheit zusammen, um noch schneller zu Ergebnissen zu kommen. Damit kann mindestens eine Materialeigenschaft, wie eine Viskosität der Prüfmasse oder eine Fließgeschwindigkeit, z. B. die Schmelze-Massefließrate bzw. die Schmelze-Volumenfließrate, insbesondere bei einer konstanten Temperatur, bestimmt werden. Die Schmelze-Massefließrate bzw. die Schmelze-Volumenfließrate dienen als ein Maß der Viskosität einer Schmelze eines Kunststoffs. Rückschlüsse auf die mittlere Anzahl von Monomereinheiten in einem Polymermolekül eines Prüfmaterials sind möglich.

Das Reinigungsverfahren weist vorteilhafterweise mindestens einen Teilschritt, vorzugsweise eine Folge von Teilschritten, auf. Durch eine Verwendung von Teilschritten lässt sich eine Taktung bei einer Fließprüfung besonders günstig und zeiteffizient strukturieren. Einer oder mehrere der folgenden, insbesondere weiteren, Teilschritte können vorgesehen sein.

In einem Teilschritt, z. B. einem ersten Teilschritt, erfolgt das Entkoppeln der Antriebseinheit durch einen Aktuator. Die Antriebseinheit und der Prüfkolben werden voneinander getrennt. Die Trennung erfolgt durch Ansteuerung des Aktuators.

Ein anderer Teilschritt, z. B. ein zweiter Teilschritt, umfasst das Herausziehen des Prüfkolbens aus dem Prüfkanal. Das Herausziehen kann durch die Antriebseinheit automatisch oder manuell durch einen Maschinenbediener erfolgen.

Ein anderer Teilschritt, z. B. ein dritter Teilschritt, umfasst das Ankoppeln des Reinigungskolbens an die Antriebseinheit. Beim Ankoppeln erfolgt insbesondere eine Ausrichtung des Reinigungskolbens bezüglich einer Zentralachse des Prüfkanals.

Ein anderer, z. B. vierter, Teilschritt umfasst das Einfahren des Reinigungskolbens in den Prüfkanal. Die Antriebseinheit bewegt den Reinigungskolben in den Prüfkanal hinein, vorzugsweise bis die Düse erreicht wird.

Ein anderer, z. B. fünfter, Teilschritt umfasst das Ausfahren des Reinigungskolbens aus dem Prüfkanal. Das Ausfahren erfolgt z. B. mittels Antriebseinheit. Es ist auch möglich, das Ausfahren zumindest auf einer Teilstrecke insbesondere als manuelles Herausziehen zu realisieren. Das Ausfahren kann z. B. den auspressseitigen Auswurf eines Reinigungsmittels, wie eines Reinigungsstopfens oder Reinigungstuchs, aus dem Prüfkanal umfassen.

Ein anderer, z. B. sechster, Teilschritt umfasst das Entkoppeln des Reinigungskolbens von der Antriebseinheit. Das Entkoppeln kann über einen Aktuator veranlasst werden.

Ein anderer, z. B. siebter, Teilschritt umfasst das Ankoppeln des Prüfkolbens an die Antriebseinheit. Das Ankoppeln ist z. B. durch einen Aktuator ausführbar.

Ein anderer, z. B. achter, Teilschritt umfasst das Fahren des Prüfkolbens in dem Prüfkanal. Das Fahren kann mittels Antriebseinheit erfolgen. Vorzugsweise wird der Prüfkolben bis zu einer Düse gefahren.

Ein weiterer Teilschritt, z. B ein zwischen dem siebten Teilschritt und dem achten Teilschritt ausführbarer Teilschritt, umfasst das Befüllen des Prüfkanals mit einem Prüfmaterial, insbesondere mit einer Prüfmasse, die, vorzugsweise zur Reinigung, z. B. in einem Zerkleinerer, einer Waschvorrichtung, einer Mischvorrichtung oder einem Exsikkator vorbehandelt wurde. Es ist auch möglich z. B. ein Reinigungsgranulat oder eine Reinigungsflüssigkeit zwischen Teilschritten in den Prüfkanal einzufüllen.

Ein Kolben, wie ein Prüfkolben oder ein Servicekolben, kann aus einem Kolbenmagazin aktuatorisch aufgenommen oder aktuatorisch in dem Kolbenmagazin abgesetzt werden. Insbesondere kann der Kolben an einem Arm mit der Antriebseinheit zwischen Prüfkanal und Kolbenmagazin bewegt werden. Das Reinigungsverfahren kann auch eine Sequenz von zu wiederholenden Teilschritten umfassen.

Während der Durchführung eines mit dem Messverfahren verbundenen oder anschließenden Reinigungsschritts ist es von Vorteil, wenn die Temperatur auf einem vorgegebenen Temperaturwert gehalten wird. Eine Temperaturkontrolle an der Fließprüfmaschine stellt sicher, dass eine Fließtemperatur des Prüfmaterials vorliegt. Die Temperaturkontrolle ist z. B. durch mindestens einen Temperatursensor, insbesondere an dem Prüfkanal, ausführbar. Die Wärmekapazität der Fließprüfmaschine, insbesondere eines Prüfkanalzylinders, ist vorzugsweise ausreichend, sodass während einer Versuchszeit die Prüfmasse fließfähig bleibt.

Günstig für einen zumindest teilautomatischen Betrieb einer Fließprüfmaschine ist es, wenn eine Steuereinheit vorgesehen ist. Die Steuereinheit kann z. B. einen Prüfzylinder oder auch einen Aktuator einer Halterung ansteuern. Die Steuereinheit kann auch einen Arm zu einem Prüfgewichtmagazin oder zu einem Kolbenmagazin führen. Besonders vorteilhaft ist es, wenn die Steuereinheit die aktuatorische Zusatzkraft dosiert. Durch die Steuereinheit wird eine aktuatorische Zusatzkraft, eine Reinigungskraft, auf den Reinigungskolben angewendet. Es ist z. B. möglich, an einem durch eine Verunreinigung in einem Prüfkanal vorliegenden Bewegungshindernis, insbesondere bei erkannter Bewegungshinderung, die auf den Reinigungskolben ausgeübte, aktuatorische Zusatzkraft durch die Steuereinheit zu erhöhen, bis das Bewegungshindernis beseitigt ist. Nach einem anderen Aspekt der Steuereinheit kann der Reinigungskolben, der z. B. einer Kraft eines Prüfgewichts oder eines Reinigungsgewichts, also einer Reinigungsgewichtskraft, unterliegt, insbesondere vor dem Erreichen einer Düse abgebremst werden. Besonders vorteilhaft ist auch eine Kontrolle der aktuatorischen Zusatzkraft durch die Steuereinheit für die Anwendung auf den Prüfkolben oder den Verdichtungskolben. Eine Steuerung der aktuatorischen Zusatzkraft erfolgt anforderungsgemäß sequentiell. Innerhalb der einzelnen Schritte des Reinigungsverfahrens, anders gesagt für die Ausführung eines Reinigungsschritts oder eines Teilschritts, ist die aktuatorische Zusatzkraft steuerbar und insbesondere variierbar. Zum Beispiel kann auf den Reinigungskolben beim Einfahren in den Prüfkanal eine im Betrag geringere Zusatzkraft aufgebracht werden, als beim Ausfahren aus dem Prüfkanal.

Der Prüfkanal ist besonders formbeständig, wenn er in einem zylinderförmigen Körper angeordnet ist. Druckkräfte in dem Prüfzylinder können damit nur eine radialsymmetrische Verformung bewirken, die auch bei einer geringen Masse des Körpers so gering ist, dass Prüfergebnisse unbeeinträchtigt bleiben. Der Prüfkanal ist vorzugsweise unnachgiebig in Bezug auf eine Antriebseinheit angeordnet. Die Einfüllöffnung kann einen konischen Kanal aufweisen, womit ein schnelles Befüllen des Prüfkanals begünstigt und insbesondere das Einsetzen eines Kolbens erleichtert wird. Zwischen der Düse und der Wägeeinrichtung ist vorzugsweise ein schaltbarer Direktionierkanal vorgesehen, der eine, vorzugsweise lotusartige, abweisende Oberflächenbeschaffenheit aufweist. In einer Schaltstellung des Direktionierkanals werden Abschnitte des Prüfmaterials, die durch Auspressschritte entstanden sind, einem Sammelbehälter zugeführt. In einer zweiten Schaltstellung wird mindestens ein Abschnitt der Prüfmasse der Wägeeinrichtung zugeführt. Damit lassen sich besonders genaue Wägungen der Prüfmasse ohne Zeitverlust durchführen.

Eine erfindungsgemäße Fließprüfmaschine ist auch für Versuche zur Materialprüfung einsetzbar, die eine Weiterentwicklung, insbesondere ausgehend von bestehenden Normen, darstellen. Aufgrund der zahlreichen Varianten und Kombinationsmöglichkeiten ergeben sich auch vorteilhafte Ausführungsformen der beschriebenen Fließprüfmaschine sowie Versuchsvarianten, insbesondere durch ihre Steuerung, die jenseits bzw. als Weiterentwicklung der Normen anzusehen sind. Beispielsweise lassen sich mit variablen oder variierbaren Kraftprofilen Prüfmassen durch austauschbare Düsen auspressen. Von (Einzel-)Versuch zu (Einzel-)Versuch lassen sich die Materialeigenschaften nach und nach präziser bestimmen. Fehlereinflüsse sind, insbesondere mithilfe der Steuereinheit, herausrechenbar bzw. eliminierbar.

Die zuvor dargestellten Kombinationen und Ausführungsbeispiele lassen sich auch in zahlreichen weiteren Verbindungen und Kombinationen betrachten.

### Figurenkurzbeschreibung

Die vorliegende Erfindung kann noch besser verstanden werden, wenn Bezug auf die beiliegenden Figuren genommen wird, die beispielhaft besonders vorteilhafte Ausgestaltungsmöglichkeiten darlegen, ohne die vorliegende Erfindung auf diese einzuschränken, wobei
Figur 1 eine Seitenansicht einer Fließprüfmaschine,
Figur 2 einen vergrößerten Ausschnitt aus Figur 1, der das Armende und das Prüfgewicht umfasst,
Figur 3 einen vergrößerten Ausschnitt aus Figur 1, der die Düse an der Abströmseite des Prüfkanals umfasst,
Figur 4 einen vergrößerten Ausschnitt aus Figur 1, in Querschnittsdarstellung, mit Prüfgewicht und Abströmseite des Prüfkanals in einem Messvorbereitungsschritt,
Figur 5 eine weitere Ausführungsform einer Fließprüfmaschine, dargestellt in einem Ausschnitt ähnlich zu Figur 4, die sich in einem Messschritt befindet,
Figur 6 die Fließprüfmaschine aus Figur 1 mit einem Reinigungskolben,
Figur 7 den Ausschnitt entsprechend Figur 4 der Fließprüfmaschine mit dem Reinigungskolben aus Figur 6 in einem Reinigungsverfahren mit durch den Prüfkanal hindurchgeschobenem Reinigungskolben und
Figur 8 einen schematischen Ablauf eines Fließprüfverfahrens mit Reinigungsverfahren zeigt.

### Figurenbeschreibung

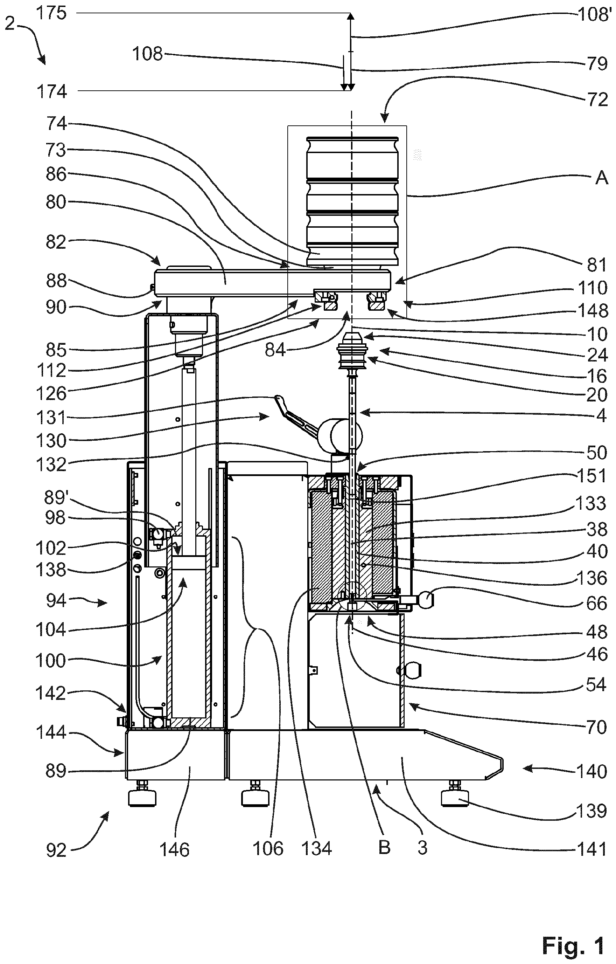

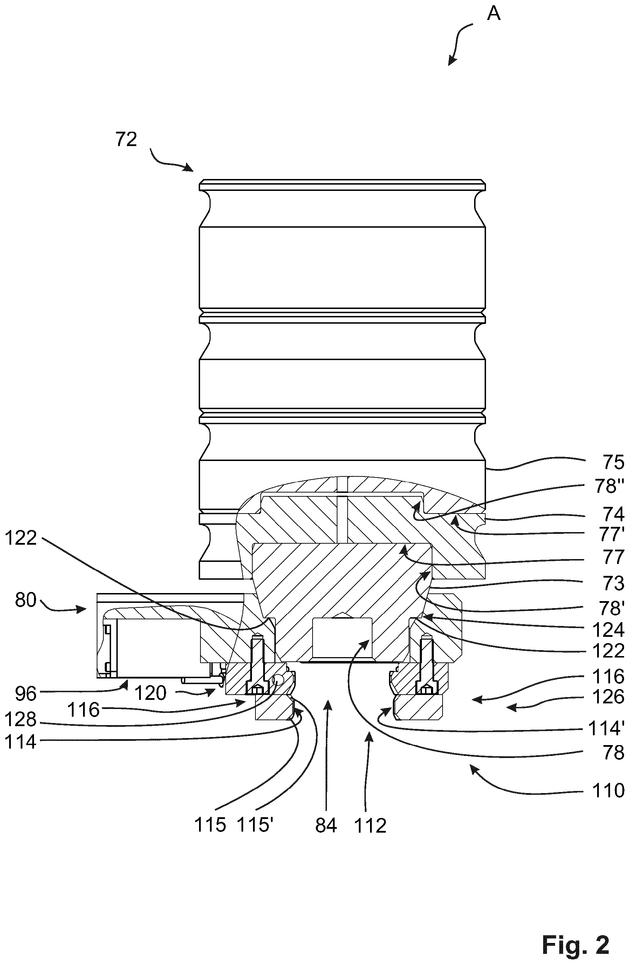

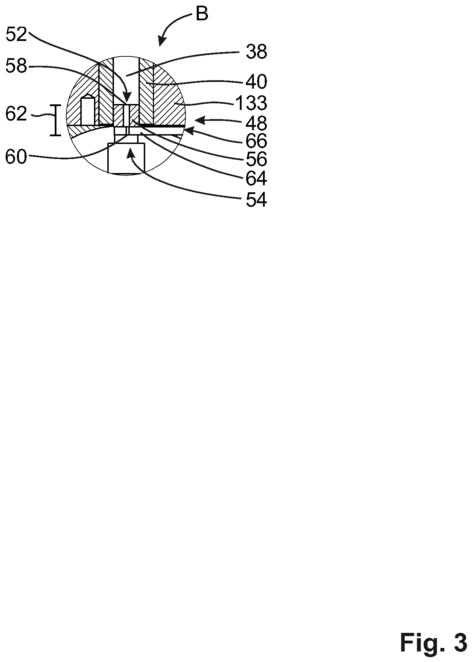

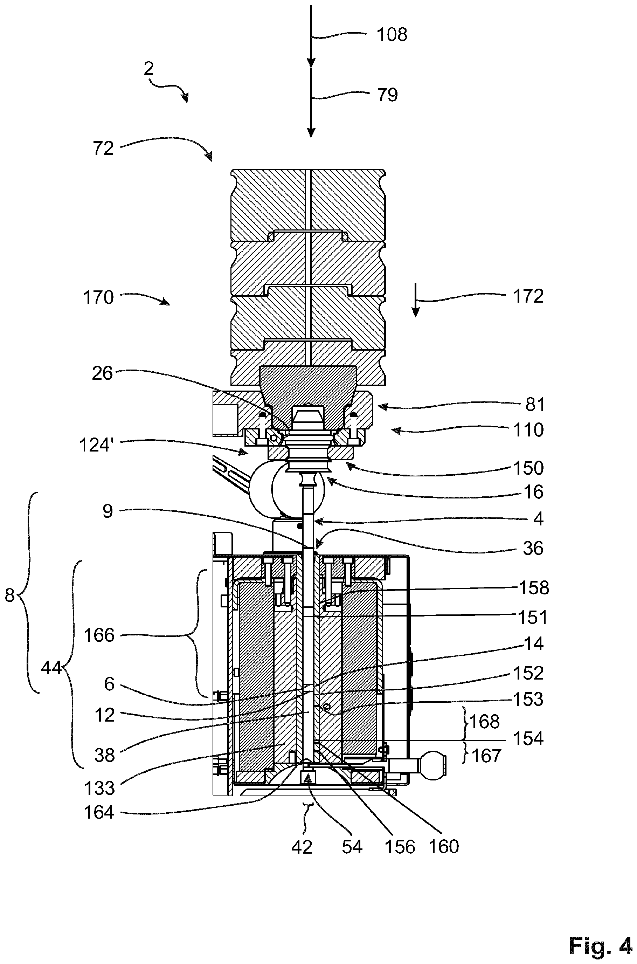

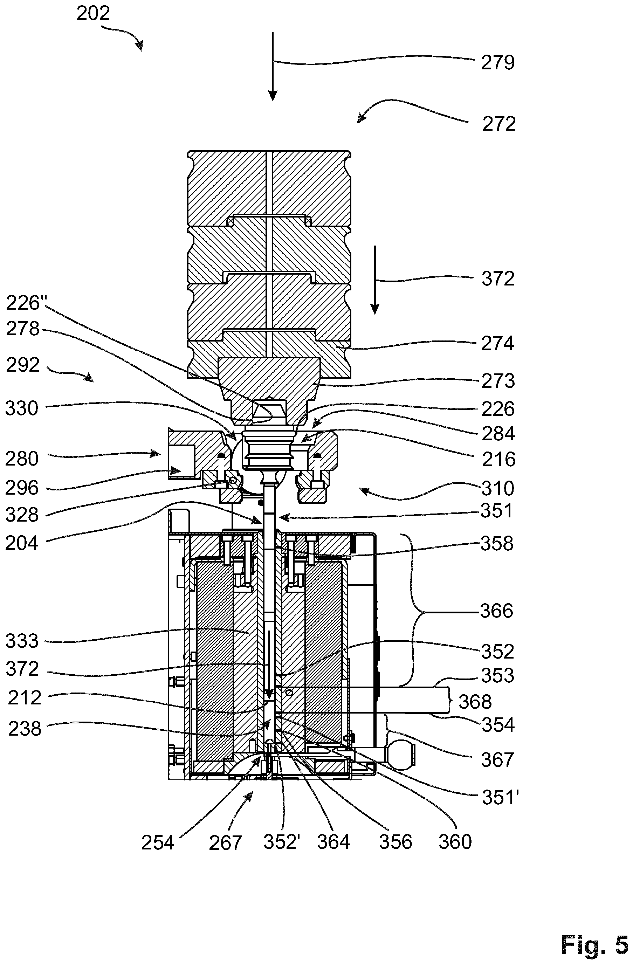

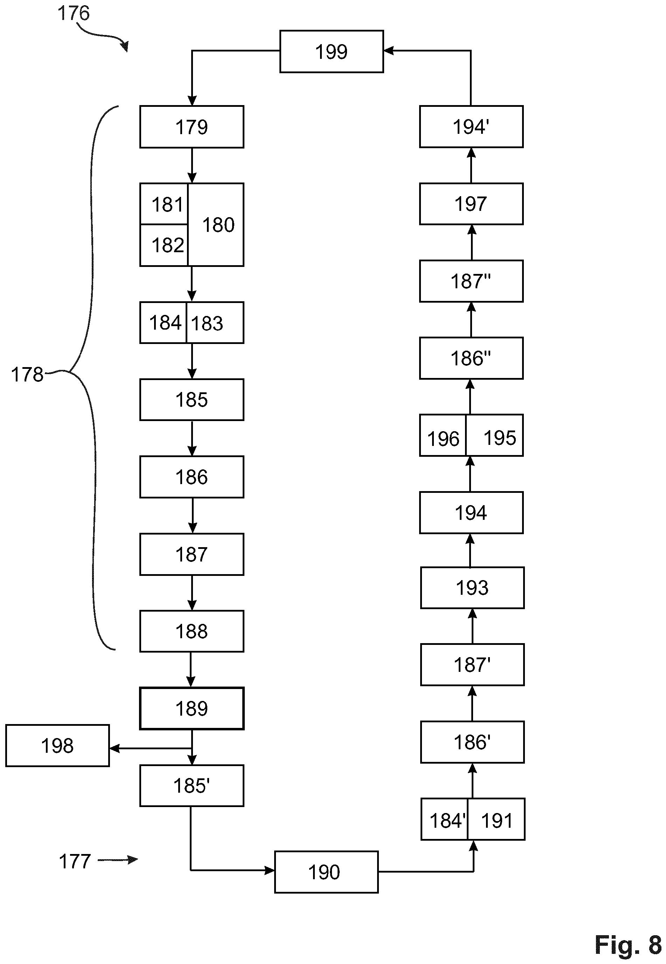

Figur 1 zeigt eine Seitenansicht einer Fließprüfmaschine 2 in schematischer Darstellung, bei der einzelne Komponenten in Querschnittsdarstellung eingezeichnet sind. Der Ausschnittsbereich A ist in Figur 2 und der Ausschnittsbereich B in Figur 3 vergrößert dargestellt. Weitere Ansichten der Fließprüfmaschine 2 sind in Figur 4, Figur 6 und Figur 7 gezeigt. Figur 5 betrifft unter anderem optionale Weiterbildungen an einer Fließprüfmaschine 202. Die Bezugszeichen von Bezugszeichen 2 bis Bezugszeichen 199 betreffen ein erstes Beispiel einer Fließprüfmaschine 2 sowie erfindungsgemäße Verfahren, die beispielhaft schematisch in Figur 8 gezeigt sind. Die weiteren Erläuterungen mit den Bezugszeichen größer als 199 lassen sich auch exemplarisch auf das erste Ausführungsbeispiel lesen bzw. übertragen, insbesondere durch Subtraktion von 200 bei den Bezugszeichen. Die Figuren 1 bis 8 werden im Folgenden daher gemeinsam diskutiert.

Die in Figur 1 gezeigte Fließprüfmaschine 2 weist einen herausnehmbaren Prüfkolben 4 mit einem Prüfkolbenkopf 16 auf, der sich entlang der Prüfkolbenachse 10 erstreckt. Der Prüfkolben 4 ist auf der Einfüllseite in die Einfüllöffnung 50 des Prüfkanals 38 eingesteckt, wobei der Prüfkanal 38 durch den Prüfkanalzylinder 40 angelegt bzw. seitlich begrenzt ist. Die Prüfkolbenachse 10 schließt an die in der Fließprüfmaschine 2 ortsfeste Zentralachse 46 an. Es kann auch gesagt werden, in der Arbeitsstellung des Prüfkolbens 4 liegen Prüfkolbenachse 10 und Zentralachse 46 übereinander. Der Prüfkolben 4 befindet sich in einer Anfahrstellung 151. In der ersten Schaltstellung 148 ist die Kupplungseinheit 110 geöffnet und bereit für die zentrierte Fixierung des Prüfkolbenkopfs 16. Die Kupplungseinheit 110 befindet sich an dem ersten Armende 81 des Arms 80. Die Halterung 112 ist an der Prüfkanalseite 85 des Arms 80 angeordnet. In der Freigabestellung 126 der Halterung 112 ist der Prüfkolbenkopf 16, insbesondere der Trägerbereich 24 und der erste Ankoppelbereich 20, in die Halterung 112 einführbar. Der Arm 80 ist ein Teil der Antriebseinheit 92. Der Arm 80 ist entlang der Prüfkolbenachse 10 auf den Prüfkolbenkopf 16 hin bewegbar, insbesondere bis der Trägerbereich 24 an die Prüfgewichtsaufnahme 73 anschließt. Die Prüfgewichtsaufnahme 73 liegt auf der Prüfgewichtsseite 86 des ersten Armendes 81. Die Prüfgewichtsaufnahme 73 ist durch eine Durchgangsöffnung 84 des ersten Armendes 81 zugänglich. Auf der Prüfgewichtsaufnahme 73 liegen weitere Prüfgewichtskörper, wie der Prüfgewichtskörper 74, auf. Die Prüfgewichtsaufnahme 73 und die ggf. weiteren Prüfgewichtskörper wie der Prüfgewichtskörper 74 bilden zusammen das (gesamte oder summarische) Prüfgewicht 72. An dem fixierbaren Drehgelenk 90 der Antriebseinheit 92 sitzt das zweite Armende 82, ausgestattet mit einem Drehbarkeitsbegrenzungsanschlag 88, der einen Anschlagssensor aufweist. An dem Anschlag 88 sind der Arm 80 und das aufliegende Prüfgewicht 72 entlang der Zentralachse 46 ausgerichtet.

Seitlich versetzt zur Einfüllöffnung 50 des Prüfkanals 38 ist die Wegmesseinrichtung 130 angeordnet (wie in Figur 1 zu sehen), die einen dem Arm 80 zugeordneten Messhebel 131 und einen dem Prüfkolben 4 zugeordneten Streckensensor 132 aufweist. Der Streckensensor 132 dient zur Messung von Teilstrecken der Bewegung des Prüfkolbens 4. Die Antriebseinheit 92 ist über die Steuereinheit 140 mit der Wegmesseinrichtung 130 elektronisch über Steuerleitungen (in der Figur 1 verdeckt) verbunden.

Die Antriebseinheit 92 umfasst einen ersten Aktuator 94 mit einem Prüfzylinder 100. In dem Prüfzylinder 100 sind eine erste Ansteuerseite 102 und eine zweite Ansteuerseite 104 mit einem Druckmittel, wie Druckluft, beaufschlagbar. Druckbeaufschlagung auf der ersten Ansteuerseite 102 bewirkt eine erste Kraftrichtung 174. Druckbeaufschlagung auf der zweiten Ansteuerseite 104 bewirkt eine zweite Kraftrichtung 175, die zur ersten Kraftrichtung 174 vektoriell entgegengerichtet ist. Die erste Kraftrichtung 174 und die zweite Kraftrichtung 175 stehen parallel zur Gewichtskraft 79 des Prüfgewichts 72. In der in Figur 1 gezeigten Anordnung des Prüfgewichts 72 liegt eine aktuatorische Zusatzkraft 108' aus der Antriebseinheit 92 vor, die betragsmäßig der Gewichtskraft 79 gleicht, und deren Richtung durch Druckbeaufschlagung der Antriebseinheit 92 auf der zweiten Ansteuerseite 104 der Gewichtskraft 79 entgegensteht. Aufgrund der Gleichheit der Gewichtskraft 79 und der Zusatzkraft 108' verharrt der Arm 80 bewegungslos. Mit Hilfe einer Umlenkvorrichtung 142, die ein Schaltwechselventil 144 in einem Schaltventilkasten 146 umfasst, ist es möglich, zwischen der Druckbeaufschlagung auf der ersten Ansteuerseite 102 und der Druckbeaufschlagung auf der zweiten Ansteuerseite 104 umzuschalten. Damit ist eine aktuatorische Zusatzkraft 108 auf den Prüfkolben 4 betragsmäßig kontrolliert zuführbar, die vektoriell die gleiche Richtung 174 wie die Gewichtskraft 79 hat. Durch die aktuatorische Zusatzkraft 108 wird die Gewichtskraft 79 betraglich additiv, also ohne Verluste durch abgewinkelte Kraftkomponenten, verstärkt. Eine Absenkung des Arms 80 zu dem Prüfkolbenkopf 16 kann allerdings auch unter Wirkung des Prüfgewichts 72 erfolgen, indem durch Verringerung eines Prüfmitteldrucks über die Druckkontrolleinheit 138 die aktuatorische Zusatzkraft 108' betragsmäßig vermindert wird. Mit der Geschwindigkeitssteuerung 98 lässt sich eine von der Antriebseinheit 92 auszuführende Hebung oder Senkung des Arms 80 in präzisen Zeitschritten kontrolliert über die Steuereinheit 140 ausführen. Ein Anschlag 89, 89' begrenzt die Bewegung der Antriebseinheit 92 entlang der Zentralachse 46 auf einem möglichen Fahrweg 106. Eine Zeitkontrolleinheit 141 in einer Basis 3 der Fließprüfmaschine 2 ist sowohl in Messvorbereitungsschritten als auch in Messschritten und Reinigungsschritten einsetzbar, z. B. um Bewegungen der Antriebseinheit 92 zeitlich zu koordinieren und in Verbindung mit der Steuereinheit 140 auszuführen. Die Steuereinheit 140 bildet die Basis 3 der Fließprüfmaschine 2. Die Basis 3 weist eine Mehrzahl von Stellfüßen, wie der Stellfuß 139, auf, mit denen eine lotrechte Ausrichtung der Zentralachse 46 des Prüfkanals 38 möglich ist. Der Prüfkanal 38 ist eingebettet in eine Heizeinrichtung 133. Die Heizeinrichtung 133 ist von einem thermischen Isolierkörper 134 ummantelt.

An der Abströmseite 48 des Prüfkanals 38 befindet sich die Düse 54, welche den Prüfkanal 38 mit der Auspressmaterialkammer 70 verbindet. Auf einer Ebene mit der Düse 54 befindet sich der Düsenschieber 66, mit dem die Düse 54 verschließbar ist. Der Düsenschieber 66 ist auch als Abschneider für Prüfmasse verwendbar. Die Auspressmaterialkammer mit Trenntür 70 ist, z. B. für Wägungen oder zur Entleerung, aus der Fließprüfmaschine 2 herausnehmbar. Sie schützt ausgepresste Prüfmasse vor Verunreinigungen, wie Wasserdampf, aus der Umgebung, die z. B. zu Fehlern bei Wägungen führen können. Zufälligem Hautkontakt mit oftmals heiß ausgepresster Prüfmasse wird vorgebeugt. Die Steuereinheit 140 arbeitet unter anderem als Temperaturmesseinheit und ist mit einem Temperaturfühler 136 und mit der Heizeinrichtung 133 verbunden. Der Temperaturfühler 136 befindet sich nahe bei dem Prüfkanalzylinder 40. Durch die Heizeinrichtung 133 kann die Steuereinheit 140 die Temperatur einer im Prüfkanal 38 enthaltenen Prüfmasse (nicht eingezeichnet) auf einen stabilen Wert einregeln. Der Prüfkanalzylinder 40 hat eine Wärmeleitfähigkeit von mindestens 15 Watt/(Meter*Kelvin). Die Wärme kann sich gleichmäßig über den Prüfkanalzylinder 40 verteilen. Aufgrund der Anordnung des Temperaturfühlers 136 separat zum Prüfkanalzylinder 40 kann der Prüfkanalzylinder 40, falls z. B. Verschleiß erkennbar sein sollte, schnell ersetzt werden.

Der Ausschnitt A, der aus Figur 1 entnommen, in Figur 2 übersichtlicher dargestellt, zeigt das auf dem Arm 80 (ausschnittsweise dargestellt) angeordnete Prüfgewicht 72 der Fließprüfmaschine 2. In dem in Querschnittsansicht dargestellten Bereich in Figur 2 ist die Kupplungseinheit 110 zu sehen, die von einem zweiten Aktuator 96 betätigbar ist. Der zweite Aktuator 96 kann ein pneumatisch zu betätigendes Schloss 120 schließen, festsetzen und öffnen. Dabei kann die Spanneinheit 116 an einen Kolbenkopf (nicht in Figur 2 eingezeichnet, siehe z. B. Figur 1) angreifen. Die Spanneinheit 116, die Teil der Halterung 112 ist, arbeitet zentrierend bezogen auf die Zentralachse 46 (vgl. Figur 1). Die Spanneinheit 116 ist - in Figur 2 - in einer Freigabestellung 126 gezeigt. Die Spanneinheit 116 umfasst die Formelemente 114, 114'. Jedes Formelement 114, 114' weist jeweils der Durchgangsöffnung 84 zugewandte Anlegeflächen, wie die Anlegeflächen 115, 115', auf. Die Anlegeflächen 115, 115' sind zum Formschluss an einen Ankoppelbereich eines Kolbenkopfs (siehe z. B. Ankoppelbereich 20 in Figur 1) ausgebildet. Der Zentrierbereich 78 kann den Trägerbereich eines Kolbenkopfs 16 (siehe z. B. Figur 1) aufnehmen. An einer der Durchgangsöffnung 84 zugewandten Seite des Arms 80 ist ein Näherungssensor 128 eingebaut. Wie sich in Zusammenschau mit Figur 1 ergibt, ist der Näherungssensor 128 über die Steuereinheit 140 mit dem ersten Aktuator 94 und mit dem zweiten Aktuator 96 (zu sehen in Figur 2) durch Steuerleitungen verbunden. Die Annäherung an einen Kolbenkopf 16 wird von dem Näherungssensor 128 induktiv erkannt. Eine Signalausgabe des Näherungssensors 128 dient zur Ansteuerung des zweiten Aktuators 96, wenn die Kupplung 110 in eine Schließstellung, wie die Schließstellung 124' in Figur 4, gebracht werden soll. Die Prüfgewichtsaufnahme 73 sitzt in der Durchgangsöffnung 84 und nimmt mit einem Halteelement 122 eine Schließstellung 124 bezüglich des Arms 80 ein. Auf die Prüfgewichtsaufnahme 73 sind weitere Prüfgewichtskörper, wie der Prüfgewichtskörper 74 und der Prüfgewichtskörper 75, aufstapelbar. Wird die Prüfgewichtsaufnahme 73 als erster Prüfgewichtskörper des Prüfgewichts 72 betrachtet, so liegt der zweite Prüfgewichtskörper 74 in dem Auflagebereich 77 zwischen Prüfgewichtsaufnahme 73 und Prüfgewichtskörper 74 auf. Der zweite Prüfgewichtskörper 74 ist in dem Zentrierbereich 78' bezüglich der Prüfgewichtsaufnahme 73 zentriert. Auf dem zweiten Prüfgewichtskörper 74 liegt in dessen Auflagebereich 77' der dritte Prüfgewichtskörper 75 auf. Der dritte Prüfgewichtskörper 75 ist in einem Zentrierbereich 78" bezüglich dem zweiten Prüfgewichtskörper 74 zentriert. Die Schwerpunkte (nicht eingezeichnet) aller Prüfgewichtskörper, wie der Prüfgewichtskörper 73, 74 und 75, liegen daher entlang einer geraden Linie, die an der Zentralachse 46, in Figur 1 eingetragen, ausgerichtet ist. Das Prüfgewicht 72 ist somit stabil auf den Prüfkolben 4, der in Figur 1 gezeigt ist, aufsetzbar.

In dem vergrößerten Ausschnittsbereich B, der in Figur 3 abgebildet ist, ist unter anderem die an der Abströmseite 48 des Prüfkanals 38 der Fließprüfmaschine 2 (vgl. Figur 1) angeordnete Düse 54 gezeigt. Die Düse 54 erstreckt sich über eine Düsenkanallänge 62 und weist an der Austrittsöffnung 52 des Prüfkanals 38 einen Düseneingangsdurchmesser 58 auf, der größer als der Düsenausgangsdurchmesser 60 ist. Der Düsenausgangsdurchmesser 60 befindet sich in einem Düsenhalter 64. Die Düse 54 umfasst also zwei Komponenten, einen Düseneinsatz 56 mit dem Düseneingangsdurchmesser 58 und den Düsenhalter 64. Der Düsenhalter 64 hält den Düseneinsatz 56, der den Düseneingangsdurchmesser 58 aufweist, in dem Prüfkanal 38. Der Düsenhalter 56 ist mit dem Düsenschieber 66 betätigbar und kann zur Seite geschwenkt werden. Der Düseneinsatz 56 sitzt herausnehmbar in dem Prüfkanalzylinder 40. Die Heizeinrichtung 133 erstreckt sich am Prüfkanalzylinder 40 bis in den Bereich der Düse 54, so dass auch die Düsenausgangsöffnung 60 auf einer für eine Fließprüfung erforderliche Temperatur gehalten wird.

Der Bereich der Fließprüfmaschine 2, der in Figur 4 gezeigt ist, stellt einen ersten Betriebszustand 170 dar. Die aktuatorische Zusatzkraft 108 wirkt über das erste Armende 81 gleichgerichtet zur Gewichtskraft 79 des Prüfgewichts 72. Der Prüfkolben 4 folgt einer Bewegungsrichtung 172 in dem Prüfkanal 38, die gleichgerichtet zu der Zusatzkraft 108 ist. Das Prüfgewicht 72 liegt auf dem ersten Armende 81 auf. Die Kupplungseinheit 110 befindet sich unter dem Prüfgewicht 72 in einer Schließstellung 124'. Es liegt eine zweite Schaltstellung 150 der Kupplungseinheit 110 vor, in der der Prüfkolbenkopf 16 an dem ersten Armende 81 fest sitzt. Eine Position des Prüfkolbenkopfs 16 ist lateral, anders gesagt, in einer rechtwinklig zur Gewichtskraft 79 ausgerichteten Hilfsebene, durch den Zentrierbereich 26, angeordnet in der Kupplungseinheit 110, begrenzt. Der Zentrierbereich 26 verbessert eine Reproduzierbarkeit einer Zuordnung bzw. Ausrichtung des Prüfkolbens 4 zu dem Prüfkanal 38. Die Schließstellung 124' ist präzise einnehmbar. Der Prüfkolben 4 durchfährt mittels Antriebseinheit 92, dargestellt in Figur 1, den Prüfraum 36, der in Figur 4 zu sehen ist. Die Prüfkolbenlänge 8 ist der Abstand zwischen Prüfkolbenkopf 16 und einer Pressfläche 12. Der Prüfkolben 4 weist Längenmarkierungen, wie die Längenmarkierung 9, auf, die z. B. als Kalibrierhilfe für eine Wegmessung verwendbar sind. Die Pressfläche 12 wirkt auf Prüfmaterial (nicht dargestellt), das in dem Prüfkanal 38 vorliegt. Der Prüfkanal 38 weist eine Kanallänge 44 auf. Der Kanaldurchmesser 42 ist größer als der Kolbendurchmesser 6 und insbesondere größer als die Auspressdüse 54. Der Kanaldurchmesser 42 hat einen Wert zwischen 8 mm und 10 mm. An die Pressfläche 12, die den Kolbendurchmesser 6 abdeckt, schließt sich seitlich ein Dichtbereich 14 längs dem Prüfkolben 4 an, um Prüfmaterial (nicht gezeigt) mit Druck zur Düse 54 hin verschieben zu können. In der gezeigten Bewegung 172 wird der Prüfkolben 4 aktuatorisch mittels Antriebseinheit 92 (vgl. Figur 1) von einer Anfahrstellung 151 auf eine Endfahrstellung 152 hin bewegt. Beim Anschmelzen hat sich das Prüfmaterial von der Versuchsbeginnstellung 158 bis zur Anfahrstellung 151 gesetzt. Auf dem ersten Auspressweg 166 wird Prüfmaterial in Richtung der Düse 54 verschoben und ein Teil des Prüfmaterials wird durch die Düse 54 hindurchgedrückt. Bei der Endfahrstellung 152, die auch als zweite Stellung des Prüfkolbens 4 bezeichnet werden kann, erfolgt eine Verringerung der aktuatorischen Zusatzkraft 108. Bei Erreichen einer dritten Stellung, der Prüfstartstellung 153, wird die Bewegung 172 des Prüfkolbens 4 ausschließlich durch das Prüfgewicht 72 verursacht. Vor dem Erreichen der Prüfstartstellung 153 kann die Kupplungseinheit 110 geöffnet werden und durch ein Absenken des ersten Armendes 81 das Prüfgewicht 72 freigegeben werden, so dass die Gewichtskraft 79 auf dem Prüfkolben 4 als einzige Antriebskraft der Bewegung 172 vorliegt. Zwischen der Prüfstartstellung 153 und einer Prüfendstellung 154, also einer vierten Stellung des Prüfkolbens 4, liegt die Versuchsstrecke 168 in dem Prüfkanal 38. Auf der Versuchsstrecke 168 erfolgt ein Auspressen der Prüfmasse insbesondere mittels Gewichtskraft 79. Das Durchfahren der Versuchsstrecke 168 mit dem Prüfkolben 4 wird von einer Zeitmessung durch die Zeitkontrolleinheit 141 (s. Figur 1) begleitet. Zurück zu Figur 4, auf die Prüfendstellung 154 folgt längs des Prüfkanals 38 die Versuchsendstellung 160, in der die Heizeinrichtung 133 ausgeschaltet wird. Zwischen der Prüfendstellung 154 und der Versuchsbereichsendstellung 164 ist auch die aus Normen bekannte Prüfbereichsendstellung 156 verortet. Zwischen der Prüfendstellung 154 und der Versuchsbereichsendstellung 164 liegt eine zweite Teilstrecke als Auspressweg 167 in dem Prüfkanal 38. Auf dem Auspressweg 166, 167 wird Prüfmaterial, das nicht für eine Messung der Fließzeit benötigt wird, mit Hilfe der aktuatorischen Zusatzkraft 108 schnell aus dem Prüfkanal 38 hinausgeschoben.

Figur 5 zeigt die Fließprüfmaschine 202 mit dem Prüfkolben 204, dessen Pressfläche 212 sich in dem Prüfkanal 238 auf der Versuchsstrecke 368 zwischen der Prüfstartstellung 353 und der Prüfendstellung 354 befindet. Die Bewegung 372, gezeigt als Bewegungsrichtungspfeil 372, durch den Prüfkanal 238 erfolgt nur unter Wirkung der Gewichtskraft 279 des Prüfgewichts 272. Der Zentrierbereich 226" des Prüfkolbens 204 ist verbunden mit dem Zentrierbereich 278 der Prüfgewichtsaufnahme. An dem Prüfkolbenkopf 216 liegt also ein erster Zentrierbereich 226 und ein zweiter Zentrierbereich 226" vor. Ein Schwerpunkt des Prüfgewichts 272 ist zentralaxial auf dem Prüfkolben 204 angeordnet. Auf der Auspressstrecke 366 ist die Bewegung 372 zwischen der Anfahrstellung 351 und der Endfahrstellung 352 verstärkt durch eine Antriebseinheit 292, vermittelt über den Arm 280 auf dem Prüfkolbenkopf 216, sodass die Bewegung 372 mit größerer Schnelligkeit erfolgt. Die Versuchsbeginnstellung 358 zeigt die ursprünglich vorliegende Füllstellung des Prüfkanals 238 mit Prüfmaterial (nicht dargestellt). Auf die Prüfendstellung 354 folgt im Prüfkanal 238 eine zweite Teilstrecke als Auspressweg 367. Die zweite Teilstrecke erstreckt sich von der Anfahrstellung 351', die einer Versuchsendstellung 360 zugeordnet ist, bis zu einer Endfahrstellung 352', welche der Versuchsbereichsendstellung 364 zugeordnet ist. Die Prüfbereichsendstellung 356 markiert eine nach (in einigen Ländern) geltenden Normen einzuhaltende Beabstandung der Versuchsstrecke 368 von der Düse 254. Die Düse 254 mündet in eine Spritzgussöffnung 267 einer Pressform, die eine optionale Zusatzausstattung der Fließprüfmaschine 202 darstellt. Damit werden Untersuchungen zum Verpressen von Prüfmasse in eine vorgegebene Form ermöglicht. Während der gesamten Bewegung 372 des Prüfkolbens 204 wird mittels der Heizeinrichtung 333 eine konstante Temperatur im Prüfkanal 238 gehalten. Die durch den zweiten Aktuator 296 offen gehaltene Kupplungseinheit 310 weist den Näherungssensor 328 auf. Die Kupplungseinheit 310 wird bei der Bewegung 372 des Prüfkolbenkopf 216 entlang der Versuchsstrecke 368 von der Antriebseinheit 292 dem Prüfkolbenkopf 216 in Bewegungsrichtung 279 voraus positioniert. Der Prüfkolben 204 wird in der Bewegung 372 durch die Wegmesseinrichtung 330 überwacht. Der Näherungssensor 328 ermöglicht nach der Prüfendstellung 354 der Antriebseinheit 292, den Prüfkolbenkopf 216 anzufahren und an den Prüfkolbenkopf 216 anzukoppeln. Die Annäherung erleichtert der Zentrierbereich 226 an dem Prüfkolbenkopf 216, der durch die Durchgangsöffnung 284 hindurchführbar ist. Die Prüfgewichtsaufnahme 273 und die Prüfgewichtskörper, wie der Prüfgewichtskörper 274, haben einen größeren Durchmesser als die Durchgangsöffnung 284. Die Prüfgewichtsaufnahme 273 ist auf dem Arm 280 von dem Prüfkolben 204 abhebbar.

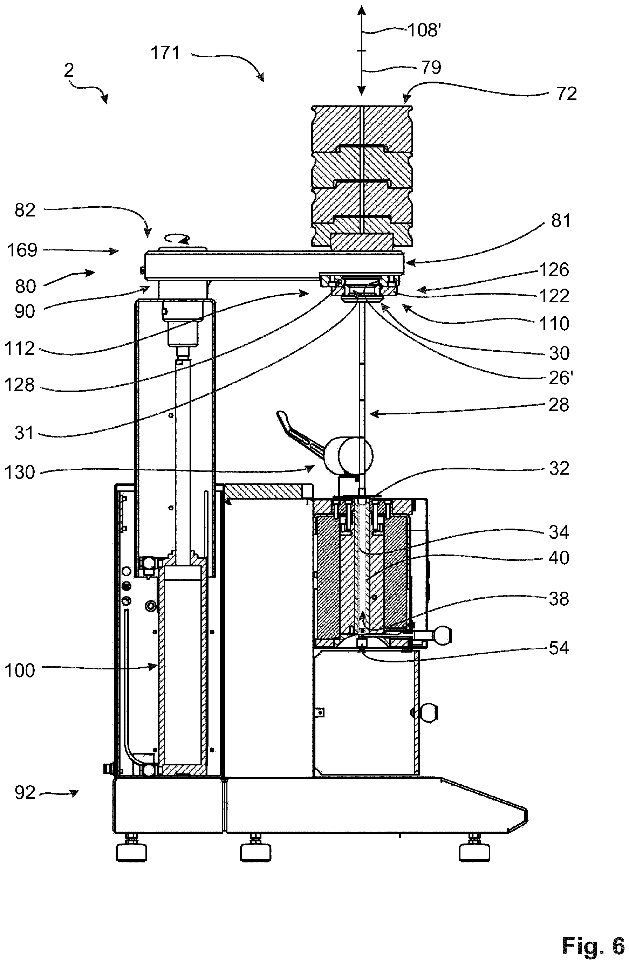

In Figur 6 befindet sich die Fließprüfmaschine 2 in einem zweiten Betriebszustand 171. Durch eine der Gewichtskraft 79 des Prüfgewichts 72 entgegengerichtet aktuatorische Zusatzkraft 108' ist das Prüfgewicht 72 durch die Antriebseinheit 92 mittels Prüfzylinder 100 bis auf Anschlag angehoben. Das Prüfgewicht 72 liegt auf dem Arm 80 am ersten Armende 81. In der Endstellung 169 des Arms 80 ist der Arm 80 am zweiten Armende 82 drehbar bezüglich dem Prüfzylinder 100. Das Drehgelenk 90, durch das der Arm 80 drehbar ist, ist in der Endstellung 169 festgesetzt, anders gesagt, rastiert. Die Kupplungseinheit 110 befindet sich in der Freigabestellung 126.

Wie in Figur 6 zu sehen ist, ist - insbesondere zu Beginn des Vorgangs des Reinigens - der Kopfbereich 30 des Reinigungskolbens 28 in der Halterung 112 an dem Zentrierbereich 26' des Reinigungskolbenkopfs 30 eingesetzt. Somit signalisiert der Näherungssensor 128, dass die Halteelemente, wie das Halteelement 122, in den zweiten Ankoppelbereich 31 einfahren können. Zwischen dem Reinigungskolben 28 und dem Prüfkanal 38 ist ein Reinigungstuch 32 eingelegt. Das Reinigungstuch 32 ist von dem Reinigungskolben 28 mittels Antriebseinheit 92 durch den Prüfkanal 38 hindurchdrückbar. Prüfmaterial 34 und Verunreinigungen werden damit von der Innenwand des Prüfkanalzylinders 40 aufgenommen und in Richtung auf die Düse 54 hin verschoben. Mit der Wegmesseinrichtung 130 lässt sich die Eindringtiefe des Reinigungskolbens 28 in dem Prüfkanal 38 überwachen.

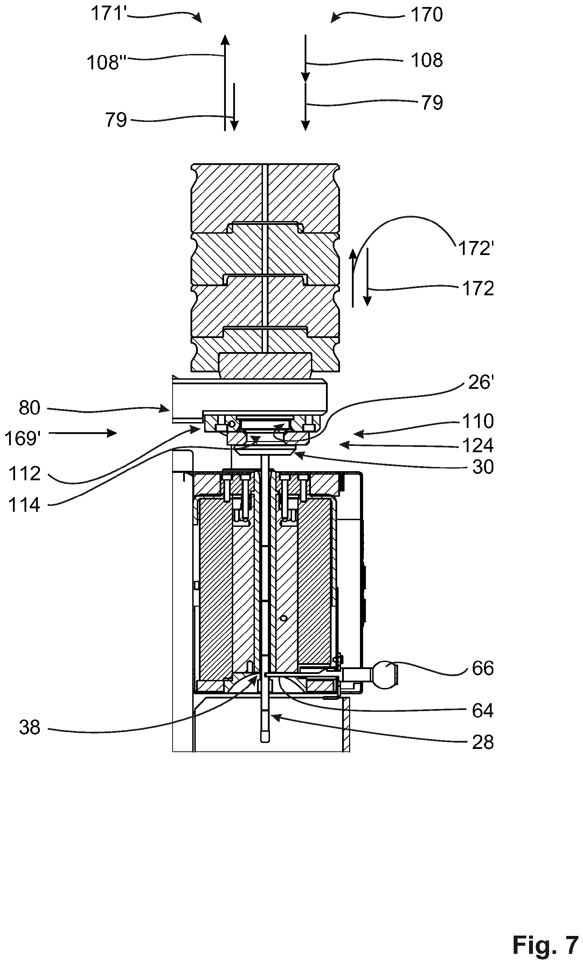

In Figur 7 ist ein durchschuboffener Prüfkanal 38 gezeigt, in dem sich der Reinigungskolben 28 befindet. Der Düseneinsatz 56, z. B. zu sehen in Figur 3, ist nach Lösen des Düsenhalters 64 aus dem Prüfkanal 38 mit Hilfe des Reinigungskolbens 28, vergleichbar mit dem in Figur 6 gezeigten Ablauf, herausstoßbar. Der Reinigungskolben 28 kann den gesamten Prüfkanal 38 durchdringen, wie Figur 7 zeigt, und dabei ein Reinigungsmittel, wie das Reinigungstuch 32 (siehe Figur 6) mitführen. In Figur 7 ist der Düsenhalter 64 mit dem Düsenschieber 66 unter dem Prüfkanal 38 seitlich weggeschwenkt, weshalb die Schnittzeichnung in Figur 7 ein Vortreten der Düsenhalterung 64 vor den Reinigungskolben 28 zeigt.

In Figur 7 befindet sich die Kupplungseinheit 110 in einer Schließstellung 124, wobei der Kopfbereich 30 des Reinigungskolbens 28 durch die Halterung 112 mit dem Formelement 114 gegenüber dem Zentrierbereich 26' fixiert ist. Der (ausschnittsweise dargestellte) Arm 80 befindet sich dabei in der Nähe einer Endstellung 169', während der Kolben 28 eine Bewegung 172 durchführt, die den Reinigungskolben 28 in den Prüfkanal 38 hinein und streckenweise hindurch führt. Die Bewegung 172 wird durch die Gewichtskraft 79 und die gleichgerichtete aktuatorische Zusatzkraft 108 angetrieben, wobei ein erster Betriebszustand 170 vorliegt. Beim Erreichen der Endstellung 169' wird ein zweiter Betriebszustand 171' eingenommen, indem die aktuatorische Zusatzkraft 108" in der Richtung umgekehrt und vom Betrag die Gewichtskraft 79 übersteigend angewendet wird, sodass eine Bewegung 172' erfolgt. Die Kraftvektoren 79, 108, 108" sind nur zur Vereinfachung der Darstellung nebeneinander und beabstandet von der Zentralachse 46, deren Lage in Figur 1 eingezeichnet ist, dargestellt worden. Es liegt kein Drehmoment vor, das den Reinigungskolben 28 von der Zentralachse 46 kippen könnte. Die Bewegung 172' führt den Arm 80 aus der Endstellung 169' zurück. Anschließend kann der Reinigungskolben 28 wieder durch einen Prüfkolben (wie Prüfkoben 4 in Figur 1) ersetzt werden.

In einer weiteren Ausführungsform einer Fließprüfmaschine ähnlich zu den Fließprüfmaschinen 2 und 202 kann der Arm 80 oder die Kupplungseinheit 110 ein, vorzugsweise motorisch oder aktuatorisch, antreibbares Drehelement, insbesondere in Verbindung mit einem Drehgelenk, aufweisen. Mit einem Drehelement kann z. B. mittels Antriebseinheit 92 der Reinigungskolben 28 drehbar, wie ein Bohrer, sein.

Schritte eines Fließprüfverfahrens 176 in schematischer Aufzählung, die z. B. in einer Schmelzviskositätsprüfung ausgeführt werden, sind in Figur 8 dargestellt. Das Fließprüfverfahren 176 ist in Kombination mit einem Reinigungsverfahren 177 als Prüfzyklus 199 mehrmals nacheinander ausführbar. Das Fließprüfverfahren 176 beginnt mit einem Messvorbereitungsschritt 178, dem mehrere Teilschritte zugeordnet werden können. In einem Befüllungsschritt 179 wird Prüfmasse für eine Fließprüfung portioniert und in eine Fließprüfmaschine 2 (z. B. nach Figur 1) eingefüllt. Es folgt ein Heizschritt 180 bis auf eine Solltemperatur mit einer Aufheizzeit 181 und einer Anschmelzzeit 182. In der Anschmelzzeit 182 erlangt die Prüfmasse einen auf Fließfähigkeit prüfbaren Zustand. Der Heizschritt 180 wird fortgesetzt, bis zu dem Zeitpunkt, zu dem zumindest ein Messschritt 189 erledigt ist, sodass bei dem Fließprüfverfahren 176 möglichst konstante Temperaturbedingungen herrschen. Auf die Anschmelzzeit 182 folgt ein erster Auspressschritt 183, wobei die Auspresszeit 184 durch Anwendung einer aktuatorischen Zusatzkraft aus einer Antriebseinheit, wie der Antriebseinheit 92 (s. Figur 1), gegenüber einem Auspressen durch Gewichtskraft verkürzt ist. Nach dem ersten Auspressschritt 183 erfolgt vorzugsweise eine Positionsbestimmung 185 des Prüfkolbens, wie dem Prüfkolben 4 (s. Figur 1), und ein Abbremsschritt 186, in dem zumindest die aktuatorische Zusatzkraft zurückgenommen wird. Es kann auch gesagt werden, dass in dem Abbremsschritt 186 ein Aktuator einer Antriebseinheit auf einen passiven, insbesondere druckdifferenzfreien Betrieb umgeschaltet wird. An den Abbremsschritt 186 schließt sich der Abkoppelschritt 187 an, in dem eine, insbesondere kraftschlüssige und formschlüssige, Verbindung zwischen einer Antriebseinheit, wie der in Figur 1 gezeigten Antriebseinheit 92, und dem Prüfkolben, wie der in Figur 1 gezeigte Prüfkolben 4, getrennt wird. Der Abkoppelschritt 187 umfasst vorzugsweise, dass die Antriebseinheit 92, die das Prüfgewicht auf den Prüfkolben 4 abgeladen hat, ohne das Prüfgewicht in einer zweiten Endstellung positioniert wird oder zumindest in einem vorgegebenen Abstand zum Prüfkolbenkopf gefahren wird. Der Abstand kann z. B. über einen Näherungssensor an einem Arm überprüft werden. Es folgt ein Relaxationsschritt 188, in dem sich Fließbedingungen in der noch im Fließprüfverfahren befindlichen Prüfmasse, also insbesondere das Prüfmassevolumen, unter Wirkung eines Prüfgewichts auf die Prüfmasse, vergleichmäßigen, wie z. B. Temperatur und Dichte der Prüfmasse. Nach dem Relaxationsschritt 188 wird der Messschritt 189 ausgeführt, der eine Versuchsstrecke umfasst. Auf der Versuchsstrecke erfolgt eine Weg-Zeit-Messung. Nach Abschluss des Messschritts 189 können unmittelbar die aufgenommenen Daten in einem Auswerteschritt 198 analysiert werden. Es können in dem Auswerteschritt 198 aber auch weitere Messungen erfolgen, wie z. B. eine Stranglängenmessung, eine Wägung, spektrometrische Untersuchungen oder chemische Analysen von der im Messschritt 189 ausgepressten Prüfmasse. Derartige Erhebungs- bzw. Messschritte, die sich an den Messschritt 189 anschließen, lassen sich summarisch in einem Auswerteschritt 198 zusammenfassen. Das Ende des Messschritts 189 geht einher mit einer Positionsbestimmung 185', die auch im Zuge einer Wegstreckenmessung erfolgen kann.

Weitere Schritte, die auf die Positionsbestimmung 185' folgen, lassen sich auch einem Reinigungsverfahren 177 zuordnen. Mit Hilfe der Positionsbestimmung 185' wird ein Näherungs- und Ankoppelschritt 190 ausgeführt. Die Antriebseinheit, wie die Antriebseinheit 92, gezeigt in Figur 1, wird in eine kraft- und formschlüssige Verbindung mit dem Prüfkolben, insbesondere mit dem Prüfkolbenkopf, verbracht. Es folgt ein zweiter Auspressschritt 191, wobei die Antriebseinheit eine aktuatorische Zusatzkraft auf den Prüfkolben aufbringt. Hierdurch wird der Prüfkolben mit einer im Vergleich zur Wirkung der Gewichtskraft erhöhten Geschwindigkeit bewegt. Die Auspresszeit 184' ist damit verkürzt. Der zweite Auspressschritt 191 kommt mit einem Abbremsschritt 186' zum Ende, in dem die aktuatorische Zusatzkraft zurückgenommen wird. Es ist auch möglich, die aktuatorische Zusatzkraft nach einer betragsmäßigen Rücknahme in der Richtung umzuschalten, um die Gewichtskraft des Prüfgewichts zu kompensieren. Damit lässt sich eine Belastung im Bereich der Düse, wie der Düse 54 (zu sehen in Figur 3), vermindern. In dem Fließprüfverfahren 176 erfolgt nun ein Abkoppelschritt 187', bei dem die kraft- und formschlüssige Verbindung zwischen Antriebseinheit und Prüfkolben geöffnet wird und die Antriebseinheit zusammen mit dem Prüfgewicht in einer ersten Endstellung positioniert wird. Es ist auch möglich, in einem alternativen Verfahren den Prüfkolben durch die Antriebseinheit aus dem Prüfkanal herausziehen zu lassen. Zur Fortsetzung des Reinigungsverfahrens 177 wird nun der Prüfkolben entnommen oder in einem Prüfkolbenhalter abgesetzt und ein Reinigungskolben eingesetzt, gem. eines Schrittes, markiert durch 193. Anders gesagt, wird der Reinigungskolben, insbesondere der Reinigungskolbenkopf, mit der einzigen Antriebseinheit, die auch im Fließprüfverfahren 176 arbeitet, verbunden. Der Reinigungskolben sitzt somit vor der Einfüllöffnung des Prüfkanals. Es folgt die Düsenentnahme 194, wobei ein Weg durch den Prüfkanal freigegeben wird. In dem Reinigungsschritt 195, der auch als zweiter Reinigungsschritt bezeichnet werden kann, wird der Reinigungskolben in einer gegenüber der Wirkung der Gewichtskraft verkürzten Reinigungszeit 196 mit Hilfe der aktuatorischen Zusatzkraft durch den Prüfkanal gestoßen. Es ist möglich, so wie in Figur 6 dargestellt, ein auf die Einfüllöffnung vor dem Reinigungskolben 28 aufgelegtes Tuch 32 durch den Prüfkanal 38 mitzuführen. Damit lassen sich auch festsitzende Schmutzpartikel oder Schmutzspuren aus dem Prüfkanal 38 beseitigen. Nachdem der Reinigungskolben 28 durch den Prüfkanal 38 hindurchgefahren ist, erfolgt ein Abbremsschritt 186", wobei durch Umschaltung der aktuatorischen Zusatzkraft und deren betragsmäßige Angleichung zur Gewichtskraft des Prüfgewichts der Reinigungskolben 28 zum Stillstand gebracht wird. Anschließend ist es möglich einen Abkoppelschritt 187" vorzunehmen, und die form- und kraftschlüssige Verbindung zwischen Reinigungskolben und Antriebseinheit zu trennen. In einer Alternative zum Reinigungsverfahren 177 ist es auch möglich, die Abkopplung des Reinigungskolbens 28 erst nach der Rückführung der Antriebseinheit in die erste Endstellung vorzunehmen. In dem Reinigungsverfahren 177 folgt auf den Abkoppelschritt 187" die Rückführung der Antriebseinheit und die Entnahme des Reinigungskolbens 28, insbesondere aus dem Prüfkanal 38, sowie ein Einsetzen eines Prüfkolbens (siehe Schritt 197). Mit dem Einsetzen der Düse gem. Schritt 194' ist das Verfahren zur Fortsetzung in einem Prüfzyklus 199 bereit.

Durch das oben beschriebene Verfahren und mithilfe der oben beschriebenen Fließprüfmaschinen 2 lassen sich insbesondere Schmelzviskositätsprüfungen mit großem Zeitgewinn und hoher Präzision ausführen. Es ist möglich, Alternativen von Verfahrensabläufen zu bilden, bei denen einzelne oder mehrere Verfahrensschritte oder Teilschritte ausgelassen, ergänzt oder vertauscht werden.

Die in den einzelnen Figuren gezeigten Ausgestaltungsmöglichkeiten lassen sich auch untereinander in beliebiger Form verbinden. Beispielhaft sei angeführt, dass eine Fließprüfmaschine mehrere Prüfkolben 4 haben kann, die Heizeinrichtung 133, 333 kürzer oder länger in Bezug zu dem Prüfkanal 38, 238 sein kann und dass die Düse 54, 254 auch mehr als nur zwei Durchmesser entlang ihrer Öffnung aufweisen kann.

### Bezugszeichenliste

- 2,202: Fließprüfmaschine
- 3: Basis
- 4, 204: Prüfkolben
- 6: Kolbendurchmesser
- 8: Kolbenlänge, insbesondere Prüfkolbenlänge
- 9: Längenmarkierung
- 10: Prüfkolbenachse
- 12,212: Pressfläche
- 14: Dichtbereich
- 16,216: Prüfkolbenkopf
- 20: erster Ankoppelbereich
- 24: Trägerbereich
- 26, 26', 226, 226": Zentrierbereich, insbesondere eines Kolbenkopfs
- 28: Servicekolben, insbesondere Reinigungskolben
- 30: Kopfbereich, insbesondere Reinigungskolbenkopf
- 31: zweiter Ankoppelbereich
- 32: Reinigungsmittel, insbesondere Tuch
- 34: Prüfmaterial, insbesondere Reste
- 36: Prüfraum
- 38, 238: Prüfkanal
- 40: Prüfkanalzylinder
- 42: Kanaldurchmesser
- 44: Kanallänge
- 46: Zentralachse
- 48: Abströmseite
- 50: Einfüllseite, insbesondere Einfüllöffnung, wie Einfüllkonus
- 52: Austrittsöffnung, insbesondere Endbereich des Prüfkanals
- 54, 254: Düse, insbesondere Auspressdüse
- 56: Düseneinsatz
- 58: Düseneingangsdurchmesser
- 60: Düsenausgangsdurchmesser
- 62: Düsenkanallänge
- 64: Düsenhalter
- 66: Düsenschieber
- 267: Spritzgussöffnung, insbesondere Pressform
- 70: \Auspressmaterialkammer, insbesondere Trenntür
- 72, 272: Prüfgewicht
- 73, 273: Prüfgewichtsaufnahme, insbesondere erster Prüfgewichtskörper
- 74, 274: Prüfgewichtskörper, insbesondere zweiter
- 75: Prüfgewichtskörper, insbesondere dritter
- 77, 77': Auflagebereich
- 78, 78', 78", 278: Zentrierbereich, insbesondere des Prüfgewichts
- 79, 279: Gewichtskraft des Prüfgewichts, insbesondere Gewichtskraftrichtung
- 80, 280: Arm, insbesondere verfahrbarer Arm
- 81: erstes Armende
- 82: zweites Armende
- 84, 284: Durchgangsöffnung
- 85: Prüfkanalseite des Arms, insbesondere des Armendes
- 86: Prüfgewichtsseite des Arms, insbesondere des Armendes
- 88: Anschlag, insbesondere Drehbarkeitsbegrenzungsanschlag
- 89, 89': Anschlag, insbesondere Endstellungsanschlag
- 90: Drehgelenk, insbesondere rastierbares Gelenk
- 92, 292: Antriebseinheit
- 94: erster Aktuator
- 96, 296: zweiter Aktuator
- 98: Geschwindigkeitssteuerung
- 100: Prüfzylinder
- 102: erste Ansteuerseite
- 104: zweite Ansteuerseite
- 106: Fahrweg
- 108, 108', 108": aktuatorische Zusatzkraft
- 110, 310: Kupplungseinheit
- 112: Halterung
- 114, 114': Formelement
- 115, 115': Anlegefläche
- 116: Spanneinheit
- 120: Schloss, insbesondere elektromagnetisches oder pneumatisches Schloss, vorzugsweise hydraulisches Schloss
- 122: Halteelement
- 124, 124': Schließstellung
- 126: Freigabestellung
- 128,328: Näherungssensor
- 130, 330: Wegmesseinrichtung, insbesondere Kolbenstellungsmesseinrichtung
- 131: Messhebel
- 132: Streckensensor
- 133, 333: Heizeinrichtung
- 134: thermischer Isolierkörper
- 136: Temperaturmesser, insbesondere Fühler
- 138: Druckkontrolleinheit, insbesondere Druckregeleinheit
- 139: Stellfuß
- 140: Steuereinheit, insbesondere Steuerungselektronik
- 141: Zeitkontrolleinheit
- 142: Umlenkvorrichtung, insbesondere Umschalter eines Bewegungselements
- 144: Schaltventil, insbesondere Schaltwechselventil
- 146: Schaltventilkasten
- 148: erste Schaltstellung, insbesondere einer Kupplungseinheit
- 150: zweite Schaltstellung, insbesondere einer Kupplungseinheit
- 151, 351, 351': erste Stellung des Prüfkolbens, insbesondere Anfahrstellung
- 152, 352, 352': zweiten Stellung des Prüfkolbens, insbesondere Endfahrstellung
- 153, 353: dritte Stellung des Prüfkolbens, insbesondere Prüfstartstellung
- 154, 354: vierte Stellung des Prüfkolbens, insbesondere Prüfendstellung
- 156, 356: Prüfbereichsendstellung
- 158, 358: Versuchsbeginnstellung
- 160, 360: Versuchsendstellung
- 164, 364: Versuchsbereichsendstellung
- 166, 366: Strecke, insbesondere erste Teilstrecke, vorzugsweise Auspressweg
- 167, 367: Strecke, insbesondere zweite Teilstrecke, vorzugsweise Auspressweg
- 168, 368: Versuchsstrecke
- 169, 169': Endstellung, insbesondere Anschlagsstellung
- 170: erster Betriebszustand
- 171, 171': zweiter Betriebszustand
- 172, 172', 372: Bewegung, insbesondere Bewegungsrichtung eines Kolbens
- 174: erste Richtung, insbesondere einer Kraft
- 175: zweite Richtung, insbesondere einer Kraft
- 176: Fließprüfverfahren, insbesondere Schmelzviskositätsprüfung
- 177: Reinigungsverfahren, vorzugsweise für eine Fließprüfmaschine
- 178: Messvorbereitungsschritt
- 179: Befüllungsschritt
- 180: Heizschritt
- 181: Aufheizzeit
- 182: Anschmelzzeit
- 183: erster Auspressschritt
- 184, 184': Auspresszeit
- 185, 185': Positionsbestimmung, insbesondere Bestimmung eines zurückgelegten Wegs
- 186, 186', 186": Abbremsschritt, insbesondere Aktuator Umschaltung
- 187: Abkoppelschritt, insbesondere Positionierung der Antriebseinheit, insbesondere ohne Prüfgewicht, in einer zweiten Endstellung
- 187', 187": Abkoppelschritt, insbesondere Positionieren der Antriebseinheit, insbesondere mit Prüfgewicht, in einer ersten Endstellung
- 188: Relaxationsschritt
- 189: Messschritt, insbesondere Weg-Zeit-Messung
- 190: Näherungs- und Ankoppelschritt
- 191: zweiter Auspressschritt, insbesondere erster Reinigungsschritt
- 193: Entnahme des Prüfkolbens und Einsetzen eines Reinigungskolben
- 194: Düsenentnahme
- 194': Düse einsetzen
- 195: Reinigungsschritt, insbesondere zweiter Reinigungsschritt
- 196: Reinigungszeit
- 197: Entnahme des Reinigungskolbens und Einsetzen eines Prüfkolben
- 198: Auswerteschritt, insbesondere Rechenschritt
- 199: Prüfzyklus
- A: Ausschnitt, umfassend erstes Armende und Prüfgewicht
- B: Ausschnitt, umfassend Düse an der Abströmseite des Prüfkanals

## Patentansprüche

1. Fließprüfmaschine (2, 202),
umfassend einen Prüfkolben (4, 204), einen Prüfkanal (38, 238), mindestens ein Prüfgewicht (72, 272) und eine Antriebseinheit (92, 292), wie ein hydraulischer oder ein pneumatischer Aktuator (94, 96, 296),
**dadurch gekennzeichnet, dass**
das Prüfgewicht (72, 272) und die Antriebseinheit (92, 292) in einem Antriebszustand in einer Kraftkopplungsverbindung stehen,
wobei das Prüfgewicht (72, 272) von der Antriebseinheit (92, 292) anhebbar und absenkbar ist,
wobei eine Gewichtskraft (79, 279) des Prüfgewichts (72, 272) auf den Prüfkolben (4, 204) aufbringbar ist und der Prüfkolben (4, 204) mit Hilfe der Gewichtskraft (79, 279) durch den Prüfkanal (38, 238) bewegbar ist,
wobei zwischen wenigstens einer ersten Stellung (151, 156, 158, 160, 351, 351', 356, 358, 360) des Prüfkolbens (4, 204), einer Anfahrstellung (151, 351, 351'), und wenigstens einer zweiten Stellung (152, 160, 164, 352, 352', 360, 364) des Prüfkolbens (4, 204), einer Endfahrstellung (152, 352, 352'), mittels der Antriebseinheit (92, 292) die Gewichtskraft (79, 279) mit einer aktuatorischen Zusatzkraft (108), die über eine Verbindung zur Kraftübertragung zwischen der Antriebseinheit (92, 292) und dem Prüfkolben (4, 204) ausübbar ist, für eine im Vergleich zu einer Wirkung der Gewichtskraft (79, 279) verschnellerte Bewegung (172, 372) des Prüfkolbens (4, 204) in eine erste Richtung (174) einer Kraft, der Gewichtskraft (79, 279), auf mindestens einer Strecke (166, 167, 168, 366, 367, 368) entlang des Prüfkanals (38, 238) beaufschlagt ist.

2. Fließprüfmaschine (2, 202) nach Anspruch 1, **dadurch gekennzeichnet, dass** die Antriebseinheit (92, 292) mindestens eine Kupplungseinheit (110, 310) aufweist, durch die die aktuatorische Zusatzkraft (108, 108', 108"), insbesondere in einer ersten Schaltstellung (148), von dem Prüfkolben (4, 204) trennbar ist,
und insbesondere in einer zweiten Schaltstellung (150) über die Kupplungseinheit (110, 310) die aktuatorische Zusatzkraft (108, 108', 108") auf den Prüfkolben (4, 204) aufbringbar ist.

3. Fließprüfmaschine (2, 202) nach Anspruch 1 oder Anspruch 2, **dadurch gekennzeichnet, dass**
der Prüfkolben (4, 204) einen Prüfkolbenkopf (16, 216) mit einem ersten Ankoppelbereich (20) für die Antriebseinheit (92, 292) aufweist, der mit einem Trägerbereich (24) für das Prüfgewicht (72, 272) an einem Ende des Prüfkolbens (4, 204) verbunden ist,
und insbesondere mindestens ein Servicekolben, wie ein Reinigungskolben (28) oder ein Verdichtungskolben, vorgesehen ist, der einen Kopfbereich (30) umfasst, welcher einen zweiten Ankoppelbereich (31) aufweist,
wobei vorzugsweise wenigstens einer der Ankoppelbereiche (20, 31) für die Herstellung einer form- und/oder kraftschlüssigen Verbindung zwischen der Antriebseinheit (92, 292) und dem Prüfkolben (4, 204) oder zwischen der Antriebseinheit (92, 292) und dem Servicekolben (28) gestaltet ist.

4. Fließprüfmaschine (2, 202) nach einem der vorhergehenden Ansprüche,
**dadurch gekennzeichnet, dass**
die Antriebseinheit (92, 292) mit mindestens einer in eine Schließstellung (124, 124') oder in eine Freigabestellung (126) schaltbare Halterung (112), wie eine um eine Prüfkolbenachse (10) zentrierende Spanneinheit (116), z. B. eine in einer Prüfkolbenachsenrichtung (10) rastierende Klammer oder Zange oder ein Schloss (120), wie ein elektromagnetisches Schloss oder ein pneumatisches Schloss, verbunden ist,
die insbesondere einen, vorzugsweise induktiven, Näherungssensor (128, 328, 132) zur Erkennung einer Prüfkolbenposition (185, 185') aufweist.

5. Fließprüfmaschine (2, 202) nach einem der vorhergehenden Ansprüche,
**dadurch gekennzeichnet, dass**
eine Wegmesseinrichtung (130, 330), wie eine Kolbenstellungsmesseinrichtung (132), vorgesehen ist, von der mindestens ein Stellungssignal, insbesondere des Prüfkolbens (4, 204), zur Ansteuerung eines Halteelements (122), das sich an dem Prüfkolben (4, 204), an dem Prüfgewicht (72, 73, 74, 75, 272, 273, 274) und/oder an einer Prüfgewichtsaufnahme (73, 273) befindet, bereitstellbar ist.

6. Fließprüfmaschine (2, 202) nach einem der vorhergehenden Ansprüche,
**dadurch gekennzeichnet, dass**
die Fließprüfmaschine (2, 202) eine Heizeinrichtung (133, 333) und eine Zeitkontrolleinheit (141) umfasst,
wobei die Zeitkontrolleinheit (141) zur Bestimmung von Schaltzeiten (181, 182, 184, 184', 196, 198) im Ablauf eines einzelnen Prozessschrittes (178, 180, 183, 186, 186', 186", 187, 187', 187", 188, 189, 190, 191, 195, 198, 199) vorgesehen ist,
wobei insbesondere von der Fließprüfmaschine (2, 202) mittels Antriebseinheit (92, 292) nach Erreichen einer vorgebaren Schmelztemperatur und nach Erreichen einer vorgebbaren Anschmelzzeit (182) einer Prüfmasse (34), wie einem thermoplastischen Kunststoff, ein Antriebszustand (170, 171, 171') einnehmbar ist.

7. Fließprüfmaschine (2, 202) nach einem der vorhergehenden Ansprüche,
**dadurch gekennzeichnet, dass**
durch die Antriebseinheit (92, 292) eine Kraftwirkung, insbesondere in einem zweiten Betriebszustand (171, 171'), in einer zweiten, der Gewichtskraft (79, 279) entgegengesetzten Richtung (175) mittels einer Umlenkvorrichtung (142), wie einem Schaltventil (144), insbesondere einem hydraulischen und/oder pneumatischen Schaltwechselventil (144), erzeugbar ist,
wobei vorzugsweise die Kraftwirkung in der zweiten Richtung (175) auf das Prüfgewicht (72, 272) aufbringbar ist.

8. Fließprüfmaschine (2, 202) nach einem der vorhergehenden Ansprüche,
**dadurch gekennzeichnet, dass**
eine Druckkontrolleinheit (138) vorgesehen ist und die aktuatorische Zusatzkraft (108, 108', 108"), insbesondere stufenlos bis zum Erreichen einer Prüfkolbengeschwindigkeit, regelbar einem durch die Druckkontrolleinheit (138) eingestellten Druck folgt,
und insbesondere ein maximal mit der Antriebseinheit (92, 292) auf den Prüfkolben (4, 204) beaufschlagbarer Druck, wie ein pneumatischer Druck, ein hydraulischer Druck oder ein elektrisch oder mechanisch erzeugter Druck, durch die Druckkontrolleinheit (138) vorgebbar ist.

9. Fließprüfmaschine (2, 202) nach einem der vorhergehenden Ansprüche,
**dadurch gekennzeichnet, dass**
eine der zweiten Stellungen (164, 352', 364) als Versuchsbereichsendstellung (164, 364) sich im Bereich einer Abströmseite (B, 48) des Prüfkanals (38, 238), insbesondere an einer den Prüfkanal (38, 238) begrenzenden Düse (54, 56, 58, 254) befindet und insbesondere eine der ersten Stellungen (156, 160, 351', 356, 360), vorzugsweise als Prüfbereichsendstellung (156, 356), insbesondere weniger als zwei Zentimeter, von der Versuchsbereichsendstellung (164, 364) beabstandet ist.

10. Fließprüfmaschine (2, 202) nach einem der vorhergehenden Ansprüche,
**dadurch gekennzeichnet, dass**
der Prüfkolben (4, 204) zwischen einer der ersten Stellungen (151, 156, 158, 351, 351', 356, 358), insbesondere einer Versuchsbeginnstellung (158, 358), und einer der zweiten Stellungen (152, 160, 164, 352, 352', 360, 364), insbesondere einer Versuchsendstellung (160, 360), eine dritte Stellung (153, 353) und eine vierte Stellung (154, 354), die insbesondere als transitorische Stellungen ausgebildet sind, aufweist, wobei die dritte Stellung (153, 353), eine Prüfstartstellung, von der vierten Stellung (154, 354), eine Prüfendstellung, durch eine Versuchsstrecke (168, 368) beabstandet ist,
und insbesondere die vierte Stellung (154, 354) näher an jener zweiten Stellung (160, 164, 352', 360, 364) liegt als die dritte Stellung (153, 353) und insbesondere in einer Position eines Prüfkolbenendes (12, 212) in der Versuchsstrecke (168, 368) die aktuatorische Zusatzkraft (108, 108', 108") aus der Antriebseinheit (92, 292) entkoppelt von dem Prüfkolben (4, 204) vorliegt.

11. Fließprüfmaschine (2, 202) nach einem der vorhergehenden Ansprüche,
**dadurch gekennzeichnet, dass**
die Richtung (174) der Kraftwirkung der aktuatorischen Zusatzkraft (108, 108', 108") vor dem Erreichen der zweiten Stellung (152, 164, 352, 352', 364), insbesondere nach einem Durchgang durch die vierte Stellung (154, 354) oder vor einem Durchgang durch die dritte Stellung (153, 353), umkehrbar ist, wobei der Prüfkolben (4, 204) vor einem Ende eines Auspresswegs (166, 167, 366, 367) abbremsbar (186, 186"), vorzugsweise bis zu einem Stillstand, ist.

12. Fließprüfmaschine (2, 202) nach einem der vorhergehenden Ansprüche, **dadurch gekennzeichnet, dass**
ein in einer Prüfrichtung (174) durch die Antriebseinheit (92, 292) verfahrbarer Arm (80, 280) zur Aufnahme des Prüfgewichts (72, 272) vorgesehen ist, wobei ein erstes Armende (81) eine Durchgangsöffnung (84, 284) aufweist, die um den Prüfkolben (4, 204), insbesondere um den Prüfkolbenkopf (16, 216), anordenbar ist, und insbesondere an einer Unterseite (85) des ersten Armendes (81) ein Haltebereich für den Prüfkolbenkopf (16, 216) vorhanden ist,
und wobei der Arm (80, 280), vorzugsweise zumindest in einer Endstellung (169, 169'), wie einer ersten (169) oder einer zweiten Endstellung (169'), in einer Ebene schwenkbar ist, und insbesondere mit Hilfe des Arms (80, 280) ein erstes Prüfgewicht (72) durch ein zweites Prüfgewicht (272), das vorzugsweise in einem Prüfgewichtmagazin vorliegt, ersetzbar ist.

13. Fließprüfmaschine (2, 202) nach einem der vorhergehenden Ansprüche,
**dadurch gekennzeichnet, dass**
mindestens ein Anschlag (88, 89, 89') an der Antriebseinheit (92, 292), wie ein Anschlag (88) an einem zweiten Armende (82) oder ein Anschlag (89), welcher der zweiten Stellung (352', 364) zugeordnet ist, vorgesehen ist, wobei in einer Anschlagsstellung (169, 169') der mit dem Prüfkolben (4, 204) verbundenen Antriebseinheit (92, 292) der Prüfkolben (4, 204) von der Antriebseinheit (92, 292) abkoppelbar ist.

14. Verfahren (176) zur Durchführung einer Schmelzviskositätsprüfung, insbesondere für thermoplastische Kunststoffe, vorzugsweise durch Verwendung einer Fließprüfmaschine (2, 202) nach einem der vorhergehenden Ansprüche,
wobei in einem ersten Schritt, einem Heizschritt (180), ein Prüfmaterial (34) erwärmt wird und in einem Messschritt (189) das erwärmte Prüfmaterial (34), insbesondere in einem aufgrund einer Einwirkung der Wärme eingenommenen Fließzustand, von einem Prüfkolben (4, 204) durch einen Prüfkanal (38, 238) gepresst wird,
**dadurch gekennzeichnet, dass**
in einem Messvorbereitungsschritt (178), insbesondere nach einer Anschmelzzeit (182), und/oder in einem Reinigungsschritt (191), der insbesondere in einer Bewegungsrichtungskontinuität nach dem Messschritt (189) erfolgt, eine in dem Prüfkanal (38, 238) verschiebbare Masse des, insbesondere noch vorhandenen, Prüfmaterials (34) entlang einer Gewichtskraftrichtung (79, 279) unter einer Wirkung einer aktuatorischen Zusatzkraft (108), die zu der Gewichtskraftrichtung (79, 279) parallel gerichtet oder entgegengerichtet und an den Prüfkolben (4, 204) angekoppelt ist, verschoben wird.

15. Verfahren (176) nach Anspruche 14, **dadurch gekennzeichnet, dass** der Prüfkolben (4, 204) unter Beibehaltung seiner Bewegungsrichtung (172, 172', 372), insbesondere bis zum Erreichen einer Endstellung (169, 169'), wie eines Anschlags (89, 89'), durch die aktuatorische Zusatzkraft (108, 108', 108") eine Beschleunigung erfährt und
insbesondere der Prüfkolben (4, 204) nach der Beschleunigung durch eine aktuatorische Zusatzkraft (108, 108', 108"), insbesondere durch ein Umschalten (186, 186', 186") eines Bewegungselements (142) in einer Antriebseinheit (92, 292), abgebremst wird, wobei Prüfmaterial (34) durch eine Düse (54, 56, 58, 254) den Prüfkanal (38, 238) verlässt.

16. Verfahren (176) nach Anspruch 14 oder 15, **dadurch gekennzeichnet, dass** eine Kraftrichtung (174, 175) der aktuatorischen Zusatzkraft (108, 108', 108") während eines Auspressschritts (183, 191) umgekehrt wird,
wobei insbesondere die Kraftrichtung (174, 175) auf den Kolben (4, 204, 28) in allen Stellungen (151, 152, 156, 158, 160, 164, 351, 351', 352, 352', 356, 358, 360, 364) des Kolbens (4, 204, 28) bis zum Erreichen eines Kraftwendepunkts der Gewichtskraftrichtung (79, 279) gleichgerichtet ist und ab dem Kraftwendepunkt zu der Gewichtskraftrichtung (79, 279) entgegengerichtet wirkt (186, 186', 186"),
wobei insbesondere mit einer Wegmesseinrichtung (130, 131, 132, 330) ermittelt wird, ob der Prüfkolben (4, 204) den Kraftwendepunkt erreicht (185) hat.

17. Verfahren (176) nach einem der vorhergehenden Ansprüche 14 bis 16,
**dadurch gekennzeichnet, dass**
nach dem Messvorbereitungsschritt (178) eine kraftschlüssige Verbindung zwischen dem Prüfkolben (4, 204) und einer Antriebseinheit (92, 292), insbesondere mittels einer Kupplung (110, 148, 319), getrennt (187) wird
und anschließend, insbesondere nach einer Relaxationszeit zur Erlangung einer gleichförmigen Prüfkolbenbewegung und vorzugsweise einer Vergleichmäßigung eines Prüftemperaturprofils in einem Prüfraum (36), der Messschritt (189) beginnt,
wobei in dem Messschritt (189) eine Gewichtskraft (79, 279) eines Prüfgewichts (72, 272) die Prüfmasse antreibt.

18. Verfahren (176) nach einem der vorhergehenden Ansprüche 14 bis 17,
**dadurch gekennzeichnet, dass**
aus einer Bewegung (172, 372) des Prüfkolbens (4, 204), insbesondere mittels einer Weg-Zeit-Messung (130, 189, 330, 141), und einer Wägung eines Prüfmassenabschnitts mindestens eine Materialeigenschaft, wie eine Fließgeschwindigkeit oder eine Viskosität, der Prüfmasse in einer Recheneinheit (3) bestimmt (198) wird.

19. Verfahren zur Reinigung (177) einer Fließprüfmaschine (2, 202), insbesondere nach einem der Ansprüche 1 bis 13, insbesondere vor oder nach Schmelzviskositätsprüfungen (176) nach einem der vorhergehenden Ansprüche 14 bis 18,
wobei mindestens ein Servicekolben (28) und ein Prüfzylinder (40, 100) der Fließprüfmaschine (2, 202) zum Einsatz kommen,
**dadurch gekennzeichnet, dass**
mindestens ein Reinigungsschritt (191, 195) mittels einer aktuatorischen Zusatzkraft (108, 108', 108"), geformt bzw. ausgeführt aus bzw. aufgebracht von einer Antriebseinheit (92, 292) der Fließprüfmaschine (2, 202) und an den Prüfkolben (4, 204) angekoppelt ausgeführt wird und
dass die Antriebseinheit (92, 292) in einer Schmelzviskositätsprüfung (176) zu einem Antrieb einer Bewegung (172, 172', 372) eines Prüfkolbens (4, 204) zumindest durch eine Teilstrecke (166, 366, 167, 367, 168, 368, 62) des Prüfkanals (38, 238) der Fließprüfmaschine (2, 202), vorzugsweise in Richtung auf eine Auspressdüse (54, 56, 58, 60, 254) hin, dient und
der Servicekolben (28), als ein Reinigungskolben (28) ausgebildet, an Stelle des Prüfkolbens (4, 204) in die Fließprüfmaschine (2, 202) einsetzbar ist.

20. Verfahren (177) nach Anspruch 19, **dadurch gekennzeichnet, dass** für den Reinigungsschritt (191, 195) mindestens einer der folgenden Teilschritte vorgesehen ist:
- Entkoppeln (126, 187', 187") der Antriebseinheit (92, 292) von dem Prüfkolben (4, 204) durch einen Aktuator (96, 296),
- Herausziehen (172', 193) des Prüfkolbens (4, 204) aus dem Prüfkanal (38, 238),
- Ankoppeln (124, 124', 193) des Reinigungskolbens (28) an die Antriebseinheit (92, 292),
- Einfahren (106, 172) des Reinigungskolbens (28) in den Prüfkanal (38, 238) mittels der Antriebseinheit (92, 292),
- Ausfahren (106, 172') des Reinigungskolbens (28) aus dem Prüfkanal (38, 238) mittels der Antriebseinheit (92, 292),
- Entkoppeln (197) der Antriebseinheit (92, 292) von dem Reinigungskolben (28), insbesondere durch einen Aktuator (96, 296),
- Ankoppeln (190, 197) des Prüfkolbens (4, 204) an die Antriebseinheit (92, 292), insbesondere durch den Aktuator (94, 96, 296),
- Fahren (172, 191) des Prüfkolbens (4, 204) in dem Prüfkanal (38, 238), insbesondere mittels Antriebseinheit (92, 292), bis zu einer Düse (54, 56, 58, 254).

21. Verfahren (177) nach Anspruch 19 oder Anspruch 20, **dadurch gekennzeichnet, dass** die aktuatorische Zusatzkraft (108, 108', 108") aus der Antriebseinheit (92, 292) von einer Steuereinheit (140) sequentiell (199) für die Anwendung auf den Reinigungskolben (28) und insbesondere auf einen Verdichtungskolben und auf den Prüfkolben (4, 204) gesteuert wird.

## Claims

1. Flow test machine (2, 202),
comprising a test piston (4, 204), a test channel (38, 238), at least one test weight (72, 272), and a drive unit (92, 292), such as a hydraulic or a pneumatic actuator (94, 96, 296),
**characterized in that**
in a drive state, the test weight (72, 272) and the drive unit (92, 292) are in a force-coupling connection,
wherein the test weight (72, 272) can be raised and lowered by the drive unit (92, 292), wherein a weight force (79, 279) of the test weight (72, 272) can be applied to the test piston (4, 204) and the test piston (4, 204) can be moved through the test channel (38, 238) with the aid of the weight force (79, 279),
wherein, between at least one first position (151, 156, 158, 160, 351, 351', 356, 358, 360) of the test piston (4, 204), a starting drive position (151, 351, 351'), and at least one second position (152, 160, 164, 352, 352', 360, 364) of the test piston (4, 204), an actuation end position (152, 352, 352'), an additional actuating force (108), which can be exerted via a force-transmitting connection between the drive unit (92, 292) and the test piston (4, 204), is applied by means of the drive unit (92, 292) in addition to the weight force (79, 279), in order to move (172, 372) the test piston (4, 204) in an accelerated manner, compared to an effect of the weight force (79, 279), in a first direction (174) of a force, the weight force (79, 279), over at least one stretch (166, 167, 168, 366, 367, 368) along the test channel (38, 238).

2. Flow test machine (2, 202) according to claim 1, **characterized in that** the drive unit (92, 292) has at least one coupling unit (110, 310), by which the additional actuating force (108, 108', 108") can be removed from the test piston (4, 204), in particular in a first switching position (148),
and in particular the additional actuating force (108, 108', 108") can be applied to the test piston (4, 204) via the coupling unit (110, 310) in a second switching position (150).

3. Flow test machine (2, 202) according to claim 1 or claim 2, **characterized in that** the test piston (4, 204) has a test piston head (16, 216) with a first coupling region (20) for the drive unit (92, 292), which is connected to a carrier region (24) for the test weight (72, 272) at one end of the test piston (4, 204),
and in particular at least one service piston is provided, such as a cleaning piston (28) or
a compression piston, which service piston comprises a head region (30) that has a second coupling region (31),
wherein preferably at least one of the coupling regions (20, 31) is designed to establish a form-fitting and/or force-fitting connection between the drive unit (92, 292) and the test piston (4, 204) or between the drive unit (92, 292) and the service piston (28).

4. Flow test machine (2, 202) according to any one of the preceding claims, **characterized in that**
the drive unit (92, 292) is connected to at least one holder (112) that can be switched into a closed position (124, 124') or into a release position (126), such as a clamping unit (116) configured for centring around a test piston axis (10), for example a clamp or claw that latches in a test piston axis direction (10), or a lock (120), such as an electromagnetic lock or a pneumatic lock,
which in particular has a, preferably inductive, proximity sensor (128, 328, 132) for detecting a test piston position (185, 185').

5. Flow test machine (2, 202) according to any one of the preceding claims, **characterized in that**
a displacement measuring device (130, 330) is provided, such as a piston position measuring device (132), by which at least one position signal, in particular of the test piston (4, 204), can be made available for actuating a holding element (122) located on the test piston (4, 204), on the test weight (72, 73, 74, 75, 272, 273, 274) and/or on a test weight mount (73, 273).

6. Flow test machine (2, 202) according to any one of the preceding claims, **characterized in that**
the flow test machine (2, 202) comprises a heating device (133, 333) and a time control unit (141),
wherein the time control unit (141) is provided for determining switching times (181, 182, 184, 184', 196, 198) in the course of a single process step (178, 180, 183, 186, 186', 186", 187, 187', 187", 188, 189, 190, 191, 195, 198, 199),
wherein in particular a drive state (170, 171, 171') can be assumed by the flow test machine (2, 202) by means of the drive unit (92, 292) once a predefinable melting temperature is reached and once a predefinable time (182) for melting of a test mass (34), such as a thermoplastic, is reached.

7. Flow test machine (2, 202) according to any one of the preceding claims, **characterized in that**
in particular in a second operating state (171, 171'), a force effect can be generated by the drive unit (92, 292) in a second direction (175), a direction counter to the weight force (79, 279), by means of a diverting device (142), such as a switching valve (144), in particular a hydraulic and/or pneumatic switching valve (144) of the changeover type,
wherein preferably the force effect in the second direction (175) can be applied to the test weight (72, 272).

8. Flow test machine (2, 202) according to any one of the preceding claims, **characterized in that**
a pressure control unit (138) is provided, and the additional actuating force (108, 108', 108") follows an adjustable pressure set by the pressure control unit (138), in particular in a continuously adjustable manner until a test piston speed is reached,
and in particular a maximum pressure that can be applied to the test piston (4, 204) by the drive unit (92, 292), such as a pneumatic pressure, a hydraulic pressure or an electrically or mechanically generated pressure, can be predefined by the pressure control unit (138).

9. Flow test machine (2, 202) according to any one of the preceding claims, **characterized in that**
one of the second positions (164, 352', 364), as a test area end position (164, 364), is located in the region of an outflow side (B, 48) of the test channel (38, 238), in particular at a nozzle (54, 56, 58, 254) that bounds the test channel (38, 238),
and in particular one of the first positions (156, 160, 351', 356, 360), preferably as an end position (156, 356) of a testing area, is spaced apart from the test area end position (164, 364), in particular by less than two centimetres.

10. Flow test machine (2, 202) according to any one of the preceding claims, **characterized in that**
between one of the first positions (151, 156, 158, 351, 351', 356, 358), in particular a test start position (158, 358), and one of the second positions (152, 160, 164, 352, 352', 360, 364), in particular a test end position (160, 360), the test piston (4, 204) has a third position (153, 353) and a fourth position (154, 354), which in particular are designed as transitory positions, wherein the third position (153, 353), a position of begin of testing, is spaced apart from the fourth position (154, 354), a position of end of testing, by a test stretch (168, 368),
and in particular the fourth position (154, 354) is closer to said second position (160, 164, 352', 360, 364) than the third position (153, 353), and in particular the additional actuating force (108, 108', 108") from the drive unit (92, 292) is decoupled from the test piston (4, 204) when a test piston end (12, 212) is positioned in the test stretch (168, 368).

11. Flow test machine (2, 202) according to any one of the preceding claims,
**characterized in that**
the direction (174) of the force effect of the additional actuating force (108, 108', 108") can be reversed prior to reaching the second position (152, 164, 352, 352', 364), in particular after passing through the fourth position (154, 354) or before passing through the third position (153, 353), wherein the test piston (4, 204) can be braked (186, 186"), preferably to a standstill, before an end of a press-out path (166, 167, 366, 367).

12. Flow test machine (2, 202) according to any one of the preceding claims,
**characterized in that**
an arm (80, 280), which can be moved in a test direction (174) by the drive unit (92, 292), is provided for mounting the test weight (72, 272), wherein a first arm end (81) has a through-opening (84, 284) which can be arranged around the test piston (4, 204), in particular around the test piston head (16, 216), and in particular a holding region for the test piston head (16, 216) is present on an underside (85) of the first arm end (81),
and wherein the arm (80, 280), preferably at least in one end position (169, 169'), such as a first (169) or a second end position (169'), is pivotable in one plane, and in particular a first test weight (72) can be replaced with a second test weight (272), which is preferably located in a test weight magazine, with the aid of the arm (80, 280).

13. Flow test machine (2, 202) according to any one of the preceding claims,
**characterized in that**
at least one stop (88, 89, 89') is provided on the drive unit (92, 292), such as a stop (88) at a second arm end (82) or a stop (89) assigned to the second position (352', 364), wherein, in a stop position (169, 169') of the drive unit (92, 292) connected to the test piston (4, 204), the test piston (4, 204) can be uncoupled from the drive unit (92, 292).

14. Method (176) for carrying out a melt viscosity test, in particular for thermoplastics, preferably by using a flow test machine (2, 202) according to any one of the preceding claims,
wherein in a first step, a heating step (180), a test material (34) is heated,
and in a measurement step (189) the heated test material (34) is pressed through a test channel (38, 238) by a test piston (4, 204), in particular in a flow state that is assumed due to the effect of the heat,
**characterized in that**
in a measurement preparation step (178), in particular after a time for incipient melting (182), and/or in a cleaning step (191), which in particular takes place in a movement direction continuity after the measurement step (189), a mass of the, in particular still present, test material (34) that is displaceable in the test channel (38, 238) is displaced along a weight force direction (79, 279) under the effect of an additional actuating force (108), which is directed parallel to the weight force direction (79, 279) or counter to the latter and is coupled to the test piston (4, 204).

15. Method (176) according to claim 14, **characterized in that**
the test piston (4, 204) is accelerated by the additional actuating force (108, 108', 108") while maintaining its movement direction (172, 172', 372), in particular until an end position (169, 169'), such as a stop (89, 89'), is reached, and
in particular the test piston (4, 204), after being accelerated by an additional actuating force (108, 108', 108"), is braked, in particular by switching over (186, 186', 186") a movement element (142) in a drive unit (92, 292), wherein test material (34) leaves the test channel (38, 238) through a nozzle (54, 56, 58, 254).

16. Method (176) according to claim 14 or 15, **characterized in that** a force direction (174, 175) of the additional actuating force (108, 108', 108") is reversed during a press-out step (183, 191),
wherein in particular the force direction (174, 175) is directed toward the piston (4, 204, 28) in all positions (151, 152, 156, 158, 160, 164, 351, 351', 352, 352', 356, 358, 360, 364) of the piston (4, 204, 28) until a force reversal point of the weight force direction (79, 279) is reached, and after the force reversal point acts counter to (186, 186', 186") the weight force direction (79, 279),
wherein in particular a displacement measuring system (130, 131, 132, 330) is used to determine whether the test piston (4, 204) has reached the force reversal point (185).

17. Method (176) according to any one of the preceding claims 14 to 16,
**characterized in that**
after the measurement preparation step (178) a force-fitting connection between the test piston (4, 204) and a drive unit (92, 292), in particular by means of a coupling (110, 148, 319), is disconnected (187),
and then, in particular after a relaxation time to achieve a uniform test piston movement and preferably an equalization of a test temperature profile in a test space (36), the measurement step (189) begins,
wherein in the measurement step (189) a weight force (79, 279) of a test weight (72, 272) drives the test mass.

18. Method (176) according to any one of the preceding claims 14 to 17,
**characterized in that**
at least one material property, such as a flow rate or a viscosity, of the test mass is determined (198) in a computing unit (3) from a movement (172, 372) of the test piston (4, 204), in particular by means of a distance-time measurement (130, 189, 330, 141), and a weighing of a test mass portion.

19. Method for cleaning (177) a flow test machine (2, 202), in particular according to any one of claims 1 to 13, in particular before or after melt viscosity tests (176) according to any one of the preceding claims 14 to 18,
wherein at least one service piston (28) and a test cylinder (40, 100) of the flow test machine (2, 202) are used,
**characterized in that**
at least one cleaning step (191, 195) is carried out by means of an additional actuating force (108, 108', 108"), which is formed and/or implemented and/or applied by a drive unit (92, 292) of the flow test machine (2, 202) and is coupled to the test piston (4, 204), and **in that** in a melt viscosity test (176) the drive unit (92, 292) serves to drive a movement (172, 172', 372) of a test piston (4, 204) at least through a partial stretch (166, 366, 167, 367, 168, 368, 62) of the test channel (38, 238) of the flow test machine (2, 202), preferably in the direction of a press-out nozzle (54, 56, 58, 60, 254), and
the service piston (28), designed as a cleaning piston (28), is insertable in place of the test piston (4, 204) in the flow test machine (2, 202).

20. Method (177) according to claim 19, **characterized in that** for the cleaning step (191, 195) at least one of the following sub-steps is provided:
- uncoupling (126, 187', 187") the drive unit (92, 292) from the test piston (4, 204) by way of an actuator (96, 296),
- pulling (172', 193) the test piston (4, 204) out of the test channel (38, 238),
- coupling (124, 124', 193) the cleaning piston (28) to the drive unit (92, 292),
- moving (106, 172) the cleaning piston (28) into the test channel (38, 238) by means of the drive unit (92, 292),
- extracting (106, 172') the cleaning piston (28) from the test channel (38, 238) by means of the drive unit (92, 292),
- uncoupling (197) the drive unit (92, 292) from the cleaning piston (28), in particular by way of an actuator (96, 296),
- coupling (190, 197) the test piston (4, 204) to the drive unit (92, 292), in particular by way of the actuator (94, 96, 296),
- moving (172, 191) the test piston (4, 204) in the test channel (38, 238), in particular by means of the drive unit (92, 292), as far as a nozzle (54, 56, 58, 254).

21. Method (177) according to claim 19 or claim 20, **characterized in that** the additional actuating force (108, 108', 108") from the drive unit (92, 292) is sequentially (199) controlled by a control unit (140) for application to the cleaning piston (28) and in particular to a compression piston and to the test piston (4, 204).

## Revendications

1. Machine d'essai d'écoulement (2, 202)
comprenant un piston d'essai (4, 204), un canal d'essai (38, 238), au moins un poids d'essai (72, 272) et une unité d'entraînement (92, 292) ainsi qu'un actionneur hydraulique ou pneumatique (94, 96, 296),
**caractérisée en ce que**
le poids d'essai (72, 272) et l'unité d'entraînement (92, 292) sont, dans un état d'entraînement, dans une relation de couplage de force,
cependant que le poids d'essai (72, 272) peut être soulevé et abaissé par l'unité d'entraînement (92, 292)
cependant qu'une force de poids (79, 279) du poids d'essai (72, 272) peut être appliquée au piston d'essai (4, 204) et que le piston d'essai (4, 204) peut être déplacé à travers le canal d'essai (38, 238) à l'aide de la force de poids (79, 279),
cependant que la force de poids (79, 279) est appliquée entre au moins une première position (151, 156, 158, 160, 351, 351', 356, 358, 360) du piston d'essai (4, 204), une position de démarrage (151, 351, 351') et au moins une seconde position (152, 160, 164, 352, 352', 360, 364) du piston d'essai (4, 204), une position de fin de course (152, 352, 352') au moyen de l'unité d'entraînement (92, 292) avec une force supplémentaire d'actionnement (108), qui peut être exercée par une liaison pour la transmission de force entre l'unité d'entraînement (92, 292) et le piston d'essai (4, 204), pour un déplacement (172, 372) du piston d'essai (4, 204) qui est accéléré en comparaison avec un effet de la force de poids (79, 279) dans une première direction (174) d'une force, la force de poids (79, 279), sur au moins un trajet (166, 167, 168, 366, 367, 368) le long du canal d'essai (38, 238).

2. Machine d'essai d'écoulement (2, 202) selon la revendication 1, **caractérisée en ce que** l'unité d'entraînement (92, 292) présente au moins une unité de couplage (110, 310) par laquelle la force supplémentaire d'actionnement (108, 108', 108") peut être déconnectée du piston d'essai (4, 204), en particulier dans une première position de couplage (148) et, en particulier dans une seconde position de couplage (148) la force supplémentaire d'actionnement (108, 108', 108") peut être appliquée sur le piston d'essai (4, 204) par l'unité de couplage (110, 310).

3. Machine d'essai d'écoulement (2, 202) selon la revendication 1 ou 2, **caractérisée en ce que** le piston d'essai (4, 204) présente une tête de piston d'essai (16, 216) avec une première zone de couplage (20) pour l'unité d'entraînement (92, 292) qui est reliée à une extrémité du piston d'essai (4, 204) à une zone de support (24) pour le poids d'essai (72, 272) et qu'il est prévu en particulier au moins un piston de service, comme un piston de nettoyage (28) ou un piston de compactage, qui comprend une zone de tête (30) qui présente une seconde zone de couplage (31), cependant que de préférence au moins l'une des zones de couplage (20, 31) est configurée pour établir une liaison de forme et/ou de force entre l'unité d'entraînement (92, 292) et le piston d'essai (4, 204) ou entre l'unité d'entraînement (92, 292) et le piston de service (28).

4. Machine d'essai d'écoulement (2, 202) selon l'une des revendications précédentes, **caractérisée en ce que** l'unité d'entraînement (92, 292) est reliée à au moins un support (112) commutable dans une position de fermeture (124, 124') ou dans une position de déblocage (126), comme une unité de serrage (116) configurée pour se centrer autour d'un axe du piston d'essai (10), par exemple un crampon ou une pince qui s'enclenche dans une direction de l'axe du piston d'essai (10), ou une serrure (120), comme une serrure électromagnétique ou une serrure pneumatique, qui présente en particulier un détecteur de proximité, de préférence inductif (128, 328, 132), pour reconnaître une position du piston d'essai (185, 185').

5. Machine d'essai d'écoulement (2, 202) selon l'une des revendications précédentes, **caractérisée en ce qu'**il est prévu un dispositif de mesure de course (130, 300), comme un dispositif de mesure de déplacement du piston (132), par lequel au moins un signal de position, en particulier du piston d'essai (4, 204), peut être délivré pour la commande d'un élément de support (122) qui se trouve sur le piston d'essai (4, 204), sur le poids d'essai (72, 73, 74, 75, 272, 273, 274) et/ou sur un logement du poids d'essai (73, 273).

6. Machine d'essai d'écoulement (2, 202) selon l'une des revendications précédentes, **caractérisée en ce que** la machine d'essai d'écoulement (2, 202) comprend un dispositif de chauffage (133, 333) et une unité de contrôle du temps (141), cependant que l'unité de contrôle du temps (141) est prévue pour déterminer des temps de commutation (181, 182, 184, 184', 196, 198) au cours d'une étape individuelle de processus (178, 180, 186, 186', 186", 187, 187', 187", 188, 189, 190, 191, 195, 199), cependant qu'un état d'entraînement (170, 171, 171') peut être pris en particulier par la machine d'essai d'écoulement (2, 202) au moyen de l'unité d'entraînement (92, 292) après avoir atteint une température de fusion qui peut être prédéterminée et après avoir atteint un temps de fusion qui peut être prédéterminé (182) d'une masse d'essai (34) comme une matière synthétique thermoplastique.

7. Machine d'essai d'écoulement (2, 202) selon l'une des revendications précédentes, **caractérisée en ce qu'**un effet de force peut être produit par l'unité d'entraînement (92, 292), en particulier dans un second état de fonctionnement (171, 171'), dans une seconde direction (175) opposée à la force de poids (79, 279), au moyen d'un dispositif de renvoi (142), comme une soupape de commutation (144), en particulier une soupape hydraulique et/ou pneumatique de changement de commutation (144), cependant que de préférence l'effet de force dans la seconde direction (175) peut être appliqué sur le poids d'essai (72, 272).

8. Machine d'essai d'écoulement (2, 202) selon l'une des revendications précédentes, **caractérisée en ce qu'**il est prévu une unité de contrôle de la pression (138) et que la force supplémentaire d'actionnement (108, 108', 108"), en particulier continue jusqu'à atteindre une vitesse du piston d'essai, suit de manière réglable une pression ajustée par l'unité de contrôle de la pression (138), et qu'en particulier une pression qui peut être appliquée de manière maximale au piston d'essai (4, 204) avec l'unité d'entraînement (92, 292), comme une pression pneumatique, une pression hydraulique ou une pression produite électriquement ou mécaniquement, peut être prédéterminée par l'unité de contrôle de la pression (138).

9. Machine d'essai d'écoulement (2, 202) selon l'une des revendications précédentes, **caractérisée en ce qu'**une des secondes positions (164, 352', 364) se trouve en tant que position d'extrémité de la zone d'expérimentation (164, 364) dans la zone d'un côté sortie d'écoulement (B, 48) du canal d'essai (38, 238), en particulier sur une tuyère (54, 56, 58, 254) qui délimite le canal d'essai (38, 238) et qu'en particulier l'une des premières positions (156, 160, 351', 356, 360) est espacée, de préférence en tant que position d'extrémité de la zone d'essai (156, 356), de la position d'extrémité de la zone d'expérimentation (164, 364), en particulier de moins de deux centimètres.

10. Machine d'essai d'écoulement (2, 202) selon l'une des revendications précédentes, **caractérisée en ce que** le piston d'essai (4, 204) présente, entre l'une des premières positions (151, 156, 158, 351, 351', 356, 358), en particulier entre une position de début d'expérimentation (1158, 358) et l'une des secondes positions (152, 160, 164, 352, 352', 360, 364), en particulier une position de fin d'expérimentation (160, 360), une troisième position (153, 353) et une quatrième position (154, 354), qui sont configurées en particulier comme des positions transitoires, cependant que la troisième position (153, 353), une position de démarrage d'essai, est espacée de la quatrième position (154, 354), une position de fin d'essai, par un trajet d'expérimentation (168, 368) et qu'en particulier la quatrième position (154, 354) est située plus près de la seconde position (160, 164, 352', 360, 364) qu'en particulier dans une position d'une extrémité de piston d'essai ( 12, 212) dans le trajet d'expérimentation (168, 368) la force supplémentaire d'actionnement (108, 108', 108") provenant de l'unité d'entraînement (92, 292) est découplée du piston d'essai (4, 204).

11. Machine d'essai d'écoulement (2, 202) selon l'une des revendications précédentes, **caractérisée en ce que** la direction (174) de l'effet de force de la force supplémentaire d'actionnement (108, 108', 108") peut être inversée avant d'atteindre la seconde position (152, 164, 352, 352', 364), en particulièr après un passage par la quatrième position (154, 354) ou avant un passage par la troisième position (153, 353), cependant que le piston d'essai (4, 204) peut être freiné (186, 186") avant une extrémité d'un trajet d'éjection (166, 167, 366, 367), de préférence jusqu'à un arrêt.

12. Machine d'essai d'écoulement (2, 202) selon l'une des revendications précédentes, **caractérisée en ce qu'**un bras (80, 280) déplaçable par l'unité d'entraînement (92, 292) dans une direction d'essai (174) est prévu pour recevoir le poids d'essai (72, 272), une première extrémité de bras (81) présentant une ouverture de passage (84, 284) qui peut être placée autour du piston d'essai (4, 204), en particulier autour de la tête du piston d'essai (16, 216), et qu'il existe en particulier une zone de retenue pour la tête du piston d'essai (16, 216) sur une face inférieure (85) de la première extrémité de bras (81) et le bras (80, 280) pouvant pivoter dans un plan, de préférence au moins dans une position d'extrémité (169, 169') comme une première (169) ou une seconde position d'extrémité (169') et en particulier qu'un premier poids d'essai (72) peut être remplacé à l'aide du bras (80, 280) par un second poids d'essai (272) qui se trouve de préférence dans un magasin de poids d'essai.

13. Machine d'essai d'écoulement (2, 202) selon l'une des revendications précédentes, **caractérisée en ce qu'**au moins une butée (88, 89, 89') est prévue sur l'unité d'entraînement (92, 292) comme une butée (88) à une seconde extrémité du bras (82) ou une butée (89) qui est associée à la seconde position (352', 364), cependant que le piston d'essai (4, 204) peut être découplé de l'unité d'entraînement (92, 292) dans une position de butée (169, 169') de l'unité d'entraînement (92, 292) reliée au piston d'essai (4, 204).

14. Procédé (176) pour effectuer un essai de la viscosité à l'état fondu, en particulier pour des matières synthétiques thermoplastiques, de préférence en utilisant une machine d'essai d'écoulement (2, 202) selon l'une des revendications précédentes, cependant qu'un matériau d'essai (34) est réchauffé dans une première étape, une étape de chauffage (180), et que, dans une étape de mesure (189), le matériau d'essai réchauffé (34) est pressé par un piston d'essai (4, 204) à travers le canal d'essai (38, 238), en particulier dans un état d'écoulement pris en raison de l'action de la chaleur,
**caractérisé en ce que**,
dans une étape de préparation de la mesure (178), en particulier après un temps de fusion (182) et/ou dans une étape de nettoyage (191) qui est effectuée après l'étape de mesure (189), en particulier dans une continuité de la direction de déplacement, une masse déplaçable dans le canal d'essai (38, 238) du matériau d'essai (34) qui en particulier est encore présent est déplacée le long d'une direction de la force de poids (79, 279) sous l'effet d'une force supplémentaire d'actionnement (108) qui est orientée parallèlement à ou en sens contraire de la direction de la force de poids (79, 279) et qui est couplée au piston d'essai (4, 204).

15. Procédé (176) selon la revendication 14, **caractérisé en ce que** le piston d'essai (4, 204) subit une accélération par la force supplémentaire d'actionnement (108, 108', 108") en conservant sa direction de déplacement (172, 172', 372), en particulier jusqu'à atteindre une position d'extrémité (169, 169') comme celle d'une butée (89, 89'), et en particulier le piston d'essai (4, 204) est freiné après l'accélération par une force supplémentaire d'actionnement (108, 108', 108"), en particulier par une commutation (186, 186', 186") d'un élément de déplacement (142) dans une unité d'entraînement (92, 292), cependant que du matériau d'essai (34) quitte le canal d'essai (38, 238) par une tuyère (54, 56, 58, 254).

16. Procédé (176) selon la revendication 14 ou 15, **caractérisé en ce qu'**une direction de force (174, 175) de la force supplémentaire d'actionnement (108, 108', 108") est inversée pendant une étape d'éjection (183, 191), cependant qu'en particulier la direction de force (174, 175) est orientée sur le piston (4, 204) dans la même direction dans toutes les positions (151, 152, 156, 158, 160, 164, 351, 351', 352, 352', 356, 358, 360, 364) du piston (4, 204, 28) jusqu'à atteindre un point de basculement de la force de la direction de la force de poids (79, 279) et agit en étant orientée en sens inverse à partir du point de basculement de la force de poids (79, 279) cependant qu'il est déterminé avec un dispositif de mesure de course (130, 131, 132, 330) si le piston d'essai (4, 204) a atteint (185) le point de basculement de la force (185).

17. Procédé (176) selon l'une des revendications précédentes 14 à 16, **caractérisé en ce qu'**après l'étape de préparation de la mesure (178) une liaison de force entre le piston d'essai (4, 204) et une unité d'entraînement (92, 292), en particulier au moyen d'un couplage (110, 148, 319), est séparée et qu'ensuite l'étape de mesure (189) commence, en particulier après un temps de relaxation pour obtenir un déplacement uniforme du piston d'essai et de préférence une homogénéisation d'un profil de température d'essai dans un espace d'essai (36), cependant que dans l'étape de mesure (189) une force de poids (79, 279) d'un poids d'essai (72, 272) entraîne la masse d'essai.

18. Procédé (176) selon l'une des revendications précédentes 14 à 17, **caractérisé en ce qu'**au moins une propriété du matériau, comme une vitesse d'écoulement ou une viscosité, de la masse d'essai est déterminée (198) dans une unité de calcul (3) à partir d'un déplacement (172, 372) du piston d'essai (4, 204), en particulier au moyen d'une mesure course/temps (130, 189, 330, 141) et d'une pesée d'une partie de la masse d'essai.

19. Procédé pour le nettoyage (177) d'une machine d'essai d'écoulement (2, 202) en particulier selon l'une des revendications 1 à 13, en particulier avant ou après des contrôles de la viscosité à l'état fondu (176) selon l'une des revendications 14 à 18, cependant qu'au moins un piston de service (28) et un vérin d'essai (40, 100) de la machine d'essai d'écoulement (2, 202) sont utilisés, **caractérisé en ce**
**qu'**au moins une étape de nettoyage (191, 195) est exécutée au moyen d'une force supplémentaire d'actionnement (108, 108', 108") en étant formée ou exécutée à partir de ou appliquée par une unité d'entraînement (92, 292) de la machine d'essai d'écoulement (2, 202) et couplée au piston d'essai (4, 204) et que l'unité d'entraînement (92, 292) sert, dans un essai de la viscosité à l'état fondu (176), à un entraînement d'un déplacement (172, 172', 372) d'un piston d'essai (4, 204) au moins à travers un tronçon (166, 366, 167, 367, 168, 368, 62) du canal d'essai (38, 238) de la machine d'essai d'écoulement (2, 202), de préférence en direction d'une tuyère d'éjection (54, 56, 58, 60, 254), et que le piston de service (28), configuré comme un piston de nettoyage (28), peut être intégré dans la machine d'essai d'écoulement (2, 202) à la place du piston d'essai (4, 204).

20. Procédé (177) selon la revendication 19, **caractérisé en ce qu'**il est prévu, pour l'étape de nettoyage (191, 195), au moins l'une des étapes partielles suivantes :
- découplage (126, 187', 187") de l'unité d'entraînement (92, 292) du piston d'essai (4, 204) par un actionneur (96, 296),
- extraction (172', 193) du piston d'essai (4, 204) du canal d'essai (38, 238),
- couplage (124, 124', 193) du piston de nettoyage (28) à l'unité d'entraînement (92, 292),
- introduction (106, 172) du piston de nettoyage (28) dans le canal d'essai (38, 238) au moyen de l'unité d'entraînement (92, 292),
- sortie (106, 172') du piston de nettoyage (28) du canal d'essai (38, 238) au moyen de l'unité d'entraînement (92, 292),
- découplage (197) de l'unité d'entraînement (92, 292) du piston de nettoyage (28) en particulier par un actionneur (94, 96, 296),
- couplage (190, 197) du piston d'essai (4, 204) à l'unité d'entraînement (92, 292), en particulier par un actionneur (94, 96, 296),
- déplacement (172, 191) du piston d'essai (4, 204) dans le canal d'essai (38, 238), en particulier au moyen de l'unité d'entraînement (92, 292), jusqu'à une tuyère (54, 56, 58, 254).

21. Procédé (177) selon la revendication 19 ou la revendication 20, **caractérisé en ce que** la force supplémentaire d'actionnement (108, 108', 108") provenant de l'unité d'entraînement (92, 292) est commandée par une unité de commande (140) de manière séquentielle (199) pour l'application au piston de nettoyage (28) et en particulier à un piston de compactage et au piston d'essai (4, 204).
